# EUROPÄISCHE PATENTSCHRIFT

(11) **EP 1 613 600 B1**
(45) Veröffentlichungstag und Bekanntmachung des Hinweises auf die Patenterteilung: **23.12.2015**
(21) Anmeldenummer: 04719379.2
(22) Anmeldetag: 11.03.2004
(51) Int. Cl.: C07D 217/16, C07D 401/12, A61K 31/47, A61P 9/00, A61P 11/00, A61P 13/12

(54) **SUBSTITUIERTE 4-PHENYLTETRAHYDROISOCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 4-PHENYLTETRAHYDROISOQUINOLINES, METHOD FOR THE PRODUCTION THEREOF, THE USE OF THE SAME AS MEDICAMENTS, AND MEDICAMENT CONTAINING SUCH COMPOUNDS
4-PHENYLTETRAHYDROISOQUINOLEINE SUBSTITUEE, SON PROCEDE DE FABRICATION, SON UTILISATION COMME MEDICAMENT, ET MEDICAMENT CONTENANT CE COMPOSE

(30) Priorität: 24.03.2003 DE 10312963
(43) Veröffentlichungstag der Anmeldung: 11.01.2006
(73) Patentinhaber: Sanofi-Aventis Deutschland GmbH, 65929 Frankfurt am Main (DE)
(72) Erfinder: HOFMEISTER, Armin, 65926 Frankfurt (DE); HEINELT, Uwe, 65187 Wiesbaden (DE); LANG, Hans-Jochen, 65719 Hofheim (DE); FRICK, Wendelin, 65510 Hünstetten-Beuerbach (DE); BLEICH, Markus, 24226 Heikendorf (DE); WIRTH, Klaus, 65830 Kriftel (DE)
(86) Internationale Anmeldenummer: PCT/EP2004/002497
(87) Internationale Veröffentlichungsnummer: WO 2004/085404

(56) Entgegenhaltungen:
- WO-A-01/32624
- WO-A-03/051866
- WO-A-03/055490
- WO-A1-03/048129
- WO-A2-03/055880

## Beschreibung

Die Erfindung betrifft Verbindungen vom Typ der substituierten 4-Phenyltetrahydroisochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I 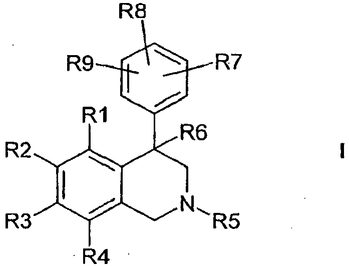 worin bedeuten:
- R1 und R3: gleich H, und R2 und R4 gleich Cl;
- R5: H, CH₃, CH₂CH₃, CF₃ oder CH₂CF₃;
- R6: H, OH, CH₃, CF₃, OCH₃ oder OCOCH₃;
- R7, R8 und R9: unabhängig voneinander H, F, Cl, Br, I, OH, CH₃, CH₂CH₃, CF₃, CH₂CF₃, OCH₃, OCH₂CH₃, OCF₃, OCH₂CF₃, SO₂R47, SO₃R60, COR47, COOR60, NR51R52 oder eine Gruppe -L-G;
R47 H, CH₃, CH₂CH₃, CF₃, CH₂CF₃ oder NR48R49;
R48 und R49 unabhängig voneinander H, CH₃, CH₂CH₃, CF₃, CH₂CF₃, COCH₃, COCH₂CH₃, COCF3 oder COCH₂CF₃;
R60 H, CH₃, CH₂CH₃, CF₃, CH₂CF₃;
R51 und R52 unabhängig voneinander H, CH₃, CH₂CH₃, CF₃, CH₂CF₃, COCH₃, COCH₂CH₃, COCF₃ oder COCH₂CF₃;
L NR30-, -NR30CO-, -CONR30-, -NR30CONR31-oder -NR30COO-;
R30 und R31unabhängig voneinander H, CH₃, CH₂CH₃, CF₃ oder CH₂CF₃;
G eine Gruppe Cₐ(OR32)ₓH₂ₐ₊₁₋ₓ, wobei eine oder mehrere CH₂-Gruppen durch O oder NR33 ersetzt sein können, C_{b}(OR32)yH_{2b-1-y}, wobei eine oder mehrere CH₂-Gruppen durch O oder NR33 ersetzt sein können,
-(CH₂)_{z}-COOR34, -(CH₂)_{z} -SO₃R34, -(CH₂)_{z} -N⁺R35R36R37, wobei 1 oder 2 H-Atome der -(CH₂)_{z}- Einheiten durch OR32-Gruppen ersetzt sein können, -CR38R39-COOR40 oder -CR38R39NR41 R42;
a 2, 3, 4, 5, 6, 7 oder 8;
x 2, 3, 4, 5, 6, 7 oder 8;
R32 H, CH₃, CH₂CH₃, CF₃, CH₂CF₃, COCH₃, COCH₂CH₃, COCF₃ oder COCH₂CF₃;
R33 H, CH₃, CH₂CH₃, CF₃ oder CH₂CF₃;
b 3, 4, 5, 6 oder 7;
y 2, 3, 4, 5, 6 oder 7;
z 1 oder 2;
R34, R35, R36 und R37 unabhängig voneinander H, CH₃, CH₂CH₃, CF₃ oder CH₂CF₃;
R38 -(CH₂)ₙ -Y;
n 0, 1, 2, 3 oder 4;
Y Alkyl mit 1, 2, 3 oder 4 C-Atomen, die teilweise oder vollständig fluoriert sein können und in dem eine oder mehrere CH₂-Gruppen durch O, S oder NR43 ersetzt sind, COOR44, CONR45R46, NHC(NH)NH₂, Phenyl oder Heteroaryl, ausgewählt aus der Gruppe Imidazolyl, Pyrazolyl, Pyrrolyl, Triazolyl, Tetrazolyl, Thiazolyl, Oxazolyl und Pyridyl, wobei die Phenyl-und Heteroarylreste substituiert sein können mit bis zu 3 Substituenten ausgewählt aus der Gruppe CH₃, CF₃, OH, OCH₃ oder NH₂;
R43, R44, R45 und R46
unabhängig voneinander H, CH₃, CH₂CH₃,
CF₃ oder CH₂CF₃;
R39 H, CH₃, CH₂CH₃, CF₃ oder CH₂CF₃;
R40 H, CH₃, CH₂CH₃, CF₃ oder CH₂CF₃;
R41 und R42 unabhängig voneinander H, CH₃, CH₂CH₃, CF₃, CH₂CF₃, COCH₃, COCH₂CH₃, COCF₃ oder COCH₂CF₃;
wobei mindestens einer der Reste R7, R8 oder R9 durch die Gruppe -L-G definiert sein muss,
sowie deren pharmazeutisch verträgliche Salze und Trifluoressigsäuresalze.

Bevorzugt sind Verbindungen der Formel I, worin bedeuten
- R1, und R3: gleich H, und R2 und R4 gleich Cl;
- R5: CH₃;
- R6: H;
- R7, R8 und R9: unabhängig voneinander H, OH, CH₃, CF₃, OCH₃, SO₂R47, SO₃R60, COR47, COOR60, NR51 R52 oder eine Gruppe -L-G;
R47 H, CH₃ oder NR48R49;
R48 und R49 unabhängig voneinander H, CH₃, oder COCH₃,
R60 H oder CH_{3;}
R51 und R52 unabhängig voneinander H, CH₃, CH₂CH₃, oder COCH₃,
L -NR30CO-, -CONR30- oder -NR30CONR31-;
R30 und R31 gleich H;
G eine Gruppe der Form Cₐ(OR32)ₓH₂ₐ₊₁₋ₓ, wobei eine oder mehrere CH₂-Gruppen durch O oder NR33 ersetzt sein können, C_{b}(OR32)yH_{2b-1-y}, wobei eine oder mehrere CH₂-Gruppen durch O oder NR33 ersetzt sein können,
-(CH₂)₂-COOH, -(CH₂)₂ -SO₃H, -(CH₂)₂ -N(CH3) 3⁺, wobei 1 oder 2 H-Atome der -(CH₂)₂- Einheiten durch OH-Gruppen ersetzt sein können, -CR38R39-COOR40 oder -CR38R39NR41R42;
a 3, 4, 5 oder 6;
x 2, 3, 4 oder 5;
R32 H;
R33 H oder CH₃;
b 5 oder 6;
y 2, 3, 4 oder 5;
R38 CH₂OH, CH₂SH, CH₂NH₂, CH(OH)CH₃, CH₂CH₂SCH₃, CH₂CH₂CH₂NH₂, CH₂CH₂CH₂CH₂NH₂, CH₂CH₂CH₂NHC(NH)NH₂, CH₂COOH, CH₂CONH₂, CH₂CH₂COOR44, CH₂CH₂CONH₂, COOH, Phenyl, 4-Hydroxyphenyl, 4-Imidazolyl oder 3-Indolyl;
R39 H;
R40 H, CH₃ oder CH₂CH₃;
R41 und R42 unabhängig voneinander H, CH₃ oder COCH₃; und
R44 H, CH₃ oder CH₂CH₃;
wobei mindestens einer der Reste R7, R8 oder R9 durch die Gruppe -L-G definiert sein muss,
sowie deren pharmazeutisch verträgliche Salze und Trifluoressigsäuresalze.

In einer Ausführungsform sind Verbindungen der Formel I bevorzugt, bei denen R5 beschrieben wird durch H, CH₃ oder CF₃.

In einer anderen Ausführungsform sind Verbindungen der Formel I bevorzugt, bei denen R6 beschrieben wird durch H, OH, CH₃, CF₃, OCH₃ oder OCOCH₃, bevorzugt sind dabei Verbindungen, in denen R6 durch H beschrieben wird.

In einer Ausführungsform sind Verbindungen der Formel I bevorzugt, bei denen R7, R8 und R9 unabhängig voneinander beschrieben werden durch H, OH, CH₃, CF₃, OCH₃, SO₂R47, SO₃R60, COR47, COOR60, NR51R52 oder eine Gruppe -L-G, wobei
- R47: H, CH₃ oder NR48R49;
R48 und R49 unabhängig voneinander H, CH₃ oder COCH₃;
- R60: H, CH₃;
- R51 und R52: unabhängig voneinander H, CH₃, CH₂CH₃ oder COCH₃;
- L: -NR30CO-, -CONR30- oder -NR30CONR31-;
R30 und R31 H;
- G: eine Gruppe der Form Cₐ(OR32)ₓH₂ₐ₊₁₋ₓ, C_{b}(OR32)_{y}H_{2b-1-y}, wobei eine CH₂-Gruppe durch O ersetzt sein kann, -(CH₂)₂-COOH, -(CH₂)₂ -SO₃H, -(CH₂)₂ -N⁺(CH₃)₃, wobei 1 oder 2 H-Atome der -(CH₂)₂- Einheiten durch OH-Gruppen ersetzt sein können, -CR38R39-COOR40 oder -CR38R39NR41 R42
a 3, 4, 5 oder 6;
x 2, 3, 4 oder 5;
R32 H;
b 5 oder 6;
y 2, 3, 4 oder 5;
R38 Alkyl mit 1, 2, 3 oder 4 C-Atomen, CH₂OH, CH₂SH, CH₂NH₂, CH(OH)CH₃, CH₂CH₂SCH₃, CH₂CH₂CH₂NH₂, CH₂CH₂CH₂CH₂NH₂, CH₂CH₂CH₂NHC(NH)NH₂, CH₂COOH, CH₂CONH₂, CH₂CH₂COOR44, CH₂CH₂CONH₂, COOH, Phenyl, 4-Hydroxyphenyl oder 4-Imidazolyl;
R39 H;
R40 H, CH₃ oder CH₂CH₃;
R41 und R42 unabhängig voneinander H, CH₃ oder COCH₃;
R44 H, CH₃ oder CH₂CH₃;
wobei mindestens einer der Reste R7, R8 oder R9 durch die Gruppe -L-G definiert sein muss.

In einer Ausführungsform sind dabei Verbindungen der Formel I bevorzugt, in denen einer der Reste R7, R8 oder R9 durch LG beschrieben wird und die anderen Reste R7, R8, R9 durch H, OH, CH₃, CF₃, OCH₃, SO₂R47, SO₃R60, COR47, COOR60 oder NR51 R52 beschrieben werden, insbesondere durch Wasserstoff oder COOH; besonders bevorzugt sind Verbindungen der Formel I, in denen einer der Reste R7, R8 oder R9 durch LG beschrieben wird und die anderen Reste R7, R8, R9 durch H beschrieben werden

In einer Ausführungsform werden Verbindungen der Formel bevorzugt, in denen zwei der Reste R7, R8 oder R9 durch LG beschrieben werden und einer der Reste R7, R8 oder R9 durch Wasserstoff.

Speziell bevorzugt sind Verbindungen der Formel I ausgewählt aus der Gruppe
2,3,4,5,6-Pentahydroxy-hexan-säure-[4-(6,8-dichloro-2-methyl-1,2,3,4-tetrahydroisochinolin-4-yl)-phenyl]-amid,
2,3,4,5,6-Pentahydroxy-hexan-säure-[3-(6,8-dichloro-2-methyl-1,2,3,4-tetrahydro-isochinolin-4-yl)-phenyl]-amid,
2,3,4,5,6-Pentahydroxy-hexan-säure-[2-(6,8-dichloro-2-methyl-1,2,3,4-tetrahydro-isochinolin-4-yl)-phenyl]-amid,
2,3,4,5,6-Pentahydroxy-hexan-säure-[3-((S)-6,8-dichloro-2-methyl-1,2,3,4-tetrahydro-isochinolin-4-yl)-phenyl]-amid,
2,3,4,5,6-Pentahydroxy-hexan-säure-[3-((R)-6,8-dichloro-2-methyl-1,2,3,4-tetrahydro-isochinolin-4-yl)-phenyl]-amid,
1-[3-(6,8-Dichlor-2-methyl-1,2,3,4-tetrahydro-isochinolin-4-yl)-phenylj-3-(2-hydroxy-1-hydroxymethyl-ethyl)-harnstoff,
1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff,
1-[3-(6,8-Dichlor-2-methyl-1,2,3,4-tetrahydro-isochinolin-4-yl)-phenyl]-3-(2,3,4,5,6-pentahydroxy-hexyl)-harnstoff,
1-[3-(6,8-Dichlor-2-methyl-1,2,3,4-tetrahydro-isochinolin-4-yl)-phenyl]-3-(2,4,5-trihydroxy-6-hydroxymethyl-tetrahydro-pyran-3-yl)-harnstoff,
{N-[3-(6,8-Dichloro-2-methyl-1,2,3,4-tetrahydro-isochinolin-4-yl)-phenyl]-N'-ethansulfonsäure-2-yl}-harnstoff,
{N-[3-(6,8-Dichloro-2-methyl-1,2,3,4-tetrahydro-isochinolin-4-yl)-phenyl]-N'-(ethyl-2-trimethyl-ammonium}-harnstoff-chlorid,
{N-[3-(6,8-Dichloro-2-methyl-1,2,3,4-tetrahydro-isochinolin-4-yl)-phenyl]-N'-3-hydroxy-propion-säure-2-yl}-harnstoff,
{N-[3-(6,8-Dichloro-2-methyl-1,2,3,4-tetrahydro-isochinolin-4-yl)-phenyl]-N'-bernsteinsäure-4-amid-2-yl}-harnstoff,
3-(6,8-Dichlor-2-methyl-1,2,3,4-tetrahydro-isochinolin-4-yl)-N-(2,3,4,5,6-pentahydroxy-hexyl)-benzamid,
3-(6,8-Dichloro-2-methyl-1,2,3,4-tetrahydro-isochinolin-4-yl)-N-(2-hydroxy-1-hydroxymethyl-ethyl)-benzamid,
2-[3-(6,8-Dichloro-2-methyl-1,2,3,4-tetrahydro-isochinolin-4-yl)-benzoylamino]-3-hydroxy-propionsäure,
2-[3-(6,8-Dichloro-2-methyl-1,2,3,4-tetrahydro-isochinolin-4-yl)-benzoylamino]-bernsteinsäure,
2-[3-(6,8-Dichloro-2-methyl-1,2,3,4-tetrahydro-isochinolin-4-yl)-benzoylamino]-bernsteinsäure-4-amid,
N-[3-(6,8-Dichloro-2-methyl-1,2,3,4-tetrahydro-isochinolin-4-yl)-phenyl]-N'-[5-g uan id i no-penta nsäu re-2-yl]-harnstoff,
{N-[3-(6,8-Dichloro-2-methyl-1,2,3,4-tetrahydro-isochinolin-4-yl)-phenyl]-N'-bernsteinsäure-4-amid-2-yl}-harnstoff,
{N-[3-(6,8-Dichloro-2-methyl-1,2,3,4-tetrahydro-isochinolin-4-yl)-phenyl]-N'-3-hydroxy-propion-säure-2-yl}-harnstoff,
1-[3-(6, 8-Dichloro-2-methyl-1,2, 3,4-tetrahydro-isochinolin-4-yl)-phenyl]-3-(2-hydroxy-1,1-bis-hydroxymethyl-ethyl)-harnstoff,
1-[3-(6,8-Dichlor-2-methyl-1,2,3,4-tetrahydro-isochinolin-4-yl)-phenyl]-3-(2,3,4,5,6-pentahydroxy-hexyl)-harnstoff,
5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid,
5-(6,8-Dichloro-2-methyl-1,2,3,4-tetrahydro-isochinolin-4-yl)-N,N'-bis-(2-hydroxy-1,1-bis-hydroxymethyl-ethyl)-isophthalsäure-amid,
5-(6,8-Dichloro-2-methyl-1,2,3,4-tetrahydro-isochinolin-4-yl)-N-(2-hydroxy-1,1-bis-hydroxymethyl-ethyl)-isophthalsäure-amid,
5-(6,8-Dichlor-2-methyl-1,2,3,4-tetrahydro-isochinolin-4-yl)-N,N'-bis-(2,3,4,5,6-pentahydroxy-hexyl)-isophthalsäure-amid,
5-(6,8-Dichlor-2-methyl-1,2,3,4-tetrahydro-isochinolin-4-yl)-N-(2,3,4,5,6-pentahydroxy-hexyl)-isophthalsäure-amid,
2-[3-(1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure,
2-Amino-5-guanidino-pentansäure-[3-(6,8-dichlor-2-methyl-1,2,3,4-tetrahydro-isochinolin-4-yl)-phenyl]-amid,
2-Amino-5-guanidino-pentansäure-[4-(6,8-dichlor-2-methyl-1,2,3,4-tetrahydro-isochinolin-4-yl)-phenyl]-amid,
2-Amino-N-[3-(6,8-dichloro-2-methyl-1,2,3,4-tetrahydro-isochinolin-4-yl)-phenyl]-3-(1H-imidazol-4-yl)-propionsäure-amid und
2-Amino-N-[4-(6,8-dichloro-2-methyl-1,2,3,4-tetrahydro-isochinolin-4-yl)-phenyl]-3-(1H-imidazol-4-yl)-propionsäure-amid und
[3-(6,8-Dichlor-2-methyl-1,2,3,4-tetrahydro-isochinolin-4(S)-yl)-phenyl]-carbaminsäure-2-methoxy-ethylester;
sowie deren pharmazeutisch verträgliche Salze und Trifluoressigsäuresalze.

Ganz speziell bevorzugt sind Verbindungen der Formel I ausgewählt aus der Gruppe
(2R,3S,4R,5R)-2,3,4,5,6-Pentahydroxy-hexan-säure-[4-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[2-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-((S)-6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-((R)-6,8-dichloro-2-methyl-1,2,3,4-tetrahydro-isochinolin-4-yl)-phenyl]-amid,
1-[3-(6,8-Dichlor-2-methyl-1,2,3,4-tetrahydro-isochinolin-4-yl)-phenyl]-3-(2-hydroxy-1-hydroxymethyl-ethyl)-harnstoff,
1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff,
1-[3-(6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff,
1-[3-(6,8-Dichlor-2-methyl-1,2,3,4-tetrahydro-isochinolin-4-yl)-phenyl]-3-((4R,5S,6R)-2,4,5-trihydroxy-6-hydroxymethyl-tetrahydro-pyran-3-yl)-harnstoff,
{N-[3-(6,8-Dichloro-2-methyl-1,2,3,4-tetrahydro-isochinolin-4-yl)-phenyl]-N'-ethansulfonsäure-2-yl}-harnstoff,
{N-[3-(6,8-Dichloro-2-methyl-1,2,3,4-tetrahydro-isochinolin-4-yl)-phenyl]-N'-(ethyl-2-trimethyl-ammonium}-harnstoff-chlorid,
{N-[3-(6,8-Dichloro-2-methyl-1,2,3,4-tetrahydro-isochinolin-4-yl)-phenyl]-N'-3-hydroxy-propion-säure-2S-yl}-harnstoff,
{N-[3-(6,8-Dichloro-2-methyl-1,2,3,4-tetrahydro-isochinolin-4-yl)-phenyl]-N'-bernsteinsäure-4-amid-2S-yl}-harnstoff,
3-(6,8-Dichlor-2-methyl-1,2,3,4-tetrahydro-isochinolin-4-yl)-N-((2S,3R,4R,5R)-2,3,4,5,6-pentahydroxy-hexyl)-benzamid,
3-(6,8-Dichloro-2-methyl-1,2,3,4-tetrahydro-isochinolin-4-yl)-N-(2-hydroxy-1-hydroxymethyl-ethyl)-benzamid,
(S)-2-[3-(6,8-Dichloro-2-methyl-1,2,3,4-tetrahydro-isochinolin-4-yl)-benzoylamino]-3-hydroxy-propionsäure,
(S)-2-[3-(6,8-Dichloro-2-methyl-1,2,3,4-tetrahydro-isochinolin-4-yl)-benzoylamino]-bernsteinsäure,
(S)-2-[3-(6,8-Dichloro-2-methyl-1,2,3,4-tetrahydro-isochinolin-4-yl)-benzoylamino]-bernsteinsäure-4-amid,
N-[3-(6,8-Dichloro-2-methyl-1,2,3,4-tetrahydro-isochinolin-4-yl)-phenyl]-N'-[5-guanidino-pentansäure-2S-yl]-harnstoff,
{N-[3-(6,8-Dichloro-2-methyl-1,2,3,4-tetrahydro-isochinolin-4(R)-yl)-phenyl]-N'-bernsteinsäure-4-amid-2S-yl}-harnstoff,
{N-[3-(6,8-Dichloro-2-methyl-1,2,3,4-tetrahydro-isochinolin-4(S)-yl)-phenyl]-N'-bernsteinsäure-4-amid-2S-yl}-harnstoff,
{N-[3-(6,8-Dichloro-2-methyl-1,2,3,4-tetrahydro-isochinolin-4(R)-yl)-phenyl]-N'-3-hydroxy-propion-säure-2S-yl}-harnstoff,
{N-[3-(6,8-Dichloro-2-methyl-1,2,3,4-tetrahydro-isochinolin-4(S)-yl)-phenyl]-N'-3-hydroxy-propion-säure-2S-yl}-harnstoff,
1-[3-(6,8-Dichloro-2-methyl-1,2,3,4-tetrahydro-isochinolin-4(R)-yl)-phenyl]-3-(2-hydroxy-1,1-bis-hydroxymethyl-ethyl)-harnstoff,
1-[3-(6,8-Dichloro-2-methyl-1,2,3,4-tetrahydro-isochinolin-4(S)-yl)-phenyl]-3-(2-hydroxy-1,1-bis-hydroxymethyl-ethyl)-harnstoff,
1-[3-((R)-6,8-Dichlor-2-methyl-1,2,3,4-tetrahydro-isochinolin-4-yl)-phenyl]-3-((2S,3R,4R,5R)-2.3,4,5,6-pentahydroxy-hexyl)-harnstoff,
1-[3-((S)-6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff,
5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid,
5-(6,8-Dichloro-2-methyl-1,2,3,4-tetrahydro-isochinolin-4-yl)-N,N'-bis-(2-hydroxy-1,1-bis-hydroxymethyl-ethyl)-isophthalsäure-amid,
5-(6,8-Dichloro-2-methyl-1,2,3,4-tetrahydro-isochinolin-4-yl)-N-(2-hydroxy-1,1-bis-hydroxymethyl-ethyl)-isophthalsäure-amid,
5-(6,8-Dichlor-2-methyl-1,2,3,4-tetrahydro-isochinolin-4-yl)-N,N'-bis-((2S,3R,4R,5R)-2,3,4,5,6-pentahydroxy-hexyl)-isophthalsäure-amid,
5-(6,8-Dichlor-2-methyl-1,2,3,4-tetrahydro-isochinolin-4-yl)-N-((2S,3R,4R,5R)-2,3,4,5,6-pentahydroxy-hexyl)-isophthalsäure-amid,
(S)-2-[3-((S)-1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure,
(S)-2-Amino-5-guanidino-pentansäure-[3-(6,8-dichlor-2-methyl-1,2,3,4-tetrahydro-isochinolin-4-yl)-phenyl]-amid,
(S)-2-Amino-5-guanidino-pentansäure-[4-(6,8-dichlor-2-methyl-1,2,3,4-tetrahydro-isochinolin-4-yl)-phenyl]-amid,
(S)-2-Amino-N-[3-(6,8-dichloro-2-methyl-1,2,3,4-tetrahydro-isochinolin-4-yl)-phenyl]-3-(1 H-imidazol-4-yl)-propionsäure-amid und
(S)-2-Amino-N-[4-(6,8-dichloro-2-methyl-1,2,3,4-tetrahydro-isochinolin-4-yl)-phenyl]-3-(1 H-imidazol-4-yl)-propionsäure-amid;
sowie deren pharmazeutisch verträgliche Salze und Trifluoressigsäuresalze.

Die unter G beschriebenen Gruppen der Form Cₐ(OR32)ₓH₂ₐ₊₁₋ₓ können sowohl geradkettig als auch verzweigt vorliegen. Man gelangt somit zu polyhydroxylierten Alkylketten, wie sie sich beispielsweise von Monosaccharidbausteinen ableiten, die über eine Linkereinheit L an den Phenylrest angebunden sind. Entsprechend beschreibt die Formel C_{b}(OR32)yH_{2b-1-y} bevorzugt polyhydroxylierte, cyclische Alkylsubstituenten. Durch Austausch einer CH₂-Einheit durch O gelangt man zum Beispiel in die Klasse der pyranosiden oder furanosiden Kohlenhydratbausteine, wie in Beispiel 9 verwirklicht. G kann sich ebenso aus der Gruppe der Aminosäuren ableiten, die über die Aminosäureamino- oder die Aminosäurecarboxylfunktion angebunden sind, wobei dabei die Amino- oder Carbonylfunktion zu der Linkereinheit L gezählt wird. Die Aminosäureseitenketten finden sich damit in R38 wieder.

Enthalten die Verbindungen der Formel I ein oder mehrere Asymmetriezentren, so können diese unabhängig voneinander sowohl S als auch R konfiguriert sein. Die Verbindungen können als optische Isomere, als Enantiomere, als Diastereomere, als Racemate oder als Gemische in allen Verhältnissen derselben vorliegen.

Die vorliegende Erfindung umfasst alle tautomeren Formen der Verbindungen der Formeln I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl). Bevorzugte Alkylreste sind Methyl, Ethyl, n-Propyl, Isopropyl, n-Butyl, tert-Butyl. In Alkylresten können ein oder mehrere, zum Beispiel 1, 2, 3, 4, 5, 6, 7, 8 oder 9, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein, zum Beispiel mit Hydroxy. In den Alkylresten können eine oder mehrere CH₂-Gruppen durch O, NH oder N-Alkyl ersetzt sein.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie Phenylalkyl, Phenylcarbonyl, etc. Phenylalkylreste sind zum Beispiel Benzyl, 1-Phenylethyl oder 2-Phenylethyl. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind bevorzugt Imidazolyl, Pyrazolyl, Pyrrolyl, Triazolyl, Tetrazolyl, Thiazolyl und Oxazolyl und Pyridyl.

Als CH₂-Einheiten gelten auch die in einer Alkylkette terminalen CH₃-Gruppen, die in diesem Zusammenhang als CH₂-H Gruppierungen aufgefasst werden.

Beschrieben werden auch Methoden zur Herstellung der Verbindungen der Formel I.

Die hier beschriebenen Verbindungen der Formel I lassen sich ausgehend von den Benzylamin-Vorläufern IV herstellen. Diese wiederum können, falls nicht käuflich erhältlich, nach dem Fachmann bekannten Standardverfahren aus den entsprechenden Benzylchloriden oder -bromiden III und dem entsprechenden Amin synthetisiert werden.

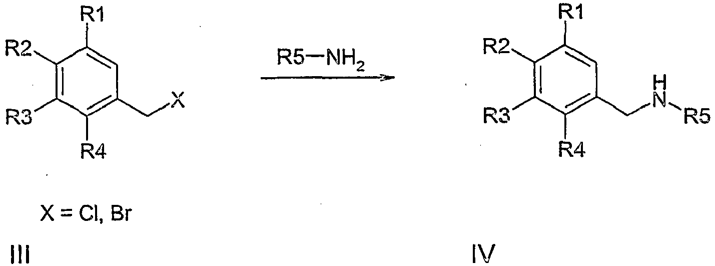

Die so erhaltenen Benzylamine IV, werden in dem Fachmann bekannter Weise mit den entsprechend substituierten alpha-Bromacetophenonverbindungen V alkyliert.

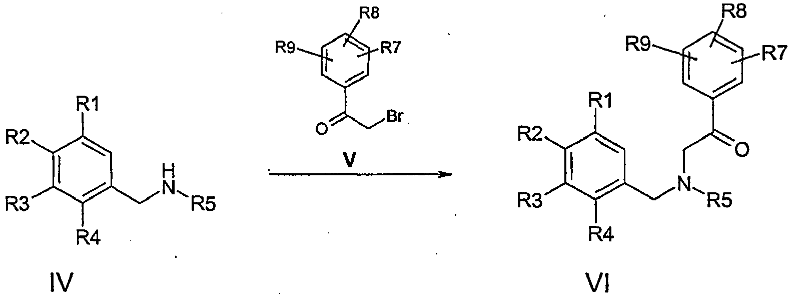

Die alpha-Bromacetophenonverbindungen V lassen sich in literatur-bekannten Verfahren aus den entsprechenden Acetophenonvorläufern durch Bromierung gewinnen.

Durch Reduktion der Carbonylgruppierung in VI und anschließender säurekatalysierter Cyclisierung der entsprechenden Alkohole VII (vgl. Tetrahedron Lett.; 1989, 30, 5837; Org. Prep. Proced. Int.; 1995, 27, 513) können die gewünschten Tetrahydroisochinoline I nach bekannten Verfahren gewonnen werden.

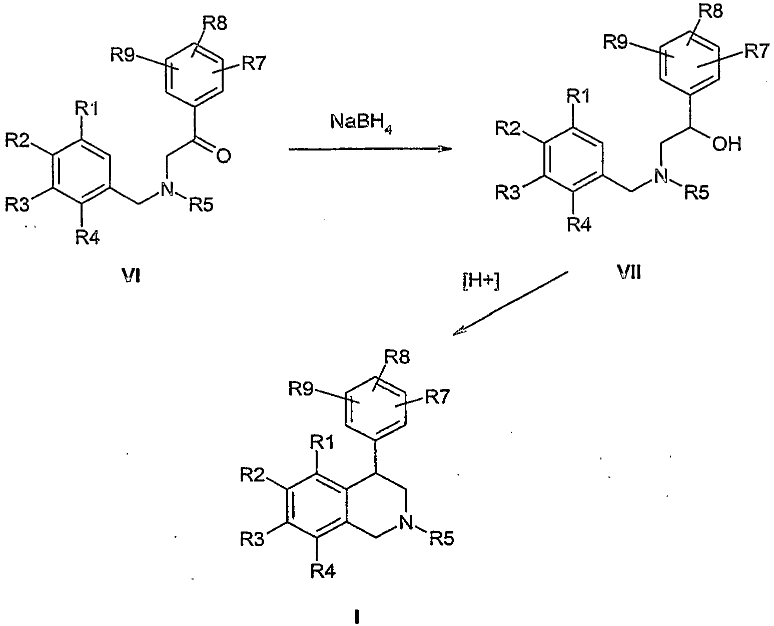

Für R6 ungleich H lassen sich die gewünschten Verbindungen der Formel I zum Beispiel aus den Iodiden VIII (vgl. Chem. Pharm. Bull.; 1994, 42, 67) durch Halogen-Metall-Austausch und anschließendem nucleophilen Angriff der intermediären lithiumorganischen Spezies an der Carbonylgruppe herstellen (vgl. Chem. Pharm. Bull., 1995, 43, 1543).

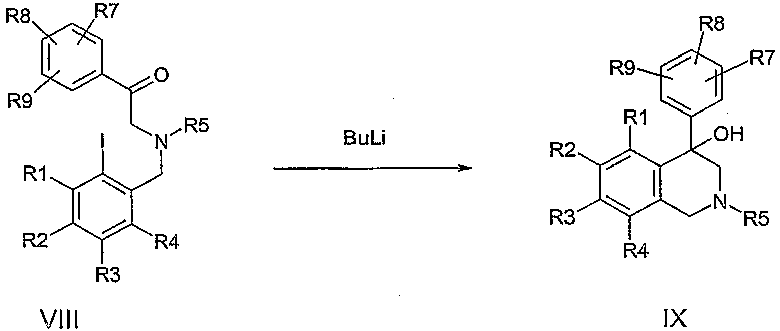

Die dabei synthetisierten tertiären Alkohole IX lassen sich durch bekannte Methoden in weitere Derivate, wie zum Beispiel die daraus abgeleiteten Ether oder Ester überführen.

Zur Herstellung alkylverzweigter Analoge (I) können die entsprechenden Diphenylessigsäureester X in alpha-Stellung nach bekannten Methoden mit R6 alkyliert werden. Die gewünschten Produkte XI können durch Standardverfahren in die entsprechenden Amide XII überführt werden, welche in einer Pictet-Spengler-analogen Reaktion in die gewünschten Tetrahydroisochinoline I überführt werden (vgl. Tetrahedron; 1987, 43, 439; Chem. Pharm. Bull.; 1985, 33, 340).

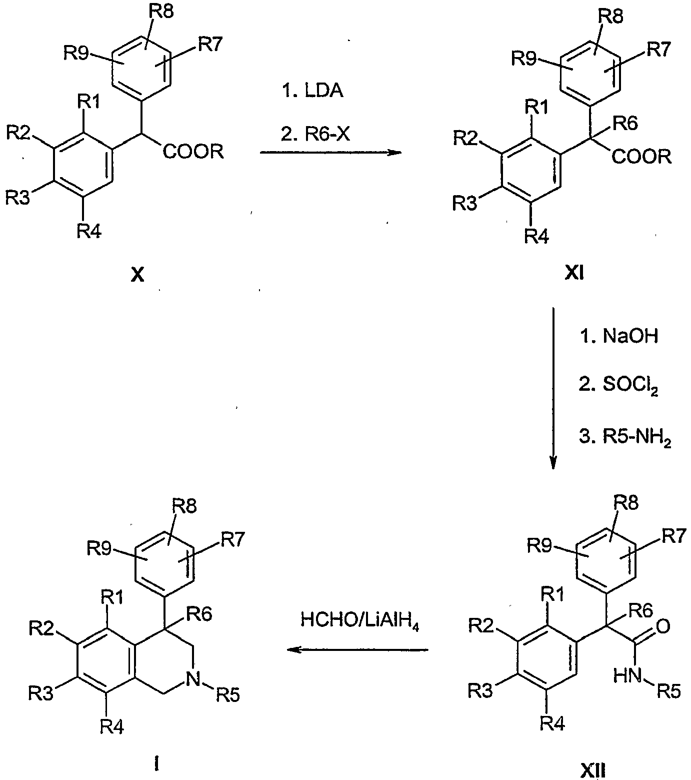

Die Anbindung der Substituenten L-G in die Positionen R7, R8 oder R9 kann beispielsweise durch eine Amidbindung erfolgen. In diesem Falle wird durch die oben beschriebenen Syntheserouten sichergestellt, dass mindestens einer der Reste R7, R8 oder R9 als NH₂- oder COOH-Gruppe vorliegt. Die Anbindung eines polaren Restes kann in dem Fachmann bekannter Weise durch Kupplung polarer Carbonsäuren (zum Beispiel Gluconsäure, die entsprechend geschützt vorliegen muss) mit der NH₂-Verbindung XIII oder durch Kupplung polarer Amine (zum Beispiel Glucamin) mit der entsprechenden --COOH-Verbindung XIV erfolgen, wobei die Carbonsäureanilide Ia, bzw. die Carbonsäureamide Ib entstehen.

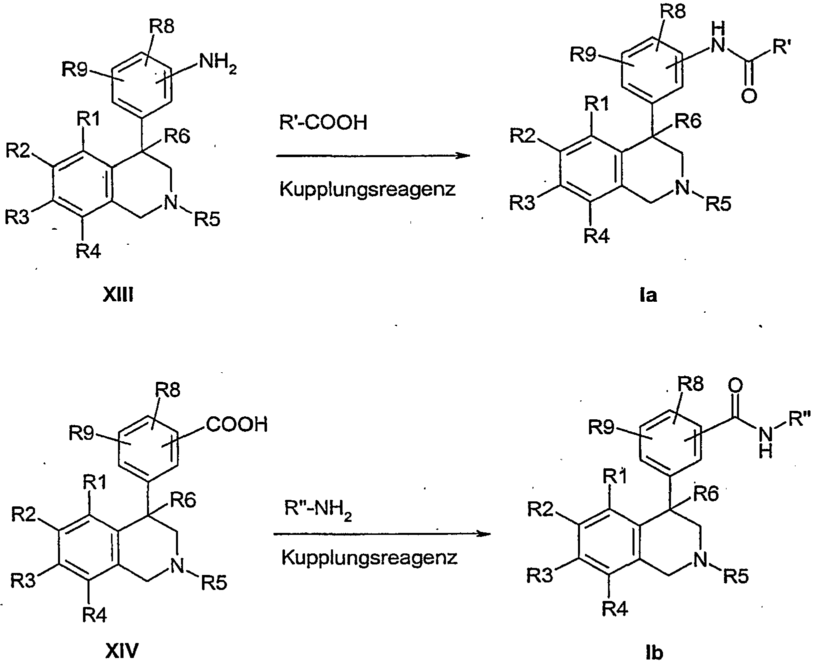

In den Verbindungen der Formeln II bis XIV haben die Reste R1 bis R9 die oben genannte Bedeutung oder es liegen funktionelle Gruppen in geschützter Form oder in Vorstufen vor.

Ebenso lassen sich aus den Vorläufermolekülen XIII in dem Fachmann bekannter Weise die davon abgeleiteten Harnstoff- oder auch Thioharnstoffverbindungen herstellen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen.

Tetrahydroisochinoline als Inhibitoren des Natrium-Wasserstoffaustauschers vom Subtyp 3 (NHE3) sind bereits in der Patentanmeldung WO03048129 beschrieben. In der Patentanmeldung WO03055880 ist die verwandte Verbindungsklasse der Tetrahydroisochinolinium-Salze als NHE3-Inhibitoren beschrieben. Die Eigenschaften dieser Verbindungen sind jedoch in verschiedener Hinsicht noch nicht befriedigend, und es besteht weiterhin Bedarf an Verbindungen mit einem günstigeren pharmakodynamischen bzw. pharmakokinetischen Eigenschaftsprofil, die zur Behandlung unterschiedlichster Krankheiten geeignet sind.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund ihrer unerwarteten NHE-inhibitorischen Eigenschaften eignen sich die Verbindungen der Formel I zur Prävention und Behandlung von Krankheiten, die durch eine Aktivierung bzw. durch einen aktivierten NHE verursacht werden. Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch Ischämie und / oder durch Reperfusion hervorgerufen werden.

Die hier beschriebenen Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der Angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Ausfiauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so dass das Schnarchen unterdrückt wird.

Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Außerdem eignen sich die hier beschriebenen Verbindungen als Medikamente zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelösten klonischen und tonischen Spasmen, psychischen Depressionszuständen, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des Natrium-abhängigen Chlorid-Bicarbonat-Austauschers (NCBE).

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können.

Außerdem können die erfindungsgemäßen Verbindungen vorteilhaft zur Prävention und Therapie von akuten und chronischen Erkrankungen des Darmtrakts verwendet werden, die durch ischämische Zustände im Intestinalbereich und / oder durch nachfolgende Reperfusion ausgelöst werden. Solche Komplikationen können beispielweise durch mangelnde Darmperistaltik hervorgerufen werden, wie sie z.B. häufig nach chirurgischen Eingriffen, bei Darmverstopfung oder stark verminderter Darmtätigkeit zu beobachten sind.

Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferation von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Mittel gegen das chronische Nierenversagen, Krebserkrankungen, fibrotische Erkrankungen des Herzens, sowie Lungenfibrose, Leberfibrose oder Nierenfibrose, Organhypertrophien und -hyperplasien, beispielsweise des Herzens und der Prostata verwendet werden, und somit zur Prävention und Behandlung der Herzinsuffizienz (congestive heart failure) oder bei Prostatahyperplasie bzw. Prostatahypertrophie benutzt werden.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Darüber hinaus sind die Verbindungen der Formel I für die präventive Therapie zur Verhinderung der Genese und zur Behandlung des Bluthochdrucks, beispielweise der essentiellen Hypertonie, geeignet, da sie die Rückresorption von NaCl im tubulären System der Niere vermindern oder vollständig inhibieren. Entsprechend sind sie auch hervorragend als Kombinations- und Formulierungspartner für Arzneimittel geeignet, die zur Bluthochdruckbehandlung verwendet werden. So können sie beispielsweise mit thiazid-artig wirkenden Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren kombiniert werden. Weiterhin können die NHE-Inhibitoren der vorliegenden Erfindung in Kombination mit ACE-Hemmern, wie beispielsweise Ramipril, Enalapril oder Captopril verwendet werden. Weitere günstige Kombinationspartner sind auch. ß-Blocker.

Die beschriebenen NHE-Inhibitoren können ebenfalls in der Prävention und zur Behandlung thrombotischer Erkrankungen verwendet werden, da sie als NHE-Inhibitoren sowohl die Plättchenaggregation selbst inhibieren können und darüber hinaus die überschießende Freisetzung von Gerinnungsmediatoren, insbesondere des von Willebrand Faktors hemmen bzw. verhindern können. Deshalb sind die NHE-Inhibitoren der vorliegenden Erfindung kombinierbar mit weiteren antikoagulativen Wirkstoffen wie beispielsweise Acetylsalizylsäure, Thrombinantagonisten, Faktor Xa-Antagonisten, fibrinolytisch wirkenden Arzneistoffen, Faktor-VIIa-Antagonisten usw. Eine kombinierte Anwendung der vorliegenden NHE-Inhibitoren mit NCBE-Inhibitoren ist besonders günstig.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Die erfindungsgemäßen Inhibitoren des NHE können in günstiger Weise auch mit anderen antiarteriosklerotischen Wirkstoffen kombiniert werden, wie einem Stoff aus der Klasse der Fibrate, einem Upregulator der LD2 Rezeptoraktivität, wie MD-700 und LY295427, oder einem Cholesterol- oder Gallensäure-Resorptionshemmer oder einem Antihyperchlesterolämikum aus der Klasse der Statine, wie beispielsweise Pravastatin, Lovastatin, Simvastatin.

Mit diesem Schutz der Gefäße gegen das Syndrom der endothelialen Dysfunktion sind Verbindungen der Formel I wertvolle Arneimittel zur Prävention und zur Behandlung koronarer Gefäßspasmen, peripherer Gefäßkrankheiten, wie claudicatio intermittens, der Atherogenese und der Atherosklerose, der linksventrikulären Hypertrophie und der dilatierten Kardiomyopathie, und thrombotischer Erkrankungen.

Die genannten Verbindungen können ebenfalls zur Behandlung von Krankheiten verwendet werden, die durch Protozoen verursacht werden und eignen sich insbesondere als Antimalariamittel.

Darüber hinaus eignen sich die Verbindungen zur Bekämpfung saugender Parasiten, wie Moskitos, Zecken, Flöhen und Pflanzenschädlingen.

Entsprechend ihrer protektiven Wirkungen sind die Verbindungen auch als Arzneimittel zur Gesunderhaltung und Lebensverlängerung geeignet.

Generell können die hier beschriebenen NHE-Inhibitoren in günstiger Weise mit anderen, den intrazellulären pH-regulierenden Verbindungen kombiniert werden, wobei Inhibitoren der Enzymgruppe der Carboanhydratase, Inhibitoren der Bicarbonationen transportierenden Systeme wie des Natrium-Bicarbonat-Cotransporters oder des Natrium-abhängigen Chlorid-Bicarbonat-Austauschers, sowie andere NHE-Inhibitoren, beispielweise mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt werden können.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikamentes zur Behandlung diabetischer Spätschäden und chronischer Nierenerkrankungen, insbesondere von allen Nierenentzündungen (Nephritiden), die mit einer vermehrten Protein-/Albuminausscheidung verbunden sind; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern, mit Diuretika, Aldosteron-Antagonisten oder Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arneirnitteln.

Die Erfindung betrifft auch Heilmittel für die humane, veterinäre oder phytoprotektive Anwendung enthaltend eine wirksame Menge einer Verbindung der Formel I und / oder eines pharmazeutisch verträglichen Salzes davon, ebenso wie Heilmittel für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v. Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Verbindungen der Formel I, die eine basische Gruppe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw.

### Versuchsbeschreibungen und Beispiele

### Liste der verwendeten Abkürzungen:

- Rt: Retentionszeit
- TFA: Trifluoressigsäure
- HPLC: High Performance Liquid Chromatography
- eq: Äquivalente
- LCMS: Liquid Chromatography Mass Spectroscopy
- MS: Mass Spectroscopy
- ESI: Elektospray-lonisation
- RT: Raumtemperatur
- THF: Tetrahydrofuran
- TOTU: O-[(Ethoxycarbonyl)-cyanomethylenamino]-N,N,N',N'-tetramethyluronium-tetrafluoroborat
- DMSO: Dimethylsulfoxid
- abs.: absolut(es)
- Zers.: Zersetzung
- DMF: Dimethylformamid
- DMAP: 4-Dimethylaminopyridin
- HOBt: 1-Hydroxybenzotriazol
- DIC: Diisopropylcarbodiimid
- i. Vak.: im Vakuum
- ACN: Acetonitril
- DEA: Diethylamin

### Beispiel 1: (2R,3S,4R,5R)-2,3,4,5,6-Pentahydroxy-hexan-säure-[4-(6,8-dichlor-2-methyl-1,2,3,4-tetrahydro-isochinolin-4-yl)-phenyl]-amid

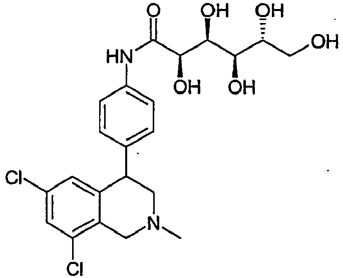

### Zwischenprodukt 1: 2,4-Dichlorbenzyl-methyl-amin

wurde nach literaturbekannten Methoden hergestellt (J. Med. Chem.; 1984, 27, 1111).

### Zwischenprodukt 2: N-[4-(2-Bromo-acetyl)-phenyl]-acetamid

wurde in dem Fachmann bekannter Weise durch Bromierung von N-(4-Acetyl-phenyl)-acetamid synthetisiert.

Die Ausgangsverbindung (0,256 mol) wurde in 300 ml Essigsäure vorgelegt und bei 60 °C eine Lösung von 39,9 g Brom (1,0 eq) in 60 ml Essigsäure zugetropft. Nach 1,5 Stunden wurde auf Raumtemperatur abkühlen lassen und die Reaktionsmischung auf 1 I Eiswasser gegeben. Der Niederschlag wurde abgesaugt, mit Wasser gewaschen und getrocknet, wobei 60 g der Titelverbindung isoliert wurden (Schmp.: 192°C).

### Zwischenprodukt 3: N-{4-[2-(2,4-Dichloro-benzylamino)-acetyl]-phenyl}-acetamid

37,1 g (0,195 mol) des Zwischenprodukts 1 wurden in 400 ml Dioxan vorgelegt und mit einer Lösung aus 60 g (0,234 mol) des Zwischenprodukts 2 in 600 ml Dioxan versetzt. Es wurden 134 ml Triethylamin zugegeben und 4 h bei Raumtemperatur gerührt. Nach Stehen über Nacht wurde der Niederschlag abfiltriert und das Filtrat i. Vak. eingeengt. Der Rückstand wurde in Essigester aufgenommen, mit NaHCO₃ und H₂O gewaschen, mit MgSO₄ getrocknet und eingeengt. Der hierbei anfallende ölige Rückstand wurde mit einem Essigester/Ether-Gemisch verrieben, wobei 36 g des Zwischenprodukt 3 in Form eines kristallinen Feststoffs anfielen (Schmp.: 115-117°C).

### Zwischenprodukt 4: N-{4-[2-(2,4-Dichloro-benzylamino)-1-hydroxy-ethyl]-phenyl}-acetamid

36 g (0,099 mol) des Zwischenprodukts 3 wurden in 500 ml Methanol gelöst und bei 0°C mit 7,8 g (2 eq) Natriumborhydrid versetzt. Es wurde noch 30 min bei 0°C und eine weitere Stunde bei Raumtemperatur nachgerührt. Zur Aufarbeitung wurde der Reaktionsansatz eingeengt und der Rückstand zwischen 1 N HCl und Essigester verteilt. Die wässrige Phase wurde abgetrennt, auf pH 9 eingestellt und zweimal mit Essigester extrahiert. Die vereinigten organischen Phasen wurden mit MgSO₄ getrocknet und eingeengt. Das so erhaltene Rohprodukt konnte ohne weitere Reinigung weiter umgesetzt wurden.

### Zwischenprodukt 5: N-[4-(6,8-Dichloro-2-methyl-1,2,3,4-tetrahydro-isochinolin-4-yl)-phenyl]-acetamid

20 g (0,054 mol) der Zwischenverbindung 4 wurden in 250 ml Dichlormethan gelöst und bei 0°C mit 250 ml konz. H₂SO₄ tropfenweise versetzt. Es wurde 2 h bei 0°C und 1 h bei Raumtemperatur gerührt. Zur Aufarbeitung wurde die Reaktionsmischung auf Eiswasser gegeben und der Niederschlag abgesaugt. Der Niederschlag wurde in 300 ml 1 N NaOH aufgenommen und dreimal mit Essigester extrahiert. Trocknen der organischen Phasen und Einengen lieferte ein Rohprodukt, das mit Diisopropylether verrieben wurde, wobei 11,7 g der Titelverbindung als kristalliner Feststoff isoliert wurden (Schmp.: 205-206°C).

### Zwischenprodukt 6: 4-(6,8-Dichloro-2-methyl-1,2,3,4-tetrahydro-isochinolin-4-yl)-phenylamin

3,0 g (8,6 mmol) N-[4-(6,8-Dichloro-2-methyl-1,2,3,4-tetrahydro-isochinolin-4-yl)-phenyl]-acetamid (Zwischenprodukt 5) wurden in 100 ml 20%iger Natriumethanolat-Lösung gelöst und vier Stunden zum Rückfluss erhitzt. Es wurden weitere 2,0 g (29,4 mmol) festes Natriumethanolat zugegeben und noch drei Stunden unter Rückfluss erhitzt. Zur Aufarbeitung wurde das Lösungsmittel i. Vak. entfernt, der Rückstand in 200 ml H₂O aufgenommen und zweimal mit Dichlormethan extrahiert. Die vereinigten organischen Phasen wurden mit MgSO₄ getrocknet und eingeengt. Zur weiteren Reinigung erfolgte eine Chromatographie an Kieselgel (Essigester/Heptan 1:1), wobei das Anilin in quantitativer Ausbeute als gelbliches Öl erhalten wurde.

### Zwischenprodukt 7: 2,3,4,5,6-Penta-O-acetyl-gluconsäurechlorid

Die Titelverbindung wurde nach literaturbekannten Verfahren synthetisiert (Org. Syntheses, 1961,41, 79-82).

Zwischenprodukt 8: (2R,3S,4R,5R)-2,3,4,5,6-Penta-O-acetyl-hexan-säure-[4-(6,8-dichlor-2-methyl-1,2,3,4-tetrahydro-isochinolin-4-yl)-phenyl]-amid 614 mg (2,0 mmol) 4-(6,8-Dichloro-2-methyl-1,2,3,4-tetrahydro-isochinolin-4-yl)-phenylamin (Zwischenprodukt 6) wurden in 20 ml Pyridin vorgelegt und bei 0 °C mit einer Lösung aus 1,28 g (3,0 mmol) 2,3,4,5,6-Penta-O-acetyl-gluconsäurechlorid in 10 ml Dichlormethan versetzt. Es wurde 15 Minuten bei 0 °C und eine Stunde bei Raumtemperatur gerührt. Zur Aufarbeitung wurde eingeengt und der Rückstand in Dichlormethan aufgenommen. Es wurde einmal mit H₂O, einmal mit gesättigter NaHCO₃-Lösung zweimal mit 1 N HCl und noch einmal mit H₂O gewaschen, mit MgSO₄ getrocknet und eingeengt. Das so erhaltene Rohprodukt (1,12 g) konnte ohne weitere Reinigung in die nächste Reaktion eingesetzt werden.

### 1: (2R,3S,4R,5R)-2,3,4,5,6-Pentahydroxy-hexan-säure-[4-(6,8-dichlor-2-methyl-1,2,3,4-tetrahydro-isochinolin-4-yl)-phenyl]-amid

Das in Zwischenprodukt 8 erhaltene Rohprodukt wurde in 30 ml Methanol gelöst und bei Raumtemperatur mit 452 mg (8,4 mmol) Natriummethanolat portionsweise versetzt. Nach ein bis zwei Stunden bei Raumtemperatur wurde mit 1 N HCl ein pH-Wert von etwa 7 eingestellt und das Lösungsmittel i. Vak. entfernt. Der Rückstand wurde in ges. NaHCO₃-Lösung aufgenommen und zweimal mit Essigester extrahiert. Die vereinten organischen Phasen wurden mit MgSO₄ getrocknet und eingeengt. Chromatographie an Kieselgel mit einem Dichlormethan/Methanol-Gemisch lieferte 373 mg der Titelverbindung als leicht gelben Feststoff.

### 1a: (2R,3S,4R,5R)-2,3,4,5,6-Pentahydroxy-hexan-säure-[4-(6,8-dichlor-2-methyl-1,2,3,4-tetrahydro-isochinolin-4-yl)-phenyl]-amid-Hydrochlorid

201 mg (0,4 mmol) (2R,3S,4R,5R)-2,3,4,5,6-Pentahydroxy-hexan-säure-[4-(6,8-dichlor-2-methyl-1,2,3,4-tetrahydro-isochinolin-4-yl)-phenyl]-amid (Beispielverbindung 1) wurden in 75 ml H₂O aufgenommen und bei Raumtemperatur mit 4,14 ml einer 0,1 M HCl versetzt. Es wurde 15 Minuten gerührt, filtriert und gefriergetrocknet, wobei 177 mg des gewünschten Hydrochlorides erhalten wurden.

### Beispiel 2: (2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid

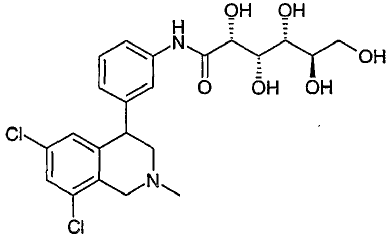

### Zwischenprodukt 1: 3-(6,8-Dichloro-2-methyl-1,2,3,4-tetrahydro-isochinolin-4-yl)-phenylamin

Ausgehend von 2,4-Dichlorbenzyl-methyl-amin (Beispiel 1, Zwischenprodukt 1) und N-[3-(2-Bromo-acetyl)-phenyl]-acetamid (vgl. Beispiel 1, Zwischenprodukt 2) wurde das Anilinderivat 3-(6,8-Dichloro-2-methyl-1,2,3,4-tetrahydro-isochinolin-4-yl)-phenylamin (vgl. Beispiel 1, Zwischenprodukt 6) analog zu der in Beispiel 1 beschriebenen Syntheseroute dargestellt.

### Zwischenprodukt 2: (2R,3S,4R,5R)-2,3,4,5,6-Penta-O-acetyl-hexan-säure-[3-(6,8-dichloro-2-methyl-1,2,3,4-tetrahydro-isochinolin-4-yl)-phenyl]-amid

3,1 g (10 mmol) 3-(6,8-Dichloro-2-methyl-1,2,3,4-tetrahydro-isochinolin-4-yl)-phenylamin (Zwischenprodukt 1) wurden in 100 ml Pyridin gelöst und bei 0 °C mit einer Lösung aus 1,2 Äquivalenten 2,3,4,5,6-Penta-O-acetyl-gluconsäurechlorid in 60 ml Dichlormethan versetzt. Es wurde bei Raumtemperatur gerührt. Nachdem die Reaktionskontrolle vollständigen Umsatz anzeigte, wurde eingeengt und der Rückstand in Dichlormethan aufgenommen. Es wurde einmal mit H₂O, einmal mit gesättigter NaHCO₃-Lösung zweimal mit 1 N HCl und noch einmal mit H₂O gewaschen, mit MgSO₄ getrocknet und eingeengt. Das so erhaltene Rohprodukt (6,91 g) konnte ohne weitere Reinigung in die nächste Reaktion eingesetzt werden.

### 2: (2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid

6,91 g des Rohprodukts aus Zwischenprodukt 2 wurden in 200 ml Methanol bei Raumtemperatur mit 2,79 g (51,7 mmol) Natriummethanolat versetzt. Nach ein bis zwei Stunden bei Raumtemperatur wurde mit 1 N HCl ein pH-Wert von etwa 7 eingestellt und das Lösungsmittel i. Vak. entfernt. Der Rückstand wurde in ges. NaHCO₃-Lösun aufgenommen und zweimal mit Essigester extrahiert. Die vereinten organischen Phasen wurden mit MgSO₄ getrocknet und eingeengt. Chromatographie an Kieselgel mit einem Dichlormethan/Methanol-Gemisch lieferte 2,05 g der Titelverbindung als leicht gelben Feststoff.

### 2a: (2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid-Hydrochlorid

300 mg (0,6 mmol) (2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid (Beispielverbindung 2) wurden in 100 ml H₂O aufgenommen und bei Raumtemperatur mit 6,18 ml einer 0,1 M HCl versetzt. Es wurde 15 Minuten gerührt, filtriert und gefriergetrocknet, wobei 294 mg des gewünschten Hydrochlorids erhalten wurden.

### Beispiel 3: (2R,3S,4R,5R)-2,3,4,5,6-Pentahydroxy-hexan-säure-[2-(6,8-dichloro-2-methyl-1,2,3,4-tetrahydro-isochinolin-4-yl)-phenyl]-amid

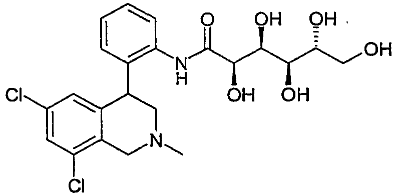

### Zwischenprodukt 1: N-[2-(2-Bromo-acetyl)-phenyl]-acetamid

31 g (0.175 mol) N-(2-Acetylphenyl)-acetamid (hergestellt durch Acylieren von 2-Aminoacetophenon mit Acetylchlorid nach Fuerstner, Alois; Jumbam, Denis N.; Tetrahedron; 48; 29; 5991-6010, (1992)) wurden in 200 ml Eisessig gelöst. Man gab 127 ml 33%-iger HBr in Eisessig zu und ließ dann bei Raumtemperatur 8,75 ml (0,175 mol) Brom langsam zulaufen. Der Ansatz wurde über Nacht bei Raumtemperatur gerührt. Der Ansatz wurde in 1,5 L Eiswasser eingerührt, das ausgefallene Produkt wurde abgesaugt, gut mit Eiswasser nachgewaschen und im Vakuum getrocknet. Das Rohprodukt enthielt laut HPLC und NMR etwas Edukt und doppelt bromiertes Produkt, war aber für die weitere Umsetzung sauber genug (ca. 85%ig).
Ausbeute: 43 g

### Zwischenprodukt 2: N-(2-{2-[(2,4-Dichloro-benzyl)-methyl-amino]-acetyl}-phenyl)-acetamid

12,4 g (65,24 mmol) 2,4-Dichlorbenzyl-methyl-amin (Beispiel 1, Zwischenprodukt 1) wurden in 200 ml Dioxan gelöst. Dazu gab man 19,96 g des Rohproduktes der vorstehenden Bromierung, ebenfalls in 200 ml Dioxan gelöst, und 45 ml Triethylamin. Der Ansatz wurde über Nacht bei Raumtemperatur gerührt und dann filtriert. Das Filtrat wurde evaporiert, der Rückstand in Essigester aufgenommen und mit gesättigter Natriumhydrogencarbonat- und Kochsalzlösung gewaschen, über Natriumsulfat getrocknet und einrotiert. Das Rohprodukt (20,4 g) war laut NMR für die weitere Umsetzung sauber genug.

### Zwischenprodukt 3: N-(2-{2-[(2,4-Dichloro-benzyl)-methyl-amino]-1-hydroxy-ethyl}-phenyl)-acetamid

20 g des Rohproduktes der vorhergehenden Stufe (ca. 50 mmol) wurden in 200 ml Methanol gelöst und im Eisbad auf < 5 °C gekühlt. Dazu gab man unter gutem Rühren portionsweise 4,3 g (109 mmol) Natriumborhydrid, so dass die Innentemperatur 10 °C nicht überschreitet. Anschließend wurde noch 30 Min. im Eisbad und 1 Stunde bei Raumtemperatur nachgerührt. Nach Stehen über Nacht wurde der Ansatz evaporiert, der Rückstand in Essigester aufgenommen, 3x mit Wasser und 1x mit Kochsalzlösung gewaschen, über Natriumsulfat getrocknet und einrotiert. Das Rohprodukt (19,4 g) wurde ohne Reinigung weiter umgesetzt.

### Zwischenprodukt 4: 1-(2-Amino-phenyl)-2-[(2,4-dichloro-benzyl)-methyl-amino]-ethanol

10g des Rohproduktes aus der vorhergehenden Stufe wurden in 300 ml Methanol gelöst. Man gab 200 ml konzentrierte Salzsäure zu und rührt 10 Stunden bei 50 °C. Man ließ abkühlen, goss den Ansatz in Wasser und stellt den pH-Wert mit 20%-iger NaOH auf 10-12 ein. Das Produkt wurde mit Essigester extrahiert, die vereinigten Extrakte mit Kochsalzlösung gewaschen, über Natriumsulfat getrocknet und evaporiert. Das Rohprodukt (9,9 g) enthielt etwas Kochsalz, was jedoch für die weitere Umsetzung nicht störte.

### Zwischenprodukt 5: 2-(6,8-Dichloro-2-methyl-1,2,3,4-tetrahydro-isochinolin-4-yl)-phenylamin

9,9 g des Rohproduktes aus der vorhergehenden Stufe wurden in 350 ml Chloroform gelöst. Unter Kühlung im Eisbad ließ man 123 ml konzentrierte Schwefelsäure zutropfen. Man rührt 2 Stunden im Eisbad nach, ließ dann langsam auf Raumtemperatur kommen und erhitzte schließlich über Nacht auf 50 °C. Der abgekühlte Ansatz wurde auf Eis gegossen und mit Natronlauge alkalisch gestellt (pH > 10). Die organische Phase wurde abgetrennt, die wässrige Phase 2x mit Methylenchlorid extrahiert, die vereinigten organischen Phasen wurden mit Wasser und NaCl gewaschen, über Natriumsulfat getrocknet und evaporiert.

### Zwischenprodukt 6: (2R,3S,4R,5R)-2,3,4,5,6-Penta-O-acetyl-hexan-säure-[2-(6,8-dichloro-2-methyl-1,2,3,4-tetrahydro-isochinolin-4-yl)-phenyl]-amid

614 mg (2,0 mmol) 2-(6,8-Dichloro-2-methyl-1,2,3,4-tetrahydro-isochinolin-4-yl)-phenylamin (Zwischenprodukt 5) wurden in 20 ml Pyridin gelöst und bei 0 °C mit einer Lösung aus 1,5 Äquivalenten 2,3,4,5,6-Penta-O-acetyl-gluconsäurechlorid in 10 ml Dichlormethan versetzt. Es wurde bei Raumtemperatur gerührt. Nachdem die Reaktionskontrolle vollständigen Umsatz anzeigte, wurde eingeengt und der Rückstand in Dichlormethan aufgenommen. Es wurde einmal mit H₂O, einmal mit gesättigter NaHCO₃-Lösung zweimal mit 1 N HCl und noch einmal mit H₂O gewaschen, mit MgSO₄ getrocknet und eingeengt. Das so erhaltene Rohprodukt (1,27 g) konnte ohne weitere Reinigung in die nächste Reaktion eingesetzt werden.

### 3: (2R,3S,4R,5R)-2,3,4,5,6-Pentahydroxy-hexan-säure-[2-(6,8-dichloro-2-methyl-1,2,3,4-tetrahydro-isochinolin-4-yl)-phenyl]-amid

1,27 g des Rohprodukts aus Zwischenprodukt 6 wurden in 30 ml Methanol bei Raumtemperatur mit 513 mg (9,5 mmol) Natriummethanolat versetzt. Nach ein bis zwei Stunden bei Raumtemperatur wurde mit 1 N HCl ein pH-Wert von etwa 7 eingestellt und das Lösungsmittel i. Vak. entfernt. Der Rückstand wurde in ges. NaHCO₃-Lösung aufgenommen und zweimal mit Essigester extrahiert. Die vereinten organischen Phasen wurden noch einmal mit H₂O gewaschen, mit MgSO₄ getrocknet und eingeengt. Chromatographie an Kieselgel mit einem Dichlormethan/Methanol-Gemisch lieferte 313 mg der Titelverbindung als leicht gelben Feststoff.

### 3a: (2R,3S,4R,5R)-2,3,4,5,6-Pentahydroxy-hexan-säure-[2-(6,8-dichloro-2-methyl-1,2,3,4-tetrahydro-isochinolin-4-yl)-phenyl]-amid-Hydrochlorid

145,5 mg (0,3 mmol) 2,3,4,5,6-Pentahydroxy-gluconsäure-[2-(6,8-dichloro-2-methyl-1,2,3,4-tetrahydro-isochinolin-4-yl)-phenyl]-amid (Beispielverbindung 3) wurden in 75 ml H₂O aufgenommen und bei Raumtemperatur mit 3,0 ml einer 0,1 M HCl versetzt. Es wurde 15 Minuten gerührt, filtriert und gefriergetrocknet, wobei 149 mg des gewünschten Hydrochlorides erhalten wurden.

### Beispiel 4:

### 4a: (R)-(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid und

### 4b: (S)-(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid

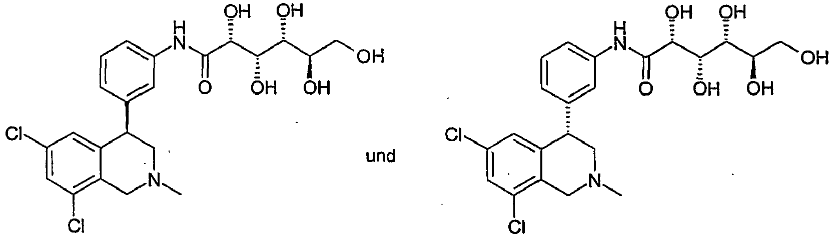

2,0 g der Beispielverbindung 2 wurden an einer chiralen Phase in die beiden Diastereomeren getrennt.

Präparative Trennbedingungen bei Basislinientrennung:

| | |
|---|---|
| Chirale Säule: | Chiralpak AD9 250 x 50 mm + Chiralpak AD2 250 x 50 mm |
| Solvent: | Heptan:Ethanol:Methanol 2:1:1 |
| Flußrate: | 150 ml/min |

Analytische Daten an einer chiralen Phase:

| | |
|---|---|
| Chirale Säule: | Chiralpak ADH/40 250 x 4,6 |
| Solvent: | Heptan:Ethanol:Methanol 2:1:1 |
| Flußrate: | 1 ml/min |
| Temperatur: | 30 °C |

Retentionszeit des Diastereomers A: 4,5 Minuten,
Ausbeute des Diastereomers A: 988 mg;
Retentionszeit des Diastereomers B: 7,5 Minuten,
Ausbeute des Diastereomers B: 942 mg.

### Beispiel 5: (R oder S)-(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid hydrochlorid

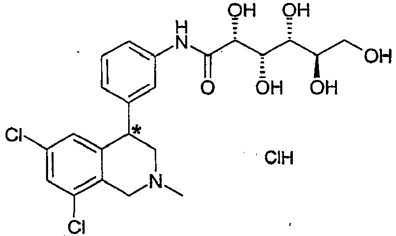

300 mg (0,6 mmol) (R oder S)-(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid (Beispielverbindung 4a oder 4b; Diastereomer B) wurden in 20 ml H₂O aufgenommen und bei Raumtemperatur mit 6,18 ml einer 0,1 M HCl versetzt. Es wurde 15 Minuten gerührt, filtriert und gefriergetrocknet, wobei 297 mg des gewünschten Hydrochlorides erhalten wurden.

### Beispiel 6: 1-[3-(6,8-Dichlor-2-methyl-1,2,3,4-tetrahydro-isochinolin-4-yl)-phenyl]-3-(2-hydroxy-1-hydroxymethyl-ethyl)-harnstoff

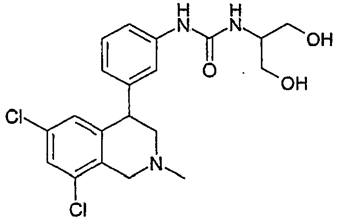

### Zwischenprodukt 1: [3-(6,8-Dichloro-2-methyl-1,2,3,4-tetrahydro-isochinolin-4-yl)-phenyl]-carbaminsäure-4-nitro-phenylester-Hydrochlorid

350 mg (1,1 mmol) 3-(6,8-Dichloro-2-methyl-1,2,3,4-tetrahydro-isochinolin-4-yl)-phenylamin (Beispielverbindung 2, Zwischenprodukt 1) wurden in 17,5 ml Dichlormethan gelöst und unter Rühren mit 230 mg (1,1 mmol) Chlorameisensäure-4-nitrophenylester versetzt. Nach 4,5 Stunden wurden weitere 0,1 Äquivalente (23 mg) Chlorameisensäure-4-nitrophenylester zugegeben und die Lösung über Nacht gerührt. Zur Aufarbeitung wurde der entstandene Niederschlag abfiltriert und mit Dichlormethan gewaschen. Das so erhaltene Rohprodukt konnte ohne weitere Reinigung weiter umgesetzt werden.

### 6: 1-[3-(6,8-Dichloro-2-methyl-1,2,3,4-tetrahydro-isochinolin-4-yl)-phenyl]-3-(2-hydroxy-1-hydroxymethyl-ethyl)-harnstoff

1,02 g (2,0 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1) wurden in 30 ml abs. DMF gelöst und bei 0 °C mit einer Lösung aus 200,5 mg (2,2 mmol) 2-Amino-1,3-propandiol in 25 ml abs. DMF versetzt. Es wurde 3 Stunden bei Raumtemperatur gerührt. Nach Stehen über Nacht wurde das Lösungsmittel i. Vak. entfernt und der Rückstand zwischen Essigester und gesättigter NaHCO₃-Lösung verteilt. Die organische Phase wurde abgetrennt und die wässrige noch zweimal mit Essigester extrahiert. Die vereinten organischen Phasen wurden mit gesättigter NaCl-Lösung gewaschen, mit Na₂SO₄ getrocknet und eingeengt. Chromatographie des so erhaltenen Rohprodukt an Kieselgel (Dichlormethan/Methanol-Gemisch) lieferte 500 mg des gewünschten Harnstoffs.

### 6a: 1-[3-(6,8-Dichloro-2-methyl-1,2,3,4-tetrahydro-isochinolin-4-yl)-phenyl]-3-(2-hydroxy-1-hydroxymethyl-ethyl)-harnstoff-Hydrochlorid

200 mg 1-[3-(6,8-Dichloro-2-methyl-1,2,3,4-tetrahydro-isochinolin-4-yl)-phenyl]-3-(2-hydroxy-1-hydroxymethyl-ethyl)-harnstoff (Beispielverbindung 6) wurden in 40 ml einer 0,1 M HCl gelöst, filtriert und gefriergetrocknet, wobei 194 mg des gewünschten Hydrochlorids erhalten wurden.

### Beispiel 7: 1-[3-(6,8-Dichlor-2-methyl-1,2,3,4-tetrahydro-isochinolin-4-yl)-phenyl]-3-(2-hydroxy-1,1-bis-hydroxymethyl-ethyl)-harnstoff

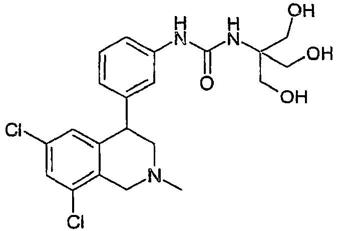

1,02 g (2,0 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden mit 2-Amino-2-hydroxymethyl-propan-1,3-diol analog zu der in Beispiel 6 beschriebenen Weise umgesetzt. Analoge Aufarbeitung lieferte 395 mg der Titelverbindung.

### 7a: 1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff-Hydrochlorid

Analoges Vorgehen zu der in Beispiel 6a beschriebenen Methode lieferte ausgehend von 200 mg 1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff (Beispielverbindung 7) 195 mg des gewünschten Hydrochlorids.

### Beispiel 8: 1-[3-(6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff

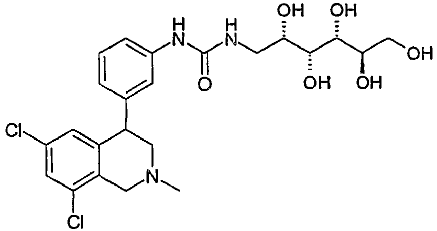

1,02 g (2,0 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden mit D-Glucamin analog zu der in Beispiel 6 beschriebenen Weise umgesetzt. Analoge Aufarbeitung lieferte 273 mg der Titelverbindung.

### 8a: 1-[3-(6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff-Hydrochlorid

Analoges Vorgehen zu der in Beispiel 6a beschriebenen Methode lieferte ausgehend von 200 mg 1-[3-(6,8-Dichloro-2-methyl-1,2,3,4-tetrahydro-isochinolin-4-yl)-phenyl]-3-(2S,3R,4R,5R,6-pentahydroxy-hexyl)-harnstoff (Beispielverbindung 8) 181 mg des gewünschten Hydrochlorids.

### Beispiel 9: 1-[3-(6,8-Dichlor-2-methyl-1,2,3,4-tetrahydro-isochinolin-4-yl)-phenyl]-3-((4R,5S,6R)-2,4,5-trihydroxy-6-hydroxymethyl-tetrahydro-pyran-3-yl)-harnstoff

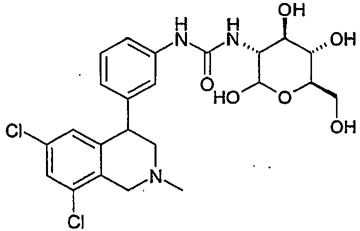

254 mg (0,5 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden in 7 ml abs. DMF vorgelegt und bei 0 °C mit einer Suspension von 119 mg (0,55 mmol) D-Glucosamin-Hydrochlorid in 5 ml abs. DMF versetzt. Analoges Vorgehen wie zu der in Beispiel 6 beschriebenen Methode lieferte nach Chromatographie an Kieselgel 87 mg der Titelverbindung.

### Beispiel 10: {N-[3-(6,8-Dichloro-2-methyl-1,2,3,4-tetrahydro-isochinolin-4-yl)-phenyl]-N'-ethansulfonsäure-2-yl}-harnstoff-Hydrochlorid

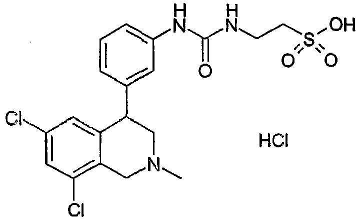

1,02 g (2,0 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden in 30 ml abs. DMF bei 0 °C mit 275 mg (2,2 mmol) 2-Aminoethansulfonsäure, sowie 0,83 ml (6,0 mmol) Triethylamin versetzt und 2 Stunden bei Raumtemperatur gerührt. Nach Stehen über Nacht wurde vom entstandenen Niederschlag abfiltriert und i. Vak. eingeengt. Der Rückstand wurde in gesättigter NaHCO₃-Lösung aufgenommen, filtriert und mit 1 N HCl neutralisiert, wobei ein Niederschlag ausfiel. Abfiltrieren und Trocknen lieferte 356 mg der Titelverbindung als Rohprodukt. Die Mutterlauge wurde gefriergetrocknet und der Rückstand mit Dichlormethan verrieben. Der unlösliche Rückstand (634 mg) wurde mit dem bereits erhaltenen Niederschlag (356 mg) vereinigt und an Kieselgel chromatographiert. Nach einer weiteren Reinigung an einer präparativen HPLC wurden die Produktfraktionen vereinigt und gefriergetrocknet. Das so erhaltene Produkt wurde in 1 N HCl gelöst und noch mal gefriergetrocknet, wobei das gewünschte Hydrochlorid erhalten wurde. Nochmaliges Lösen in H₂O und eine weitere Gefriertrocknung lieferte 271 mg der Titelverbindung.

### Beispiel 11: {N-[3-(6,8-Dichloro-2-methyl-1,2,3,4-tetrahydro-isochinolin-4-yl)-phenyl]-N'-(ethyl-2-trimethyl-ammonium}-harnstoff-chlorid-Hydrochlorid

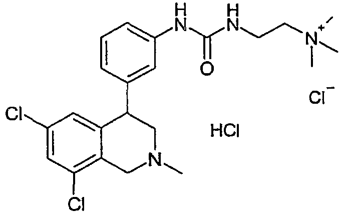

1,02 g (2,0 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden in 30 ml abs. DMF gelöst und bei 0 °C mit einer Lösung aus 333 mg (1,9 mmol) 2-Aminoethyl-trimethylammoniumchlorid-Hydrochlorid in 5 ml DMF versetzt. Nach Zugabe von 0,277 ml (2,0 mmol) Triethylamin wurde 4 Stunden bei Raumtemperatur gerührt. Nach Stehen über Nacht wurde filtriert und i. Vak. eingeengt. Der Rückstand wurde in H₂O aufgenommen und gefriergetrocknet, wobei 1,77 g Rohprodukt erhalten wurden. Reinigung an einer präparativen HPLC lieferte das gewünschte Produkt, welches durch Lösen in 1 N HCl und Gefriertrocknung in die Titelverbindung überführt werden konnte. Nach nochmaligem Lösen in H₂O und einer weiteren Gefriertrocknung wurden 544 mg des gewünschten Hydrochlorids erhalten.

### Beispiel 12: {N-[3-(6,8-Dichloro-2-methyl-1,2,3,4-tetrahydro-isochinolin-4-yl)-phenyl]-N'-3-hydroxy-propion-säure-2S-yl}-harnstoff

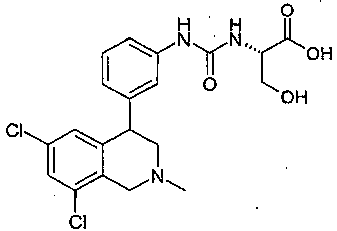

### Zwischenprodukt 1: {N-[3-(6,8-Dichloro-2-methyl-1,2,3,4-tetrahydro-isochinolin-4-yl)-phenyl]-N'-3-hydroxy-propion-säure-ethyl-ester-2S-yl}-harnstoff

254 mg (0,5 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden in 7 ml abs. DMF gelöst und bei 0 °C mit einer Lösung aus 93 mg (0,55 mmol) (S)-Serinethylester-Hydrochlorid in 5 ml abs. DMF versetzt. Nach Zugabe von 104 µl (0,75 mmol) Triethylamin wurde bei Raumtemperatur gerührt. Nach Stehen über Nacht wurde nach der in Beispiel 6 beschrieben Weise aufgearbeitet, wobei 184 mg der Titelverbindung erhalten wurden.

### 12: {N-[3-(6,8-Dichloro-2-methyl-1,2,3,4-tetrahydro-isochinolin-4-yl)-phenyl]-N'-3-hydroxy-propion-säure-2S-yl}-harnstoff

170 mg (0,36 mmol) {N-[3-(6,8-Dichloro-2-methyl-1,2,3,4-tetrahydro-isochinolin-4-yl)-phenyl]-N'-3-hydroxy-propion-säure-ethyl-ester-2S-yl}-harnstoff (Zwischenprodukt 1) wurden in 2 ml Methanol gelöst und mit 2 ml einer 2 M KOH versetzt. Nach 3 Stunden bei Raumtemperatur wurde eingeengt und der Rückstand in H₂O aufgenommen. Nachdem durch Zugabe von verdünnter HCl ein pH-Wert von ca. 7 erreicht war, wurde vom entstandenen Niederschlag abfiltriert. Trocknen lieferte 89 mg der Titelverbindung.

### 12a: {N-[3-(6,8-Dichloro-2-methyl-1,2,3,4-tetrahydro-isochinolin-4-yl)-phenyl]-N'-3-hydroxy-propion-säure-2S-yl}-harnstoff-Hydrochlorid

51 mg {N-[3-(6,8-Dichloro-2-methyl-1,2,3,4-tetrahydro-isochinolin-4-yl)-phenyl]-N'-3-hydroxy-propion-säure-2S-yl}-harnstoff (Beispielverbindung 12) wurden in 20 ml 0,1 M HCl gelöst und gefriergetrocknet, wobei 52 mg des gewünschten Hydrochlorids erhalten wurden.

### Beispiel 13: {N-[3-(6,8-Dichloro-2-methyl-1,2,3,4-tetrahydro-isochinolin-4-yl)-phenyl]-N'-bernsteinsäure-4-amid-2S-yl}-harnstoff-Hydrochlorid

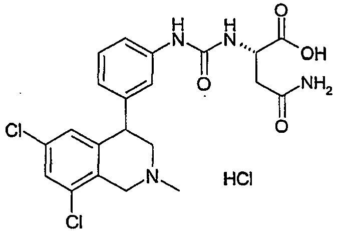

### Zwischenprodukt 1: {N-[3-(6,8-Dichloro-2-methyl-1,2,3,4-tetrahydro-isochinolin-4-yl)-phenyl]-N'-bernsteinsäure-4-amid-1-tert-butylester-2S-yl}-harnstoff

254 mg (0,5 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden mit 124 mg (0,55 mmol) (S)-Asparagin-tert-butylester-Hydrochlorid analog zu der in Beispiel 12 beschriebenen Methode umgesetzt, wobei 220 mg der Titelverbindung erhalten wurden.

### 13: {N-[3-(6,8-Dichloro-2-methyl-1,2,3,4-tetrahydro-isochinolin-4-yl)-phenyl]-N'-bernstainsäure-4-amid-2S-yl}-harnstoff-Hydrochlorid

210 mg (0,4 mmol) des Zwischenproduktes 1 wurden in 3 ml Trifluoressigsäure gelöst und 3 Stunden bei Raumtemperatur stehen gelassen. Anschließend wurde eingeengt, der Rückstand mit Ether verrieben und abgesaugt, wobei 230 mg des entsprechenden Trifluoracetats erhalten wurden. Dieses wurde durch Lösen in 0,1 N HCl und anschließende Gefriertrocknung in das gewünschte Hydrochlorid überführt.

### Beispiel 14: 3-(6,8-Dichlor-2-methyl-1,2,3,4-tetrahydro-isochinolin-4-yl)-N-((2S,3R,4R,5R)-2,3,4,5,6-pentahydroxy-hexyl)-benzamid-Hydrochlorid

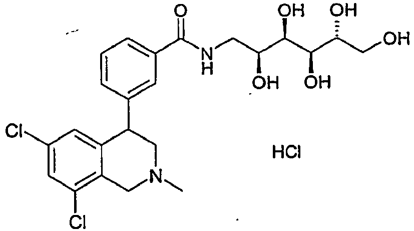

### Zwischenprodukt 1: 3-Acetyl-benzoesäure

Die Titelverbindung wurde in dem Fachmann bekannter Weise aus 3-Acetylbenzonitril durch Verseifung der Nitrilgruppe hergestellt.

### Zwischenprodukt 2: 3-Acetyl-benzoesäure-ethylester

Die Titelverbindung wurde durch säurekatalysierte Veresterung aus 3-Acetyl-benzoesäure (Zwischenprodukt 1) in dem Fachmann bekannter Weise hergestellt.

### Zwischenprodukt 3: 3-(2-Brom-acetyl)-benzoesäure-ethylester

Die Titelverbindung wurde analog zu der in Beispiel 1, Zwischenprodukt 2 beschriebenen Methode aus 3-Acetyl-benzoesäure-ethylester (Zwischenprodukt 2) synthetisiert.

### Zwischenprodukt 4: 3-(6,8-Dichloro-2-methyl-1,2,3,4-tetrahydro-isochinolin-4-yl)-benzoesäure-ethylester

Analog zu der unter Beispiel 1 beschriebenen Syntheseroute wurde ausgehend von 3-(2-Brom-acetyl)-benzoesäure-ethylester (Zwischenprodukt 3) und 2,4-Dichlorbenzyl-methyl-amin (Beispiel 1, Zwischenprodukt 1) weiterverfahren, wobei nach Alkylierungsreaktion, Reduktion und Ringschlußreaktion der 3-(6,8-Dichloro-2-methyl-1,2,3,4-tetrahydro-isochinolin-4-yl)-benzoesäure-ethylester erhalten wurde.

### Zwischenprodukt 5: 3-(6,8-Dichloro-2-methyl-1,2,3,4-tetrahydro-isochinolin-4-yl)-benzoesäure

3,3 g 3-(6,8-Dichloro-2-methyl-1,2,3,4-tetrahydro-isochinolin-4-yl)-benzoesäure-ethylester (Zwischenprodukt 4) wurden in 60 ml Methanol gelöst und mit 60 ml 2 N KOH versetzt. Nach zwei Stunden bei 50 °C wurde i. Vak. eingeengt und der Rückstand zwischen Wasser und Ether verteilt. Die Wasserphase wurde mit 2 N HCl auf einen pH-Wert von ca. 6 eingestellt und der entstandene Niederschlag abgesaugt. Trocknen lieferte 1,7 g der Titelverbindung als farblosen Feststoff.

### 14: 3-(6,8-Dichlor-2-methyl-1,2,3,4-tetrahydro-isochinolin-4-yl)-N-((2S,3R,4R,5R)-2,3,4,5,6-pentahydroxy-hexyl)-benzamid-Hydrochlorid

300 mg (0,9 mmol) 3-(6,8-Dichloro-2-methyl-1,2,3,4-tetrahydro-isochinolin-4-yl)-benzoesäure (Zwischenprodukt 5) wurden in 10 ml DMF vorgelegt und bei 0 °C mit 0,137 ml (1,0 mmol) Triethylamin, sowie 354 mg (1,1 mmol) TOTU versetzt. Es wurde 15 Minuten bei 0 °C und 30 Minuten bei Raumtemperatur gerührt. Anschließend wurde diese Lösung zu einer zweiten Lösung, bestehend aus 196 mg (1,08 mmol) D-Glucamin und 0,137 ml (1,0 mmol) Triethylamin in 10 ml DMF und 3 ml H₂O, getropft und bei Raumtemperatur gerührt. Nach 2 Stunden wurde i. Vak. eingeengt und der Rückstand an Kieselgel gereinigt. Nach einer weiteren Reinigung an einer präparativen HPLC wurde das gewünschte Benzamid als Trifluoressigsäuresalz erhalten. Lösen in 0,1 N HCl und anschließende Gefriertrocknung lieferte das gewünschte Hydrochlorid als farblosen Feststoff.

### Beispiel 15: 3-(6,8-Dichloro-2-methyl-1,2,3,4-tetrahydro-isochinolin-4-yl)-N-(2-hydroxy-1-hydroxymethyl-ethyl)-benzamid-Hydrochlorid

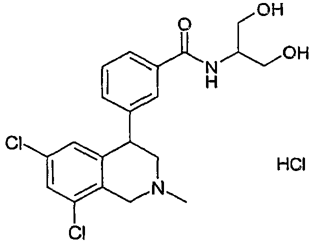

Die Titelverbindung wurde analog zu der in Beispiel 14 beschriebenen Methode ausgehend von 3-(6,8-Dichloro-2-methyl-1,2,3,4-tetrahydro-isochinolin-4-yl)-benzoesäure (Beispiel 14, Zwischenprodukt 5) und 2-Amino-1,3-propandiol hergestellt.

### Beispiel 16: (S)-2-[3-(6,8-Dichloro-2-methyl-1,2,3,4-tetrahydro-isochinolin-4-yl)-benzoylamino]-3-hydroxy-propionsäure-Hydrochlorid

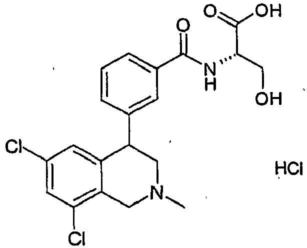

Die Titelverbindung wurde analog zu der in Beispiel 14 beschriebenen Methode ausgehend von 3-(6,8-Dichloro-2-methyl-1,2,3,4-tetrahydro-isochinolin-4-yl)-benzoesäure (Beispiel 14, Zwischenprodukt 5) und L-(+)-Serin hergestellt.

### Beispiel 17: (S)-2-[3-(6,8-Dichloro-2-methyl-1,2,3,4-tetrahydro-isochinolin-4-yl)-benzoylamino]-bernsteinsäure-Hydrochlorid

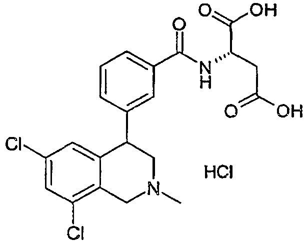

### Zwischenprodukt 1: (S)-2-[3-(6,8-Dichloro-2-methyl-1,2,3,4-tetrahydro-isochinolin-4-yl)-benzoylamino]-bernsteinsäure-di-tert-butylester

300 mg (0,9 mmol) 3-(6,8-Dichloro-2-methyl-1,2,3,4-tetrahydro-isochinolin-4-yl)-benzoesäure (Beispiel 14, Zwischenprodukt 5) wurden analog zu der in Beispiel 14 beschriebenen Methode mit 304 mg (1,08 mmol) Asparaginsäure-di-tert-butylester in einer TOTU-vermittelten Kupplungsreaktion umgesetzt, wobei nach Reinigung an einer präparativen HPLC 170 mg der Titelverbindung als Trifluoressigsäuresalz erhalten wurden.

### 17: 2S-[3-(6,8-Dichloro-2-methyl-1,2,3,4-tetrahydro-isochinolin-4-yl)-benzoylamino]-bernsteinsäure-Hydrochlorid

170 mg 2S-[3-(6,8-Dichloro-2-methyl-1,2,3,4-tetrahydro-isochinolin-4-yl)-benzoylamino]-bernsteinsäure-di-tert-butylester (Zwischenprodukt 1) wurden in 15 ml Dichlormethan gelöst und mit 5 ml Trifluoressigsäure versetzt. Nach einer Stunde bei Raumtemperatur wurde i. Vak. eingeengt, der Rückstand in 30 ml 0,1 N HCl aufgenommen und gefriergetrocknet. Nach erneutem Lösen in H₂O und einer weiteren Gefriertrocknung wurden 130 mg der Titelverbindung erhalten.

### Beispiel 18: (S)-2-[3-(6,8-Dichloro-2-methyl-1,2,3,4-tetrahydro-isochinolin-4-yl)-benzoylamino]-bernsteinsäure-4-amid-Hydrochlorid

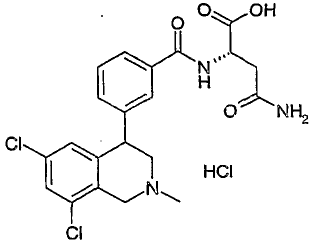

### Zwischenprodukt 1: (S)-2-[3-(6,8-Dichloro-2-methyl-1,2,3,4-tetrahydro-isochinolin-4-yl)-benzoylamino]-bernsteinsäure-4-amid-1-tert-butylester

300 mg (0,9 mmol) 3-(6,8-Dichloro-2-methyl-1,2,3,4-tetrahydro-isochinolin-4-yl)-benzoesäure (Beispiel 14, Zwischenprodukt 5) wurden analog zu der in Beispiel 14 beschriebenen Methode mit 242 mg (1,08 mmol) Asparagin-tert-butylester in einer TOTU-vermittelten Kupplungsreaktion umgesetzt, wobei nach Reinigung an Kieselgel 550 mg der Titelverbindung erhalten wurden.

### 18: (S)-2-[3-(6,8-Dichloro-2-methyl-1,2,3,4-tetrahydro-isochinolin-4-yl)-benzoylamino]-bernsteinsäure-4-amid-Hydrochlorid

550 mg 2S-[3-(6,8-Dichloro-2-methyl-1,2,3,4-tetrahydro-isochinolin-4-yl)-benzoylamino]-bernsteinsäure-4-amid-1-tert-butylester (Zwischenprodukt 1) wurden in 20 ml Dichlormethan gelöst und bei Raumtemperatur mit 5 ml Trifluoressigsäure versetzt. Nach 3 Stunden wurde i. Vak. eingeengt und einmal mit Toluol codestilliert. Der Rückstand wurde in 0,1 N HCl unter Erwärmen gelöst, filtriert und i. Vak. eingeengt. Der Rückstand wurde in H₂O gelöst und gefriergetrocknet, wobei 322 mg der Titelverbindung erhalten wurden.

### Beispiel 19: N-[3-(6,8-Dichloro-2-methyl-1,2,3,4-tetrahydro-isochinolin-4-yl)-phenyl]-N'-[5-guanidino-pentansäure-2S-yl]-harnstoff-Hydrochlorid

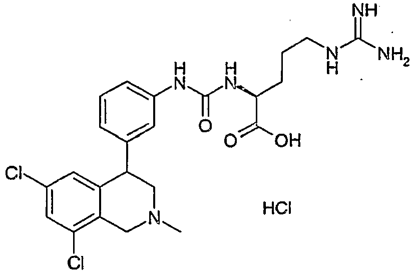

### Zwischenprodukt 1: 1-[3-(6,8-Dichloro-2-methyl-1,2,3,4-tetrahydro-isochinolin-4-yl)-phenyl]-3-[5-N'-(2,2,5,7,8-pentamethyl-chroman-6-sulfonyl)-guanidino-pentansäure-tert-butyl-ester-2S-yl]-harnstoff

373 mg (0,73 mmol) [3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 1, Beispiel 6) wurden in 10 ml abs. DMF gelöst und bei 0 °C mit 400 mg (0,8 mmol) S-2-Amino-5-(N'-(2,2,5,7,8-pentamethyl-chroman-6-sulfonyl)-guanidino-pentansäure-tert-butyl-ester analog zu der in Beispiel 6 beschriebenen Methode umgesetzt, wobei nach Chromatographie an Kieselgel 657 mg der Titelverbindung erhalten würden.

### 19: N-[3-(6,8-Dichloro-2-methyl-1,2,3,4-tetrahydro-isochinolin-4-yl)-phenyl]-N'-[5-guanidino-pentansäure-2S-yl]-harnstoff-Hydrochlorid

590 mg 1-[3-(6,8-Dichloro-2-methyl-1,2,3,4-tetrahydro-isochinolin-4-yl)-phenyl]-3-[5-N'-(2,2,5,7,8-pentamethyl-chroman-6-sulfonyl)-guanidino-pentansäure-tert-butyl-ester-2S-yl]-harnstoff wurden in 25 ml Trifluoressigsäure aufgenommen und eine Stunde bei Raumtemperatur gerührt. Danach wurde i. Vak. eingeengt, der Rückstand mit Ether verrieben und abgesaugt. Das erhaltene Rohprodukt wurde in 1 N HCl aufgenommen, filtriert und gefriergetrocknet. Nach einer Reinigung an einer präparativen HPLC wurden die Produktfraktionen eingeengt, in 0,1 N HCl gelöst und gefriergetrocknet. Danach löst man nochmals in H₂O und schloss eine weitere Gefriertrocknung an, wobei 170 mg der Titelverbindung erhalten wurden.

### Beispiel 20: {N-[3-(6,8-Dichloro-2-methyl-1,2,3,4-tetrahydro-isochinolin-4(R oder S)-yl)-phenyl]-N'-bernsteinsäure-4-amid-2S-yl}-harnstoff-Hydrochlorid

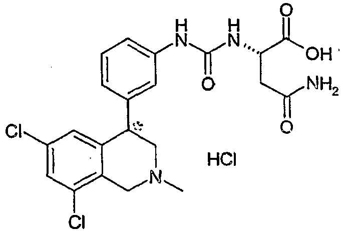

### Zwischenprodukt 1:

### 1a: (R)-3-(6,8-Dichloro-2-methyl-1,2,3,4-tetrahydro-isochinolin-4-yl)-phenylamin und 1b: (S)-3-(6,8-Dichloro-2-methyl-1,2,3,4-tetrahydro-isochinolin-4-yl)-phenylamin

10 g 3-(6,8-Dichloro-2-methyl-1,2,3,4-tetrahydro-isochinolin-4-yl)-phenylamin (Beispiel 2, Zwischenprodukt 1) wurden an einer chiralen Phase in die Enantiomere getrennt.

Präparative Trennbedingungen bei Basislinientrennung:

| | |
|---|---|
| Chirale Säule: | Chiralpak AD10 50 x 10 cm, basisch vorkonditioniert; |
| Solvent: | Acetonitril:Ethanol:Methanol 45:4:1 |
| Flußrate: | 300 ml/min |

Analytische Daten an einer chiralen Phase:

| | |
|---|---|
| Chirale Säule: | Chiralpak ADH/33 250 x 4,6, basisch vorkonditioniert; |
| Solvent: | Acetonitril:Ethanol:Methanol 45:4:1 |
| Flußrate: | 1 ml/min |
| Temperatur: | 30 °C |

Retentionszeit des Enantiomers A: 4,498 Minuten,
Ausbeute des Enantiomers A: 4,0 g;
Retentionszeit des Enantiomers B: 5,480 Minuten,
Ausbeute des Enantiomers B: 4,5 g.

### Zwischenprodukt 2: (R oder S)-[3-(6,8-Dichloro-2-methyl-1,2,3,4-tetrahydro-isochinolin-4-yl)-phenyl]-carbaminsäure-4-nitro-phenylester-Hydrochlorid

1,54 g (5,0 mmol) des Enantiomers B aus Zwischenprodukt 1 wurden mit 1,1 Äquivalenten Chlorameisensäure-4-nitrophenylester analog zu der in Beispiel 6, Zwischenprodukt 1 beschriebenen Methode umgesetzt, wobei 1,87 g der Titelverbindung erhalten wurden.

### {N-[3-(6,8-Dichloro-2-methyl-,1,2,3,4-tetrahydro-isochinolin-4(R oder S)-yl)-phenyl]-N'-bernsteinsäure-4-amid-2S-yl}-harnstoff-Hydrochlorid

Ausgehend von (R oder S)-[3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 2) und (S)-Asparaginsäure-tert-butylester-Hydrochlorid wurde die Titelverbindung analog zu der in Beispiel 13 angegebenen Syntheseroute diastereomerenrein hergestellt.

### Beispiel 21: {N-[3-(6,8-Dichloro-2-methyl-1,2,3,4-tetrahydro-isochinolin-4(R oder S)-yl)-phenyl]-N'-3-hydroxy-propion-säure-2S-yl}-harnstoff-Hydrochlorid

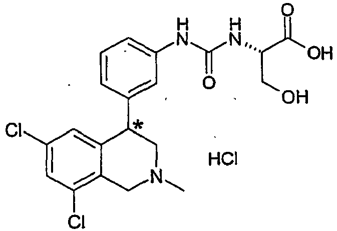

### Zwischenprodukt 1: {N-[3-(6,8-Dichloro-2-methyl-1,2,3,4-tetrahydro-isochinolin-4(R oder S)-yl)-phenyl]-N'-3-tert-butyloxy-propion-säure-tert-butyl-ester-2S-yl}-harnstoff

509 mg (1,0 mmol) (R oder S)-[3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 2, Beispiel 20) wurden mit 280 mg (1,1 mmol) (S)-2-Amino-3-tert-butoxy-propion-säure-tert-butyl-ester-Hydrochlorid analog zu der in Beispiel 12, Zwischenprodukt 1 beschriebenen Weise umgesetzt, wobei 460 mg der Titelverbindung erhalten wurden.

### 21: {N-[3-(6,8-Dichloro-2-methyl-1,2,3,4-tetrahydro-isochinolin-4(R oder S)-yl)-phenyl]-N'-3-hydroxy-propion-säure-2S-yl}-harnstoff-Hydrochlorid

270 mg (0,49 mmol) {N-[3-(6,8-Dichloro-2-methyl-1,2,3,4-tetrahydro-isochinolin-4(R oder S)-yl)-phenyl]-N'-3-tert-butyloxy-propion-säure-tert-butyl-ester-2S-yl}-harnstoff (Zwischenprodukt 1) wurden in 12 ml Trifluoressigsäure eine Stunde bei Raumtemperatur gerührt. Anschließend wurde eingeengt. Der Rückstand wurde mit Ether verrieben und abgesaugt, wobei 247 mg des Trifluoracetats erhalten wurden. Dieses wurde in 30 ml H₂O / 30 ml 1 N HCl gelöst und gefriergetrocknet. Nochmaliges Lösen in H₂O und anschließende Gefriertrocknung lieferte 182 mg der Titelverbindung.

### Beispiel 22: 1-[3-(6,8-Dichloro-2-methyl-1,2,3,4-tetrahydro-isochinolin-4(R oder S)-yl)-phenyl]-3-(2-hydroxy-1,1-bis-hydroxymethyl-ethyl)-harnstoff-Hydrochlorid

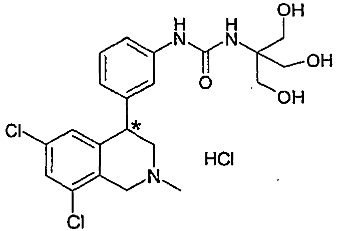

Analoge Vorgehensweise zu der in Beispiel 7/7a beschriebenen Methode lieferte ausgehend von 509 mg (1,0 mmol) (R oder S)-[3-(6,8-Dichloro-2-methyl-1,2,3,4-tetrahydro-isochinolin-4-yl)-phenyl]-carbaminsäüre-4-nitro-phenylester-Hydrochlorid (Zwischenprodukt 2, Beispiel 20) und 2-Amino-2-hydroxymethyl-propan-1,3-diol 101 mg des gewünschten enantiomerenreinen Hydrochlorids.

### Beispiel 23: 1-[3-((R oder S)-6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff-Hydrochlorid

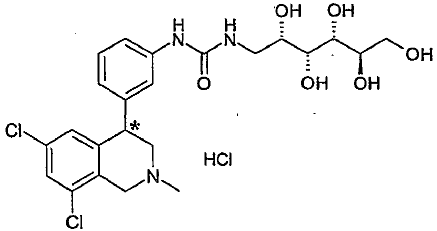

Analoge Vorgehensweise zu der in Beispiel 8/8a beschriebenen Methode lieferte ausgehend von 509 mg (1,0 mmol) (R oder S)-[3-(6,8-Dichloro-2-methyl-1,2,3,4-tetrahydro-isochinolin-4-yl)-phenyl]-carbaminsäure-4-nitro-phenylester-Hydrochlorid (Zwischenprodukt 2, Beispiel 20) und D-Glucamin 169 mg des gewünschten diastereomerenreinen Hydrochlorids.

### Beispiel 24: 5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid-Hydrochlorid

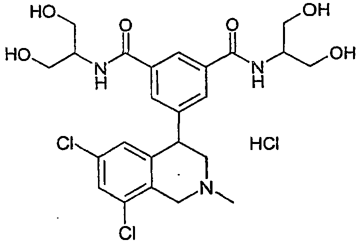

### Zwischenprodukt 1: 1-(3,5-Bis-trifluormethyl-phenyl)-ethanon

5,16 g (20 mmol) 3,5-Bis-trifluormethyl-benzoesäure wurden in 100 ml abs. THF gelöst und bei 0 °C mit 31,25 ml einer 1,6 M Lösung von Methyllithium in Diethylether versetzt. Nachdem 5 Stunden bei Raumtemperatur gerührt wurde, wurde überschüssiges Methyllithium durch Zugabe von H₂O hydrolysiert und das Lösungsmittel i. Vak. entfernt. Der Rückstand wurde in Dichlormethan aufgenommen und mit gesättigter NaHCO₃-Lösung gewaschen. Die organische Phase wurde abgetrennt, mit MgSO₄ getrocknet und eingeengt. Chromatographie an Kieselgel lieferte 3,97 g der Titelverbindung.

### Zwischenprodukt 2: 1-(3,5-Bis-trifluormethyl-phenyl)-2-brom-ethanon

2,97 g (11,6 mmol) 1-(3,5-Bis-trifluormethyl-phenyl)-ethanon (Zwischenprodukt 1) wurden in 15 ml Eisessig gelöst und bei 0 °C mit 1,82 g (11,4 mmol) Br₂ versetzt und 2,5 Stunden bei 50 °C gerührt. Nach Stehen über Nacht wurde das Reaktionsgemisch auf Eis gegeben und zweimal mit Essigester extrahiert. Die vereingten Essigester-Phasen wurden noch zweimal mit H₂O gewaschen, mit Na₂SO₄ getrocknet und eingeengt, wobei 4 g der Titelverbindung erhalten wurden.

### Zwischenprodukt 3: 4-(3,5-Bis-trifluormethyl-phenyl)-6,8-dichlor-2-methyl-1,2,3,4-tetrahydro-isochinolin

Analog zu der unter Beispiel 1 beschriebenen Syntheseroute wurde ausgehend von 1-(3,5-Bis-trifluormethyl-phenyl)-2-brom-ethanon (Zwischenprodukt 2) und 2,4-Dichlorbenzyl-methyl-amin (Beispiel 1, Zwischenprodukt 1) weiterverfahren, wobei nach Alkylierungsreaktion, Reduktion und Ringschlußreaktion das 4-(3,5-Bis-trifluormethyl-phenyl)-6,8-dichlor-2-methyl-1,2,3,4-tetrahydro-isochinolin erhalten wurde.

### Zwischenprodukt 4: 5-(6,8-Dichloro-2-methyl-1,2,3,4-tetrahydro-isochinolin-4-yl)-isophthalsäure

Zu einem Gemisch aus 6 ml Chlorsulfonsäure und 5 ml konz. H₂SO₄ wurden 800 mg (1,9 mmol) 4-(3,5-Bis-trifluormethyl-phenyl)-6,8-dichlor-2-methyl-1,2,3,4-tetrahydro-isochinolin (Zwischenprodukt 3) eingetragen und anschließend 6 Stunden auf 100 °C erhitzt. Zur Aufarbeitung wurde auf Eis gegeben, der Niederschlag abgesaugt und getrocknet, wobei 772 mg der gewünschten Isophthalsäure als Hydrogensulfat erhalten wurden.

### 24: 5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid-Hydrochlorid

95 mg des Zwischenprodukts 4 wurden in 4 ml DMF gelöst und bei 0 °C eine Lösung aus 75,9 mg (0,75 mmol) Triethylamin und 164 mg (0,5 mmol) TOTU in 3 ml DMF zugegeben. Es wurde 30 Minuten bei 0 °C und 30 Minuten bei Raumtemperatur gerührt. Die erhaltene Lösung wurde zu einer Lösung aus 46 mg (0,5 mmol) 2-Amino-1,3-propandiol in 5 ml DMF gegeben. Nach Zugabe von weiteren 50,6 mg (0,5 mmol) Triethylamin wurde bei Raumtemperatur gerührt. Zur Aufarbeitung wurde das Lösungsmittel i. Vak. entfernt und der Rückstand an Kieselgel gereinigt. Nach einer weiteren Reinigung an einer präparativen HPLC wurden die Produktfraktionen eingeengt, in 1 N HCl gelöst und gefriergetrocknet, wobei 48 mg der Titelverbindung isoliert werden konnten.

### Beispiel 25: 5-(6,8-Dichloro-2-methyl-1,2,3,4-tetrahydro-isochinolin-4-yl)-N,N'-bis-(2-hydroxy-1,1-bis-hydroxymethyl-ethyl)-isophthalsäure-amid-Hydrochlorid und Beispiel 26: 5-(6,8-Dichloro-2-methyl-1,2,3,4-tetrahydro-isochinolin-4-yl)-N-(2-hydroxy-1,1-bis-hydroxymethyl-ethyl)-isophthalsäure-amid-Hydrochlorid

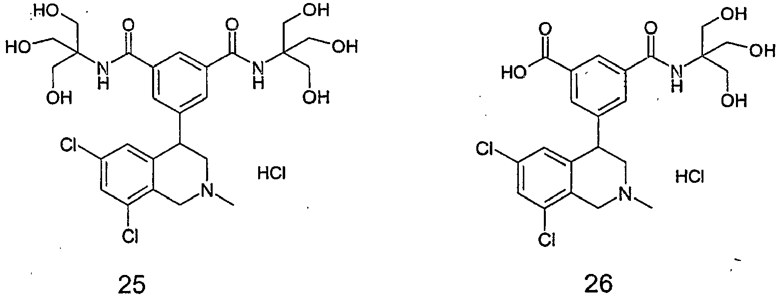

95 mg 5-(6,8-Dichloro-2-methyl-1,2,3,4-tetrahydro-isochinolin-4-yl)-isophthalsäure (Zwischenprodukt 4, Beispiel 24) wurden analog zu der in Beispiel 24 beschriebenen Methode mit 2-Amino-2-hydroxymethyl-propan-1,3-diol in einer TOTU-vermittelten Reaktion umgesetzt. Bei der abschließenden Reinigung an Kieselgel konnten 2 Fraktionen isoliert werden, die beide einer weiteren Reinigung an einer präparativen HPLC unterworfen wurden. Einengen der Produktfraktionen, Lösen in verdünnter HCl und anschließende Gefriertrocknung lieferte 17 mg der Titelverbindung zu Beispiel 25, sowie 33 mg der Titelverbindung zu Beispiel 26.

### Beispiel 27: 5-(6,8-Dichlor-2-methyl-1,2,3,4-tetrahydro-isochinolin-4-yl)-N,N'-bis-((2S,3R,4R,5R)-2,3,4,5,6-pentahydroxy-hexyl)-isophthalsäure-amid-Hydrochlorid und Beispiel 28: 5-(6,8-Dichlor-2-methyl-1,2,3,4-tetrahydro-isochinolin-4-yl)-N-((2S,3R,4R,5R)-2,3,4,5,6-pentahydroxy-hexyl)-isophthalsäure-amid-Hydrochlorid

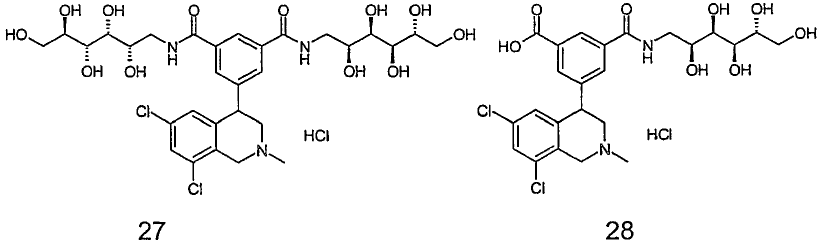

Analoges Vorgehen zu der in den Beispielen 25/26 beschrieben Methode lieferte ausgehend von 95 mg 5-(6,8-Dichloro-2-methyl-1,2,3,4-tetrahydro-isochinolin-4-yl)-isophthalsäure (Zwischenprodukt 4, Beispiel 24) 11 mg der Beispielverbindung 27, sowie 29 mg der Beispielverbindung 28.

### Beispiel 29: (S)-2-[3-((S)-1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure-Hydrochlorid

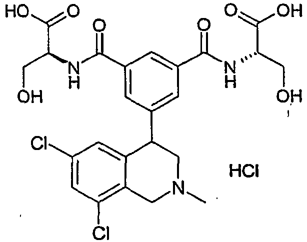

### Zwischenprodukt 1: (S)-3-tert-Butoxy-2-[3-((S)-2-tert-butoxy-1-tert-butoxycarbonyl-ethylcarbamoyl)-5-(6,8-dichloro-2-methyl-1,2,3,4-tetrahydro-isochinolin-4-yl)-benzoylamino]-propionsäüre-tert-butyl-ester

95 mg 5-(6,8-Dichloro-2-methyl-1,2,3,4-tetrahydro-isochinolin-4-yl)-isophthalsäure (Zwischenprodukt 4, Beispiel 24) wurden analog zu der in Beispiel 24 beschriebenen TOTU-Kupplung mit 127 mg (S)-2-Amino-3-tert-butoxy-propionsäure-tert-butylester-Hydrochlorid umgesetzt, wobei nach Chromatographie an Kieselgel 200 mg der Titelverbindung erhalten wurden.

### 29: (S)-2-[3-((S)-1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure-Hydrochlorid

200 mg des Zwischenprodukts 1 wurden in 5 ml Trifluoressigsäure eine Stunde bei Raumtemperatur gerührt, Anschließend wurde das Lösungsmittel i. Vak. entfernt, der Rückstand mit Ether verrieben und abgesaugt. Das so erhaltene Trifluoressigsäuresalz wurde in verdünnter HCl gelöst und gefriergetrocknet, wobei 109 mg der Titelverbindung isoliert werden konnten.

### Beispiel 30: (S)-2-Amino-5-guanidino-pentansäure-[3-(6,8-dichlor-2-methyl-1,2,3,4-tetrahydro-isochinolin-4-yl)-phenyl]-amid-Hydrochlorid

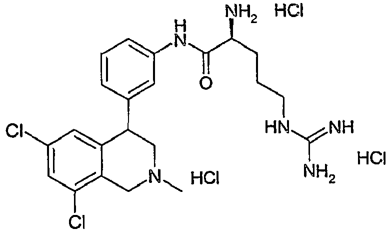

### Zwischenprodukt 1: 2S-tert-Butyl-oxycarbonyl-amino-5-(N',N"-di-tert-butyl-oxycarbonyl)-guanidino-pentan-säure-[3-(6,8-dichloro-2-methyl-1,2,3,4-tetrahydro-isochinolin-4-yl)-phenyl]-amid

570 mg (1,2 mmol) (S)-5-(N',N"-Di-tert-butyl-oxycarbonyl-guanidino)-2-tert-butyl-oxycarbonyl-amino-pentansäure wurden in 10 ml DMF vorgelegt und 405 mg (4,0 mmol) Triethylamin zugegeben. Nach Abkühlen auf 0 °C wurden 406 mg (3,0 mmol) HOBt, 379 mg (3,0 mmol) DIC, sowie 61 mg (0,5 mmol) DMAP zugegeben. Danach wurde bei 0 °C mit einer Lösung von 307 mg (1,0 mmol) 3-(6,8-Dichloro-2-methyl-1,2,3,4-tetrahydro-isochinolin-4-yl)-phenylamin (Beispiel 2, Zwischenprodukt 1) in 6 ml DMF versetzt und bei Raumtemperatur gerührt. Zur Aufarbeitung wurde das Lösungsmittel i. Vak. entfernt, der Rückstand in Essigester aufgenommen und mit gesättigter NaHCO₃-Lösung gewaschen. Die organische Phase wurde abgetrennt und die wässrige noch einmal mit Essigester extrahiert. Die vereinigten organischen Phasen wurden einmal mit 2 N HCl und einmal mit H₂O gewaschen, mit MgSO₄ getrocknet und einrotiert. Nach anschließender Chromatographie an Kieselgel wurden 237 mg der Titelverbindung erhalten.

### 30: (S)-2-Amino-5-guanidino-pentansäure-[3-(6,8-dichlor-2-methyl-1,2,3,4-tetrahydro-isochinolin-4-yl)-phenyl]-amid-Hydrochlorid

237 mg des Zwischenprodukts 1 wurden in 5 ml Dichlormethan gelöst und bei 0 °C mit 1 ml Trifluoressigsäure versetzt. Man ließ auf Raumtemperatur erwärmen und rührte für 24 Stunden. Danach wurde vom Lösungsmittel befreit und einmal mit Toluol codestilliert. Das so erhaltene Produkt wurde in 25 ml 0,1 N HCl gelöst, filtriert und gefriergetrocknet. Nochmaliges Lösen in H₂O und anschließende Gefriertrocknung lieferte 162 mg der Titelverbindung.

### Beispiel 31: (S)-2-Amino-5-guanidino-pentansäure-[4-(6,8-dichlor-2-methyl-1,2,3,4-tetrahydro-isochinolin-4-yl)-phenyl]-amid-Trifluoressigsäuresalz

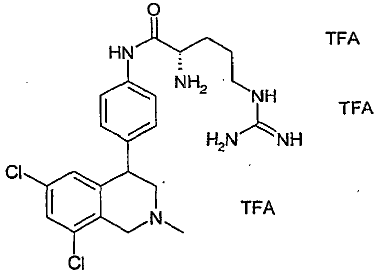

Analoge Vorgehensweise zu der in Beispiel 30 beschriebenen Methode lieferte ausgehend von 4-(6,8-Dichloro-2-methyl-1,2,3,4-tetrahydro-isochinolin-4-yl)-phenylamin (Beispiel 1, Zwischenprodukt 6) und (S)-5-(N',N"-Di-tert-butyl-oxycarbonyl-guanidino)-2-tert-butyl-oxycarbonyl-amino-pentansäure und anschließender Behandlung mit Trifluoressigsäure die Titelverbindung als Trifluoressigsäuresalz.

### Beispiel 32: (S)-2-Amino-N-[3-(6,8-dichloro-2-methyl-1,2,3,4-tetrahydro-isochinolin-4-yl)-phenyl]-3-(1H-imidazol-4-yl)-propionsäure-amid-Trifluoressigsäuresalz

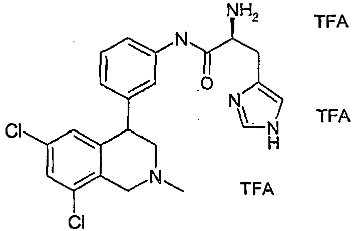

Die Titelverbindung wurde analog zu der in Beispiel 30 beschriebenen Vorgehensweise ausgehend von 3-(6,8-Dichloro-2-methyl-1,2,3,4-tetrahydro-isochinolin-4-yl)-phenylamin (Beispiel 2, Zwischenprodukt 1) und 4-((S)-2-tert-Butoxycarbonylamino-2-carboxy-ethyl)-imidazol-1-carbonsäure-tert-butyl-ester und anschließender Behandlung mit Trifluoressigsäure hergestellt.

### Beispiel 33: (S)-2-Amino-N-[4-(6,8-dichloro-2-methyl-1,2,3,4-tetrahydro-isochinolin-4-yl)-phenyl]-3-(1H-imidazol-4-yl)-propionsäure-amid-Trifluoressigsäuresalz

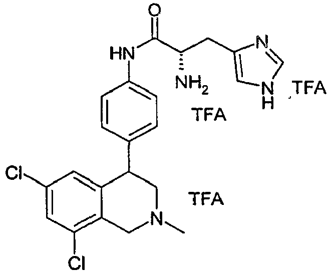

Die Titelverbindung wurde analog zu der in Beispiel 30 beschriebenen Vorgehensweise ausgehend von 4-(6,8-Dichloro-2-methyl-1,2,3,4-tetrahydro-isochinolin-4-yl)-phenylamin (Beispiel 1, Zwischenprodukt 6) und 4-((S)-2-tert-Butoxycarbonylamino-2-carboxy-ethyl)-imidazol-1-carbonsäure-tert-butyl-ester und anschließender Behandlung mit Trifluoressigsäure hergestellt.

### Beispiel 34: {3-[4-(6,8-Dichlor-2-methyl-1,2,3,4-tetrahydro-isochinolin-4-yl)-phenyl]-ureido}-essigsäureethylester-Hydrochlorid

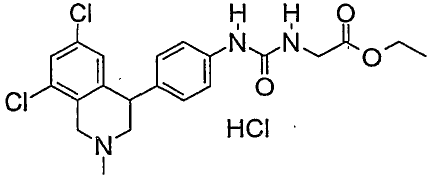

4-(6,8-Dichlor-2-methyl-1,2,3,4-tetrahydro-isochinolin-4-yl)-phenylamin (95 mg, Beispiel 1, Zwischenprodukt 6) wurde in Acetonitril (2 ml) gelöst und unter Rühren Ethylisocyanatoacetat (30 mg) zugetropft. Nach vier Stunden wurde die Lösung eingeengt und der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Essigester extrahiert. Nach Trocknen über Magnesiumsulfat wurde zur Trockne gebracht. Der Rückstand wurde mit wässriger Salzsäure aufgenommen und gefriergetrocknet. Es wurden 107 mg der gewünschten Verbindung erhalten.

### Beispiel 35: {3-[3-(6,8-Dichlor-2-methyl-1,2,3,4-tetrahydro-isochinolin-4-yl)-phenyl]-ureido}-essigsäureethylester-Hydrochlorid

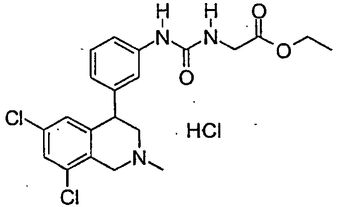

Analog zu Beispiel 34 wurden 3-(6,8-Dichlor-2-methyl-1,2,3,4-tetrahydro-isochinolin-4-yl)-phenylamin (Beispiel 2, Zwischenprodukt 1) und Ethylisocyanatoacetat umgesetzt.

### Beispiel 36: {3-[2-(6,8-Dichlor-2-methyl-1,2,3,4-tetrahydro-isochinolin-4-yl)-phenyl]-ureido}-essigsäureethylester-Hydrochlorid

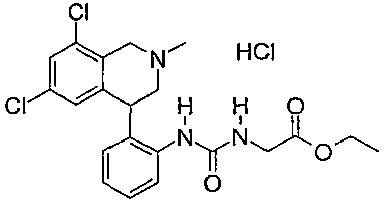

Analog zu Beispiel 34 wurden 2-(6,8-Dichlor-2-methyl-1,2,3,4-tetrahydro-isochinolin-4-yl)-phenylamin (Beispiel 3, Zwischenprodukt 5) und Ethylisocyanatoacetat umgesetzt.

### Beispiel 37: {3-[3-(6,8-Dichlor-2-methyl-1,2,3,4-tetrahydro-isochinolin-4-yl)-phenyl]-ureido}-essigsäure-Trifluoressigsäuresalz

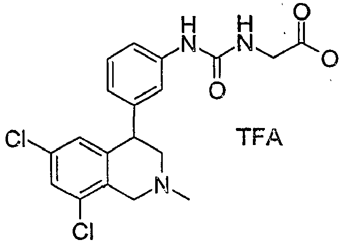

3-[3-(6,8-Dichlor-2-methyl-1,2,3,4-tetrahydro-isochinolin-4-yl)-phenyl]-ureido}-essigsäureethylester-Hydrochlorid (9 mg, Beispiel 35) wurde mit Wasser (2 ml) und gesättigter Kaliumcarbonat-Lösung (0,25 ml) versetzt und 48 h gerührt. Mit wässriger 2 N Salzsäure wurde auf pH 2 gestellt, das Lösungsmittel abgezogen und der Rückstand mittels präparativer HPLC gereinigt. Die Produkt enthaltenden Fraktionen wurden vereinigt, das Acetonitril am Rotationsverdampfer abgezogen und der Rückstand gefriergetrocknet. Es wurden 6 mg der gewünschten Verbindung erhalten.

### Beispiel 38: {3-[4-(6,8-Dichlor-2-methyl-1,2,3,4-tetrahydro-isochinolin-4-yl)-phenyl]-ureido}-essigsäure-Trifluoressigsäuresalz

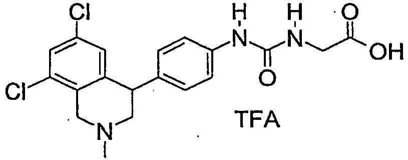

Analog zu Beispiel 4 wurde 3-[4-(6,8-Dichlor-2-methyl-1,2,3,4-tetrahydro-isochinolin-4-yl)-phenyl]-ureido}-essigsäureethylester-Hydrochlorid verseift.

### Beispiel 39: {3-[2-(6,8-Dichlor-2-methyl-1,2,3,4-tetrahydro-isochinolin-4-yl)-phenyl]-ureido}-essigsäure-Trifluoressigsäuresalz

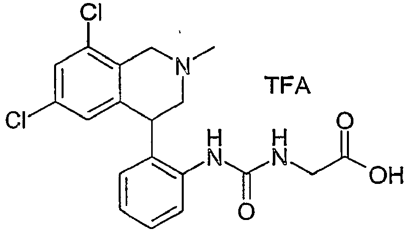

Analog zu Beispiel 4 wurde 3-[2-(6,8-Dichlor-2-methyl-1,2,3,4-tetrahydro-isochinolin-4-yl)-phenyl]-ureido}-essigsäureethylester-Hydrochlorid (Beispiel 36) verseift.

### Beispiel 40: {3-[3-(6,8-Dichlor-2-methyl-1,2,3,4-tetrahydro-isochinolin-4(R oderS)-yl)-phenyl]-ureido}-essigsäureethylester

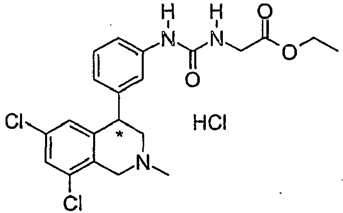

Analog zu Beispiel 34 wurden 3-(6,8-Dichlor-2-methyl-1,2,3,4-tetrahydro-isochinolin-4(R oder S)-yl)-phenylamin (Beispiel 20, Zwischenprodukt 1, Enantiomer B) und Ethylisocyanatoacetat umgesetzt, wobei statt Acetonitril Dichlormethan als Lösungsmittel verwendet wurde.

### Beispiel 41: {3-[2-(6,8-Dichlor-2-methyl-1,2,3,4-tetrahydro-isochinolin-4(R oderS)-yl)-phenyl]-ureido}-essigsäureethylester

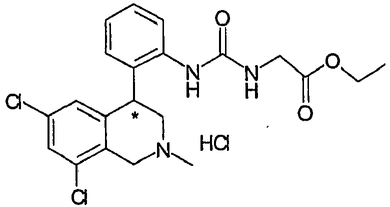

### Zwischenprodukt 1:

(R)-2-(6,8-Dichloro-2-methyl-1,2,3,4-tetrahydro-isochinolin-4-yl)-phenylamin und (S)-2-(6,8-Dichloro-2-methyl-1,2,3,4-tetrahydro-isochinolin-4-yl)-phenylamin 2-(6,8-Dichloro-2-methyl-1,2,3,4-tetrahydro-isochinolin-4-yl)-phenylamin (Beispiel 3, Zwischenprodukt 5) wurde an einer chiralen Phase in die Enantiomere getrennt.

Präparative Trennbedingungen:

| | |
|---|---|
| Chirale Säule: | Chiralpak AD10 35 x 10 cm |
| Solvent: | Acetonitril |
| Flußrate: | 300 ml/min |

Analytische Daten an einer chiralen Phase:

| | |
|---|---|
| Chirale Säule: | Chiralpak ADH 250 x 4,6 mm , |
| Solvent: | Acetonitril |
| Flußrate: | 1 ml/min |
| Temperatur: | 30 °C |

Retentionszeit des Enantiomers A: 5,1 min
Retentionszeit des Enantiomers B: 7,3 min

Analog zu Beispiel 40 wurden 2-(6,8-Dichlor-2-methyl-1,2,3,4-tetrahydro-isochinolin-4(R oder S)-yl)-phenylamin (Enantiomer B) und Ethylisocyanatoacetat umgesetzt.

### Beispiel 42: {3-[4-(6,8-Dichlor-2-methyl-1,2,3,4-tetrahydro-isochinolin-4(R oderS)-yl)-phenyl]-ureido}-essigsäureethylester

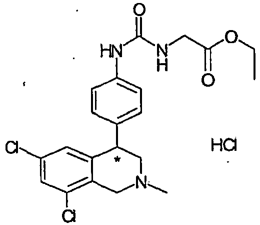

### Zwischenprodukt 1:

(R)-4-(6,8-Dichloro-2-methyl-1,2,3,4-tetrahydro-isochinolin-4-yl)-phenylamin und (S)-4-(6,8-Dichloro-2-methyl-1,2,3,4-tetrahydro-isochinolin-4-yl)-phenylamin 4-(6,8-Dichloro-2-methyl-1,2,3,4-tetrahydro-isochinolin-4-yl)-phenylamin (Beispiel 1, Zwischenprodukt 6) wurde an einer chiralen Phase in die Enantiomere getrennt.

Präparative Trennbedingungen:

| | |
|---|---|
| Chirale Säule: | Chiralpak AD10 35 x 10 cm |
| Solvent: | MeOH/0,1% DEA |
| Flußrate: | 300 ml/min |

Analytische Daten an einer chiralen Phase:

| | |
|---|---|
| Chirale Säule: | Chiralpak ADH 250 x 4,6 mm , |
| Solvent: | MeOH/0,1% DEA |
| Flußrate: | 1 ml/min |
| Temperatur: | 30 °C |

Retentionszeit des Enantiomers A: 4,8 min
Retentionszeit des Enantiomers B: 7,6 min

Analog zu Beispiel 40 wurden 4-(6,8-Dichlor-2-methyl-1,2,3,4-tetrahydro-isochinolin-4(R oder S)-yl)-phenylamin (Enantiomer B) und Ethylisocyanatoacetat umgesetzt.

### Beispiel 43: {3-[4-(6,8-Dichlor-2-methyl-1,2,3,4-tetrahydro-isochinolin-4(R oder S)-yl)-phenyl]-ureido}-essigsäure-Trifluoressigsäuresalz

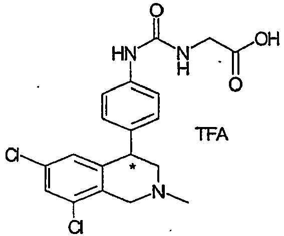

Analog zu Beispiel 4 wurde 3-[4-(6,8-Dichlor-2-methyl-1,2,3,4-tetrahydro-isochinolin-4(R oder S)-yl)-phenyl]-ureido}-essigsäureethylester-Hydrochlorid (Beispiel 42) verseift, allerdings diente Ethanol als Lösungsmittel und wässrige NatriumhydroxidLösung als Base.

### Beispiel 44: {3-[2-(6,8-Dichlor-2-methyl-1,2,3,4-tetrahydro-isochinolin-4(R oder S)-yl)-phenyl]-ureido}-essigsäure-Trifluoressigsäuresalz

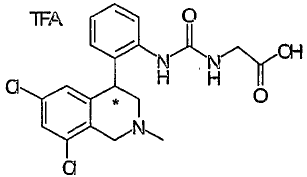

3-[2-(6,8-Dichlor-2-methyl-1,2,3,4-tetrahydro-isochinolin-4(R oder S)-yl)-phenyl]-ureido}-essigsäureethylester-Hydrochlorid (430 mg, Beispiel 41) wurde in Wasser vorgelegt (35 ml) und unter Rühren mit 10%iger Salzsäure versetzt. Nach 3 Stunden unter Rückfluss wurde verseift. Das Lösungsmittel wurde abgezogen und der Rückstand mittels präparativer HPLC gereinigt. Die Produkt enthaltenden Fraktionen wurden vereinigt, und das Lösungsmittel am Rotationsverdampfer abgezogen. Der Rückstand wurde über Kieselgel weiter aufgereinigt (Ethylacetat/Methanol 1:1), die Produkt enthaltenden Fraktionen wurden vereinigt, das Lösungsmittel am Rotationsverdampfer abgezogen und der Rückstand gefriergetrocknet. Es wurden 45 mg der gewünschten Verbindung erhalten.

### Beispiel 45: {3-[3-(6,8-Dichlor-2-methyl-1,2,3,4-tetrahydro-isochinolin-4(R oder S)-yl)-phenyl]-ureido}-essigsäure-Trifluoressigsäuresalz

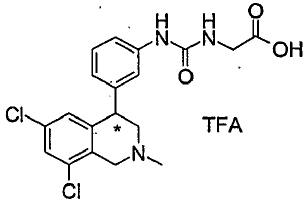

Analog zu Beispiel 4 wurde 3-[3-(6,8-Dichlor-2-methyl-1,2,3,4-tetrahydro-isochinolin-4(R oder S)-yl)-phenyl]-ureido}-essigsäureethylester-Hydrochlorid (Beispiel 40) verseift, wobei allerdings auf Acetonitril als Lösungsmittel verzichtet wurde.

### Beispiel 46: [4-(6,8-Dichlor-2-methyl-1,2,3,4-tetrahydro-isochinolin-4(R oder S)-yl)-phenyl]-carbaminsäure-2-methoxy-ethylester

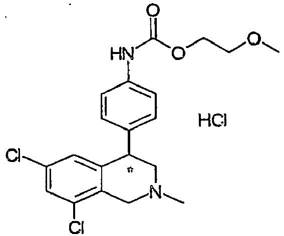

4-(5,8-Dichlor-2-methyl-1,2,3,4-tetrahydro-isochinolin-4(R oder S)-yl)-phenylamin (70 mg, Beispiel 20, Zwischenprodukt 1, Enantiomer B) wurde in Methylenchlorid (4,5 ml) gelöst und unter Rühren langsam eine Lösung aus Chlorameisensäure-2-methoxyethylester (39 mg) in Methylenchlorid (0,5 ml) zugetropft. Nach Stehenlassen über Nacht wurde das Lösungsmittel abgezogen und der Rückstand mittels präparativer HPLC gereinigt. Die Produkt enthaltenden Fraktionen wurden vereinigt, das Acetonitril am Rotationsverdampfer abgezogen, und der Rückstand mit Salzsäure versetzt und gefriergetrocknet. Es wurden 80 mg der gewünschten Verbindung erhalten.

### Beispiel 47: [3-(6,8-Dichlor-2-methyl-1,2,3,4-tetrahydro-isochinolin-4(R oder S)-yl)-phenyl]-carbaminsäure-2-methoxy-ethylester

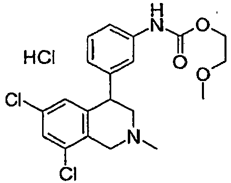

Analog zu Beispiel 13 wurde 3-(6,8-Dichlor-2-methyl-1,2,3,4-tetrahydro-isochinolin-4-yl)-phenylamin (Beispiel 2, Zwischenprodukt 1) mit Chlorameisensäure-2-methoxyethylester umgesetzt. Auf die Chromatographie konnte allerdings verzichtet wurden.

### Bedingungen

### Präp. HPLC:

Die präparative HPLC wurde sowiet nicht anders angegeben unter folgenden Bedingungen durchgeführt:

| | |
|---|---|
| stationäre Phase: | Merck Purospher RP18 (10µM) 250 x 25 mm |
| mobile Phase: | 90% H₂O (0,05% TFA)→ 90% Acetonitril; 40 min; 25 ml/min |

### LCMS-Methoden:

### Methode A:

| | |
|---|---|
| Stationäre Phase: | YMC, J'sphere ODS, H80 20x2 4p; |
| Mobile Phase: | 0 min, 90% H₂O (0,05% TFA); 2,5 min, 95% ACN; 3,3 min, 95% ACN; 3,35 min, 90% H₂O; 1 ml/min; 30 °C. |

### Methode B:

| | |
|---|---|
| Stationäre Phase: | MerckPurosphere 3µ2x55mm; |
| Mobile Phase: | 0 min, 95% H₂O (0,05% TFA); 4 min, 95% ACN; 5,5 min, 95% ACN; 6,5 min, 95% H₂O; 0,5 ml/min; 30 °C. |

### Methode C:

| | |
|---|---|
| Stationäre Phase: | MerckPurosphere 5µ2x55mm; |
| Mobile Phase: | 0 min, 95% H₂O (0,05% TFA); 3 min, 95% ACN; 4,5 min, 95% ACN; 5,5 min, 95% H₂O; 0,5 ml/min; 30 °C. |

### Methode D:

| | |
|---|---|
| Stationäre Phase: | YMC, J'sphere ODS, H80 20x2 4µ; |
| Mobile Phase: | 0 min, 90% H₂O (0,05% TFA); 1,9 min, 95% ACN; 2,4 min, 95% ACN; 2,45 min, 90% H₂O; 1 ml/min; 30 °C. |

### Methode E:

| | |
|---|---|
| Stationäre Phase: | MerckPurosphere 5µ2x55mm; |
| Mobile Phase: | 0 min, 95% H₂O (0,05% TFA); 3,5 min, 95% ACN; 4,5 min, 95% ACN; 5,5 min, 95% H₂O; 0,5 ml/min; 30 °C. |

### Methode F:

| | |
|---|---|
| stationäre Phase: | YMC J'sphere ODS H80 20 x 2,1 mm |
| mobile Phase: | 90% H₂O (0,05% TFA)→ 95% Acetonitril; 1,9 min; 95% Acetonitril; 0,5 min → 10% Acetonitril; 0,05 min; 1 ml/min. |

### Methode G:

| | |
|---|---|
| stationäre Phase: | YMC J'sphere ODS H80 20 x 2,1 mm |
| mobile Phase: | 96% H₂O (0,05% TFA)→ 95% Acetonitril; 2,0 min; 95% Acetonitril; 0,4 min → 4% Acetonitril; 0,05 min; 1 ml/min. |

**Tabelle 1: Analytische Daten der Beispielverbindungen**

| Beispiel-Nr. | Retentionszeit*) | Methode | M+H⁺ | Methode |
|---|---|---|---|---|
| 1 | 1,402 | A | 485,1 | ESI |
| 1a | 1,774 | C | 485,3 | ESI |
| 2 | 1,469 | A | 485,1 | ESI |
| 2a | 3,420 | B | 485.1 | ESI |
| 3 | 1,512 | A | 485,1 | ESI |
| 3a | 1,519 | A | 485,1 | ESI |
| 4a | 1,828 | C | 485,2 | ESI |
| 4b | 1,806 | C | 485,3 | ESI |
| 5 | 0,879 | D | 485,1 | ESI |
| 6 | 0,933 | D | 424,2 | ESI |
| 6a | 0,937 | D | 424,2 | ESI |
| 7 | 0,951 | D | 454,2 | ESI |
| 7a | 0,958 | D | 454,1 | ESI |
| 8 | 0,902 | D | 514,2 | ESI |
| 8a | 0,9121 | D | 514,2 | ESI |
| 9 | 0,891 | D | 512,1 | ESI |
| 10 | 0,979 | D | 458,1 | ESI |
| 11 | 0,883 | D | 435,2 | ESI |
| 12 | 0,973 | D | 438,1 | ESI |
| 12a | 0,970 | D | 438,2 | ESI |
| 13 | 0,945 | D | 465,2 | ESI |
| 14 | 0,834 | D | 499,1 | ESI |
| 15 | 0,887 | D | 409,1 | ESI |
| 16 | 0,959 | D | 423,1 | ESI |
| 17 | 2.081 | E | 451,1 | ESI |
| 18 | 2,027 | E | 450,1 | ESI |
| 19 | 0,925 | D | 507,2 | ESI |
| 20 | 0,959 | D | 465,1 | ESI |
| 21 | 0,982 | D | 438,1 | ESI |
| 22 | 0,971 | D | 454,2 | ESI |
| 23 | 0,925 | D | 514,2 | ESI |
| 24 | 0,633 | D | 526,1 | ESI |
| 25 | 0,711 | D | 586,2 | ESI |
| 26 | 0,853 | D | 483,1 | ESI |
| 27 | 0,402 | D | 706,1 | ESI |
| 28 | 0,803 | D | 543,1 | ESI |
| 29 | 0,820 | D | 554,1 | ESI |
| 30 | 0,772 | D | 463,2 | ESI |
| 31 | 1,805 | E | 463,2 | ESI |
| 32 | 1,917 | E | 444,1 | ESI |
| 33 | 1,817 | E | 444,1 | ESI |
| 34 | 1,14 | F | 436,5 | ESI |
| 35 | 1,15 | F | 436,5 | ESI |
| 36 | 1,13 | F | 436,5 | ESI |
| 37 | 1,08 | F | 408,4 | ESI |
| 38 | 1,00 | F | 408,4 | ESI |
| 39 | 1,04 | F | 408,4 | ESI |
| 40 | 1,15 | F | 436,5 | ESI |
| 41 | 1,13 | F | 436,5 | ESI |
| 42 | 1,05 | G | 436,5 | ESI |
| 43 | 0,94 | G | 408,1 | ESI |
| 44 | 0,95 | G | 408,1 | ESI |
| 45 | 0,96 | G | 408,1 | ESI |
| 46 | 1,10 | G | 409,1 | ESI |
| 47 | 1,13 | F | 409,1 | ESI |

| | | | | |
|---|---|---|---|---|
| *) die Retentionszeiten beziehen sich auf die Massenspektren. | | | | |

### Pharmakologische Daten:

### Testbeschreibung:

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wurde die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wurde ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCl, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wurde ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wurde ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

Beispielhaft sind in Tabelle 2 die inhibitorischen Daten einiger Beispielverbindungen dargestellt.

**Tabelle 2: Inhibitorische Daten einiger Beispielverbindungen am NHE3**

| Beispielverbindung | IC₅₀-Wert (µM) |
|---|---|
| 2 | 0,0036 |
| 13 | 0,0176 |
| 16 | 0,1594 |
| 31 | 0,0291 |

## Patentansprüche

1. Verbindungen der Formel I 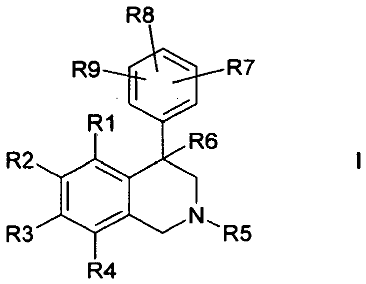 worin bedeuten
R1 und R3 gleich H und R2 und R4 gleich Cl;
R5 H, CH₃, CH₂CH₃, CF₃ oder CH₂CF₃;
R6 H, OH, CH₃, CF₃, OCH₃ oder OCOCH₃;
R7, R8 und R9 unabhängig voneinander H, F, Cl, Br, I, OH, CH₃, CH₂CH₃, CF₃, CH₂CF₃, OCH₃, OCH₂CH₃, OCF₃, OCH₂CF₃, SO₂R47, SO₃R60, COR47, COOR60, NR51 R52 oder eine Gruppe -L-G;
R47 H, CH₃, CH₂CH₃, CF₃, CH₂CF₃ oder NR48R49;
R48 und R49 unabhängig voneinander H, CH₃, CH₂CH₃, CF₃, CH₂CF₃, COCH₃, COCH₂CH₃, COCF₃ oder COCH₂CF₃;
R60 H, CH₃, CH₂CH₃, CF₃, CH₂CF₃;
R51 und R52 unabhängig voneinander H, CH₃, CH₂CH₃, CF₃, CH₂CF₃, COCH₃, COCH₂CH₃, COCF₃ oder COCH₂CF₃;
L -NR30-, -NR30CO-, -CONR30-, -NR30CONR31-, -NR30COO-; R30 und R31 unabhängig voneinander H, CH₃, CH₂CH₃, CF₃ oder CH₂CF3;
G eine Gruppe Cₐ(OR32)ₓH₂ₐ₊₁₋ₓ, wobei eine oder mehrere CH₂-Gruppen durch O oder NR33 ersetzt sein können, C_{b}(OR32)_{y}H_{2b-1-y}, wobei eine oder mehrere CH₂-Gruppen durch O oder NR33 ersetzt sein können, -(CH₂)_{z}-COOR34, -(CH₂)_{z}-SO₃R34, -(CH₂)_{z}-N⁺R35R36R37, wobei 1 oder 2 H-Atome der -(CH₂)_{z}- Einheiten durch OR32-Gruppen ersetzt sein können, -CR38R39-COOR40 oder -CR38R39NR41 R42;
a 2, 3, 4, 5, 6, 7 oder 8;
x 2, 3, 4, 5, 6, 7 oder 8;
R32 H, CH₃, CH₂CH₃, CF₃, CH₂CF₃, COCH₃, COCH₂CH₃, COCF₃ oder COCH₂CF₃;
R33 H, CH₃, CH₂CH₃, CF₃ oder CH₂CF₃;
b 3, 4, 5, 6 oder 7;
y 2, 3, 4, 5, 6 oder 7;
z 1 oder 2;
R34, R35, R36 und R37 unabhängig voneinander H, CH₃, CH₂CH₃, CF₃ oder CH₂CF₃;
R38 -(CH₂)ₙ-Y;
n 0, 1, 2, 3 oder 4;
Y Alkyl mit 1, 2, 3 oder 4 C-Atomen, die teilweise oder vollständig fluoriert sein können und in dem eine oder mehrere CH₂-Gruppen durch O, S oder NR43 ersetzt sind, COOR44, CONR45R46, NHC(NH)NH₂, Phenyl oder Heteroaryl ausgewählt aus der Gruppe Imidazolyl, Pyrazolyl, Pyrrolyl, Triazolyl, Tetrazolyl, Thiazolyl, Oxazolyl und Pyridyl, wobei die Phenyl- und Heteroarylreste substituiert sein können mit bis zu 3 Substituenten ausgewählt aus der Gruppe CH₃, CF₃, OH, OCH₃ oder NH₂;
R43, R44, R45 und R46 unabhängig voneinander H, CH₃, CH₂CH₃, CF₃ oder CH₂CF₃;
R39 H, CH₃, CH₂CH₃, CF₃ oder CH₂CF₃;
R40 H, CH₃, CH₂CH₃, CF₃ oder CH₂CF₃;
R41 und R42 unabhängig voneinander H, CH₃, CH₂CH₃, CF₃, CH₂CF₃, COCH₃, COCH₂CH₃, COCF₃ oder COCH₂CF₃;
wobei mindestens einer der Reste R7, R8 oder R9 durch die Gruppe -L-G definiert sein muss,
sowie deren pharmazeutisch verträgliche Salze und Trifluoressigsäuresalze.

2. Verbindungen der Formel I nach Anspruch 1, worin bedeuten
R1 und R3 gleich H und R2 und R4 gleich Cl;
R5 CH₃;
R6 H;
R7, R8 und R9 unabhängig voneinander H, OH, CH₃, CF₃, OCH₃, SO₂R47, SO₃R60, COR47, COOR60, NR51 R52 oder eine Gruppe -L-G;
R47 H, CH₃ oder NR48R49;
R48 und R49 unabhängig voneinander H, CH₃ oder COCH₃,
R60 H, CH₃;
R51 und R52 unabhängig voneinander H, CH₃, CH₂CH₃ oder COCH₃
L -NR30CO-, -CONR30-, -NR30CONR31-;
R30 und R31 gleich H;
G eine Gruppe der Form Cₐ(OR32)ₓH₂ₐ₊₁₋ₓ, wobei eine oder mehrere CH₂-Gruppen durch O oder NR33 ersetzt sein können, C_{b}(OR32)_{y}H_{2b-1-y}, wobei eine oder mehrere CH₂-Gruppen durch O oder NR33 ersetzt sein können, -(CH₂)₂-COOH, -(CH₂)₂-SO₃H, -(CH₂)₂-N(CH₃)₃⁺, wobei 1 oder 2 H-Atome der -(CH₂)₂- Einheiten durch OH-Gruppen ersetzt sein können, -CR38R39-COOR40 oder -CR38R39NR41 R42;
a 3, 4, 5 oder 6;
x 2, 3, 4 oder 5;
R32 H;
R33 H oder CH₃;
b 5 oder 6;
y 2, 3, 4 oder 5;
R38 CH₂OH, CH₂SH, CH₂NH₂, CH(OH)CH₃, CH₂CH₂SCH₃, CH₂CH₂CH₂NH₂, CH₂CH₂CH₂CH₂NH₂, CH₂CH₂CH₂NHC(NH)NH₂, CH₂COOH, CH₂CONH₂, CH₂CH₂COOR44, CH₂CH₂CONH₂, COOH, Phenyl, 4-Hydroxyphenyl, 4-Imidazolyl oder 3-Indolyl;
R39 H;
R40 H, CH₃ oder CH₂CH₃;
R41 und R42 unabhängig voneinander H, CH₃ oder COCH₃; und
R44 H, CH₃ oder CH₂CH₃;
wobei mindestens einer der Reste R7, R8 oder R9 durch die Gruppe -L-G definiert sein muss,
sowie deren pharmazeutisch verträgliche Salze und Trifluoressigsäuresalze.

3. Verbindungen der Formel I nach Anspruch 1 , ausgewählt aus der Gruppe
2,3,4,5,6-Pentahydroxy-hexan-säure-[4-(6,8-dichloro-2-methyl-1,2,3,4-tetrahydro-isochinolin-4-yl)-phenyl]-amid,
2,3,4,5,6-Pentahydroxy-hexan-säure-[3-(6,8-dichloro-2-methyl-1,2,3,4-tetrahydro-isochinolin-4-yl)-phenyl]-amid,
2,3,4,5,6-Pentahydroxy-hexan-säure-[2-(6,8-dichloro-2-methyl-1,2,3,4-tetrahydro-isochinolin-4-yl)-phenyl]-amid,
2,3,4,5,6-Pentahydroxy-hexan-säure-[3-((S)-6,8-dichloro-2-methyl-1,2,3,4-tetrahydro-isochinolin-4-yl)-phenyl]-amid,
2,3,4,5,6-Pentahydroxy-hexan-säure-[3-((R)-6,8-dichloro-2-methyl-1,2,3,4-tetrahydro-isochinolin-4-yl)-phenyl]-amid,
1-[3-(6,8-Dichlor-2-methyl-1,2,3,4-tetrahydro-isochinolin-4-yl)-phenyl]-3-(2-hydroxy-1-hydroxymethyl-ethyl)-harnstoff,
1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff,
1-[3-(6,8-Dichlor-2-methyl-1,2,3,4-tetrahydro-isochinolin-4-yl)-phenyl]-3-(2,3,4,5,6-pentahydroxy-hexyl)-harnstoff,
1-[3-(6,8-Dichlor-2-methyl-1,2,3,4-tetrahydro-isochinolin-4-yl)-phenyl]-3-(2,4,5-trihydroxy-6-hydroxymethyl-tetrahydro-pyran-3-yl)-harnstoff,
{N-[3-(6,8-Dichloro-2-methyl-1,2,3,4-tetrahydro-isochinolin-4-yl)-phenyl]-N'-ethansulfonsäure-2-yl}-harnstoff,
{N-[3-(6,8-Dichloro-2-methyl-1,2,3,4-tetrahydro-isochinolin-4-yl)-phenyl]-N'-(ethyl-2-trimethyl-ammonium}-harnstoff-chlorid,
{N-[3-(6,8-Dichloro-2-methyl-1,2,3,4-tetrahydro-isochinolin-4-yl)-phenyl]-N'-3-hydroxy-propion-säure-2-yl}-harnstoff
{N-[3-(6,8-Dichloro-2-methyl-1,2,3,4-tetrahydro-isochinolin-4-yl)-phenyl]-N'-bernsteinsäure-4-amid-2-yl}-harnstoff,
3-(6,8-Dichlor-2-methyl-1,2,3,4-tetrahydro-isochinolin-4-yl)-N-(2,3,4,5,6-pentahydroxy-hexyl)-benzamid,
3-(6,8-Dichloro-2-methyl-1,2,3,4-tetrahydro-isochinolin-4-yl)-N-(2-hydroxy-1-hydroxymethyl-ethyl)-benzamid,
2-[3-(6,8-Dichloro-2-methyl-1,2,3,4-tetrahydro-isochinolin-4-yl)-benzoylamino]-3-hydroxy-propionsäure,
2-[3-(6,8-Dichloro-2-methyl-1,2,3,4-tetrahydro-isochinolin-4-yl)-benzoylamino]-bernsteinsäure,
2-[3-(6,8-Dichloro-2-methyl-1,2,3,4-tetrahydro-isochinolin-4-yl)-benzoylamino]-bernsteinsäure-4-amid,
N-[3-(6,8-Dichloro-2-methyl-1,2,3,4-tetrahydro-isochinolin-4-yl)-phenyl]-N'-[5-guanidino-pentansäure-2-yl]-harnstoff,
{N-[3-(6,8-Dichloro-2-methyl-1,2,3,4-tetrahydro-isochinolin-4-yl)-phenyl]-N'-bernsteinsäure-4-amid-2-yl}-harnstoff,
{N-[3-(6,8-Dichloro-2-methyl-1,2,3,4-tetrahydro-isochinolin-4-yl)-phenyl]-N'-3-hydroxy-propion-säure-2-yl}-harnstoff,
1-[3-(6,8-Dichloro-2-methyl-1,2,3,4-tetrahydro-isochinolin-4-yl)-phenyl]-3-(2-hydroxy-1,1-bis-hydroxymethyl-ethyl)-harnstoff,
1-[3-(6,8-Dichlor-2-methyl-1,2,3,4-tetrahydro-isochinolin-4-yl)-phenyl]-3-(2,3,4,5,6-pentahydroxy-hexyl)-harnstoff,
5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid,
5-(6,8-Dichloro-2-methyl-1,2,3,4-tetrahydro-isochinolin-4-yl)-N,N'-bis-(2-hydroxy-1,1-bis-hydroxymethyl-ethyl)-isophthalsäure-amid,
5-(6,8-Dichloro-2-methyl-1,2,3,4-tetrahydro-isochinolin-4-yl)-N-(2-hydroxy-1,1-bis-hydroxymethyl-ethyl)-isophthalsäure-amid,
5-(6,8-Dichlor-2-methyl-1,2,3,4-tetrahydro-isochinolin-4-yl)-N,N'-bis-(2,3,4,5,6-pentahydroxy-hexyl)-isophthalsäure-amid,
5-(6,8-Dichlor-2-methyl-1,2,3,4-tetrahydro-isochinolin-4-yl)-N-(2,3,4,5,6-pentahydroxy-hexyl)-isophthalsäure-amid,
2-[3-(1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure,
2-Amino-5-guanidino-pentansäure-[3-(6,8-dichlor-2-methyl-1,2,3,4-tetrahydro-isochinolin-4-yl)-phenyl]-amid,
2-Amino-5-guanidino-pentansäure-[4-(6,8-dichlor-2-methyl-1,2,3,4-tetrahydro-isochinolin-4-yl)-phenyl]-amid,
2-Amino-N-[3-(6,8-dichloro-2-methyl-1,2,3,4-tetrahydro-isochinolin-4-yl)-phenyl]-3-(1H-imidazol-4-yl)-propionsäure-amid und
2-Amino-N-[4-(6,8-dichloro-2-methyl-1,2,3,4-tetrahydro-isochinolin-4-yl)-phenyl]-3-(1H-imidazol-4-yl)-propionsäure-amid ;
sowie deren pharmazeutisch verträgliche Salze und Trifluoressigsäuresalze.

4. Verbindungen der Formel I nach Anspruch 1 , ausgewählt aus der Gruppe
(2R,3S,4R,5R)-2,3,4,5,6-Pentahydroxy-hexan-säure-[4-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[2-(6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-((S)-6,8-dichloro-2-methyl-1,2,3,4-tetrahydro-isochinolin-4-yl)-phenyl]-amid,
(2R,3S,4R,5R)-2,3,4,5,6-Pentahydroxy-hexan-säure-[3-((R)-6,8-dichloro-2-methyl-1,2,3,4-tetrahydro-isochinolin-4-yl)-phenyl]-amid,
1-[3-(6,8-Dichlor-2-methyl-1,2,3,4-tetrahydro-isochinolin-4-yl)-phenyl]-3-(2-hydroxy-1-hydroxymethyl-ethyl)-harnstoff,
1-[3-(6,8-Dichloro-2-methyl-1,2,3,4-tetrahydro-isochinolin-4-yl)-phenyl]-3-(2-hydroxy-1,1-bis-hyd roxymethyl-ethyl)-harnstoff,
1-[3-(6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff,
1-[3-(6,8-Dichlor-2-methyl-1,2,3,4-tetrahydro-isochinolin-4-yl)-phenyl]-3-((4R,5S,6R)-2,4,5-trihydroxy-6-hydroxymethyl-tetrahydro-pyran-3-yl)-harnstoff,
{N-[3-(6,8-Dichloro-2-methyl-1,2,3,4-tetrahydro-isochinolin-4-yl)-phenyl]-N'-ethansulfonsäure-2-yl}-harnstoff,
{N-[3-(6,8-Dichloro-2-methyl-1,2,3,4-tetrahydro-isochinolin-4-yl)-phenyl]-N'-(ethyl-2-trimethyl-ammonium}-harnstoff-chlorid,
{N-[3-(6,8-Dichloro-2-methyl-1,2,3,4-tetrahydro-isochinolin-4-yl)-phenyl]-N'-3-hydroxy-propion-säure-2S-yl}-harnstoff,
{N-[3-(6,8-Dichloro-2-methyl-1,2,3,4-tetrahydro-isochinolin-4-yl)-phenyl]-N'-bernsteinsäure-4-amid-2S-yl}-harnstoff,
3-(6,8-Dichlor-2-methyl-1,2,3,4-tetrahydro-isochinolin-4-yl)-N-((2S,3R,4R,5R)-2,3,4,5,6-pentahydroxy-hexyl)-benzamid,
3-(6,8-Dichloro-2-methyl-1,2,3,4-tetrahydro-isochinolin-4-yl)-N-(2-hydroxy-1-hydroxymethyl-ethyl)-benzamid,
(S)-2-[3-(6,8-Dichloro-2-methyl-1,2,3,4-tetrahydro-isochinolin-4-yl)-benzoylamino]-3-hydroxy-propionsäure,
(S)-2-[3-(6,8-Dichloro-2-methyl-1,2,3,4-tetrahydro-isochinolin-4-yl)-benzoylamino]-bernsteinsäure,
(S)-2-[3-(6,8-Dichloro-2-methyl-1,2,3,4-tetrahydro-isochinolin-4-yl)-benzoylamino]-bernsteinsäure-4-amid,
N-[3-(6,8-Dichloro-2-methyl-1,2,3,4-tetrahydro-isochinolin-4-yl)-phenyl]-N'-[5-guanidino-pentansäure-2S-yl]-harnstoff,
{N-[3-(6,8-Dichloro-2-methyl-1,2,3,4-tetrahydro-isochinolin-4(R)-yl)-phenyl]-N'-bernsteinsäure-4-amid-2S-yl}-harnstoff,
{N-[3-(6,8-Dichloro-2-methyl-1,2,3,4-tetrahydro-isochinolin-4(S)-yl)-phenyl]-N'-bernsteinsäure-4-amid-2S-yl}-harnstoff,
{N-[3-(6,8-Dichloro-2-methyl-1,2,3,4-tetrahydro-isochinolin-4(R)-yl)-phenyl]-N'-3-hydroxy-propion-säure-2S-yl}-harnstoff,
{N-[3-(6,8-Dichloro-2-methyl-1,2,3,4-tetrahydro-isochinolin-4(S)-yl)-phenyl]-N'-3-hydroxy-propion-säure-2S-yl}-harnstoff,
1-[3-(6,8-Dichloro-2-methyl-1,2,3,4-tetrahydro-isochinolin-4(R)-yl)-phenyl]-3-(2-hydroxy-1,1-bis-hydroxymethyl-ethyl)-harnstoff,
1-[3-(6,8-Dichloro-2-methyl-1,2,3,4-tetrahydro-isochinolin-4(S)-yl)-phenyl]-3-(2-hydroxy-1,1-bis-hydroxymethyl-ethyl)-harnstoff,
1-[3-((R)-6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff,
1-[3-((S)-6,8-Dichlor-2-methyl-1,2,3,4-tetrahydro-isochinolin-4-yl)-phenyl]-3-((2S,3R,4R,5R)-2,3,4,5,6-pentahydroxy-hexyl)-harnstoff,
5-(6,8-Dichloro-2-methyl-1,2,3,4-tetrahydro-isochinolin-4-yl)-N,N'-bis-(2-hydroxy-1-hydroxymethyl-ethyl)-isophthalsäure-amid,
5-(6,8-Dichloro-2-methyl-1,2,3,4-tetrahydro-isochinolin-4-yl)-N,N'-bis-(2-hydroxy-1,1-bis-hydroxymethyl-ethyl)-isophthalsäure-amid,
5-(6,8-Dichloro-2-methyl-1,2,3,4-tetrahydro-isochinolin-4-yl)-N-(2-hydroxy-1,1-bis-hydroxymethyl-ethyl)-isophthalsäure-amid,
5-(6,8-Dichlor-2-methyl-1,2,3,4-tetrahydro-isochinolin-4-yl)-N,N'-bis-((2S,3R,4R,5R)-2,3,4,5,6-pentahydroxy-hexyl)-isophthalsäure-amid,
5-(6,8-Dichlor-2-methyl-1,2,3,4-tetrahydro-isochinolin-4-yl)-N-((2S,3R,4R,5R)-2,3,4,5,6-pentahydroxy-hexyl)-isophthalsäure-amid,
(S)-2-[3-((S)-1-Carboxy-2-hydroxy-ethylcarbamoyl)-5-(6,8-dichlor-2-methyl-1,2,3,4-tetrahydro-isochinolin-4-yl)-benzoylamino]-3-hydroxy-propion-säure, (S)-2-Amino-5-guanidino-pentansäure-[3-(6,8-dichlor-2-methyl-1,2,3,4-tetrahydro-isochinolin-4-yl)-phenyl]-amid,
(S)-2-Amino-5-guanidino-pentansäure-[4-(6,8-dichlor-2-methyl-1,2,3,4-tetrahydro-isochinolin-4-yl)-phenyl]-amid,
(S)-2-Amino-N-[3-(6,8-dichloro-2-methyl-1,2,3,4-tetrahydro-isochinolin-4-yl)-phenyl]-3-(1H-imidazol-4-yl)-propionsäure-amid und
(S)-2-Amino-N-[4-(6,8-dichloro-2-methyl-1,2,3,4-tetrahydro-isochinolin-4-yl)-phenyl]-3-(1H-imidazol-4-yl)-propionsäure-amid;
sowie deren pharmazeutisch verträgliche Salze und Trifluoressigsäuresalze.

5. Verbindungen der Formel I und deren pharmazeutisch verträgliche Salze nach einem oder mehreren der Ansprüche 1 bis 4 zur Verwendung als Medikament.

6. Verbindungen der Formel I und deren pharmazeutisch verträgliche Salze nach einem oder mehreren der Ansprüche 1 bis 4 zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

7. Heilmittel für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 4.

8. Heilmittel für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 4, in Kombination mit anderen pharmakologischen Wirkstoffen oder Arzneimitteln.

## Claims

1. A compound of the formula I 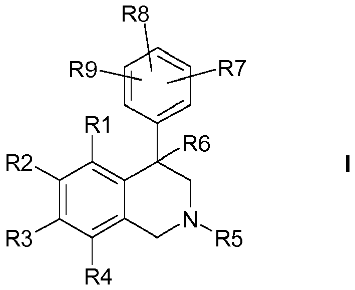 where:
R1 and R3 are H and R2 and R4 are Cl;
R5 is H, CH₃, CH₂CH₃, CF₃ or CH₂CF₃;
R6 is H, OH, CH₃, CF₃, OCH₃, OCOCH₃;
R7, R8 and R9 are each independently H, F, Cl, Br, I, OH, CH₃, CH₂CH₃, CF₃, CH₂CF₃, OCH₃, OCH₂CH₃, OCF₃, OCH₂CF₃, SO₂R47, SO₃R60, COR47, COOR60, NR51R52 or a -L-G group;
R47 is H, CH₃, CH₂CH₃, CF₃, CH₂CF₃ or NR48R49;
R48 and R49 are each independently H, CH₃, CH₂CH₃, CF₃, CH₂CF₃, COCH₃, COCH₂CH₃, COCF₃ or COCH₂CF₃
R60 is H, CH₃, CH₂CH₃, CF₃, CH₂CF₃;
R51 and R52 are each independently H, CH₃, CH₂CH₃, CF₃, CH₂CF₃, COCH₃, COCH₂CH₃, COCF₃ or COCH₂CF₃;
L is -NR30-, -NR30CO-, -CONR30-, -NR30CONR31-, -NR30COO-;
R30 and R31 are each independently H, CH₃, CH₂CH₃, CF₃ or CH₂CF₃;
G is a Cₐ(OR32)ₓH₂ₐ₊₁₋ₓ group where one or more CH₂ groups may be replaced by 0 or NR33, C_{b}(OR32)_{y}H_{2b-1-y} where one or more CH₂ groups may be replaced by 0 or NR33, -(CH₂)_{z}-COOR34, -(CH₂)_{z}-SO₃R34, -(CH₂)_{z}-N⁺R35R36R37 where 1 or 2 hydrogen atoms of the -(CH₂)_{z} units may be replaced by OR32 groups, -CR38R39-COOR40 or -CR38R39NR41R42;
a is 2, 3, 4, 5, 6, 7 or 8;
x is 2, 3, 4, 5, 6, 7 or 8;
R32 is H, CH₃, CH₂CH₃, CF₃, CH₂CF₃, COCH₃, COCH₂CH₃, COCF₃ or COCH₂CF₃;
R33 is H, CH₃, CH₂CH₃, CF₃ or CH₂CF₃;
b is 3, 4, 5, 6 or 7;
y is 2, 3, 4, 5, 6 or 7;
z is 1 or 2;
R34, R35, R36 and R37 are each independently H, CH₃, CH₂CH₃, CF₃ or CH₂CF₃;
R38 is -(CH₂)ₙ -Y;
n is 0, 1, 2, 3 or 4;
Y is alkyl having 1, 2, 3 or 4 carbon atoms of which some or all may be fluorinated and one or more CH₂ groups are replaced by 0, S or NR43, or is COOR44, CONR45R46, NHC(NH)NH₂, phenyl or heteroaryl selected from the group of imidazolyl, pyrazolyl, pyrrolyl, triazolyl, tetrazolyl, thiazolyl, oxazolyl and pyridyl, and the phenyl and heteroaryl radicals may be substituted by up to 3 substituents selected from the group of CH₃, CF₃, OH, OCH₃ or NH2;
R43, R44, R45 and R46 are each independently H, CH₃, CH₂CH₃, CF₃ or CH₂CF₃;
R39 is H, CH₃, CH₂CH₃, CF₃ or CH₂CF₃;
R40 is H, CH₃, CH₂CH₃, CF₃ or CH₂CF₃;
R41 and R42 are each independently H, CH₃, CH₂CH₃, CF₃, CH₂CF₃, COCH₃, COCH₂CH₃, COCF₃ or COCH₂CF₃;
in which at least one of the R7, R8 or R9 radicals has to be defined by the -L-G group,
and also its pharmaceutically acceptable salts and trifluoroacetates.

2. A compound of the formula I as claimed in claim 1 where
R1 and R3 are H and R2 and R4 are Cl;
R5 is CH₃;
R6 is H;
R7, R8 and R9 are each independently H, OH, CH₃, CF₃, OCH₃, SO₂R47, SO₃R60, COR47, COOR60, NR51R52 or a -L-G group;
R47 is H, CH₃ or NR48R49;
R48 and R49 are each independently H, CH₃ or COCH₃,
R60 is H, CH₃;
R51 and R52 are each independently H, CH₃, CH₂CH₃ or COCH₃
L is -NR30CO-, -CONR30-, -NR30CONR31-R30 and R31 are H;
G is a group Cₐ(OR32)ₓH₂ₐ₊₁₋ₓ where one or more CH₂ groups may be replaced by 0 or NR33, C_{b}(OR32)_{y}H_{2b-1-y} where one or more CH₂ groups may be replaced by 0 or NR33, -(CH₂)₂-COOH, -(CH₂)₂-SO₃H, -(CH₂)₂-N(CH₃)₃⁺ where 1 or 2 hydrogen atoms of the -(CH₂)₂ units may be replaced by OH groups, -CR38R39-COOR40 or -CR38R39NR41R42;
a is 3, 4, 5 or 6;
x is 2, 3, 4 or 5;
R32 is H;
R33 is H or CH₃;
b is 5 or 6;
y is 2, 3, 4 or 5;
R38 is CH₂OH, CH₂SH, CH₂NH₂, CH(OH)CH₃, CH₂CH₂SCH₃, CH₂CH₂CH₂NH₂, CH₂CH₂CH₂CH₂NH₂, CH₂CH₂CH₂NHC(NH)NH₂, CH₂COOH, CH₂CONH₂, CH₂CH₂COOR44, CH₂CH₂CONH₂, COOH, phenyl, 4-hydroxy-phenyl, 4-imidazolyl or 3-indolyl;
R39 is H;
R40 is H, CH₃ or CH₂CH₃;
R41 and R42 are each independently H, CH₃ or COCH₃; and
R44 is H, CH₃ or CH₂CH₃;
in which at least one of the R7, R8 or R9 radicals has to be defined by the -L-G group,
and also its pharmaceutically acceptable salts and trifluoroacetates.

3. A compound of the formula I as claimed in claim 1, selected from the group of
N-[4-(6,8-dichloro-2-methyl-1,2,3,4-tetrahydroisoquinolin-4-yl)phenyl]-2,3,4,5,6-pentahydroxyhexanamide,
N-[3-(6,8-dichloro-2-methyl-1,2,3,4-tetrahydroisoquinolin-4-yl)phenyl]-2,3,4,5,6-pentahydroxyhexanamide,
N-[2-(6,8-dichloro-2-methyl-1,2,3,4-tetrahydroisoquinolin-4-yl)phenyl]-2,3,4,5,6-pentahydroxyhexanamide,
N-[3-((S)-6,8-dichloro-2-methyl-1,2,3,4-tetrahydroisoquinolin-4-yl)phenyl]-2,3,4,5,6-pentahydroxyhexanamide,
N-[3-((R)-6,8-dichloro-2-methyl-1,2,3,4-tetrahydroisoquinolin-4-yl)phenyl]-2,3,4,5,6-pentahydroxyhexanamide,
1-[3-(6,8-dichloro-2-methyl-1,2,3,4-tetrahydroisoquinolin-4-yl)phenyl]-3-(2-hydroxy-1-hydroxymethylethyl)urea,
1-[3-(6,8-dichloro-2-methyl-1,2,3,4-tetrahydroisoquinolin-4-yl)phenyl]-3-(2-hydroxy-1,1-bishydroxymethylethyl)urea,
1-[3-(6,8-dichloro-2-methyl-1,2,3,4-tetrahydroisoquinolin-4-yl)phenyl]-3-(2,3,4,5,6-pentahydroxyhexyl)urea,
1-[3-(6,8-dichloro-2-methyl-1,2,3,4-tetrahydroisoquinolin-4-yl)phenyl]-3-(2,4,5-trihydroxy-6-hydroxymethyltetrahydropyran-3-yl)urea,
{N-[3-(6,8-dichloro-2-methyl-1,2,3,4-tetrahydroisoquinolin-4-yl)phenyl]-N'-(1-sulfo-2-ethyl)}urea,
{N-[3-(6,8-dichloro-2-methyl-1,2,3,4-tetrahydroisoquinolin-4-yl)phenyl]-N'-(ethyl-2-trimethylammonium)}urea chloride,
{N-[3-(6,8-dichloro-2-methyl-1,2,3,4-tetrahydroisoquinolin-4-yl)phenyl]-N'(1-carboxy-3-hydroxy-2-propyl)}urea,
{N-[3-(6,8-dichloro-2-methyl-1,2,3,4-tetrahydroisoquinolin-4-yl)phenyl]-N'-(1-carboxy-4-aminocarboxy-2-butyl)}urea,
3-(6,8-dichloro-2-methyl-1,2,3,4-tetrahydroisoquinolin-4-yl)-N-(2,3,4,5,6-pentahydroxyhexyl)benzamide,
3-(6,8-dichloro-2-methyl-1,2,3,4-tetrahydroisoquinolin-4-yl)-N-(2-hydroxy-1-hydroxymethylethyl)benzamide,
2-[3-(6,8-dichloro-2-methyl-1,2,3,4-tetrahydroisoquinolin-4-yl)benzoylamino]-3-hydroxypropionic acid,
2-[3-(6,8-dichloro-2-methyl-1,2,3,4-tetrahydroisoquinolin-4-yl)benzoylamino]succinic acid,
2-[3-(6,8-dichloro-2-methyl-1,2,3,4-tetrahydroisoquinolin-4-yl)benzoylamino]-4-succinamic acid,
N-[3-(6,8-dichloro-2-methyl-1,2,3,4-tetrahydroisoquinolin-4-yl)phenyl]-N'-[1-carboxy-5-guanidino-2-pentyl]urea,
{N-[3-(6,8-dichloro-2-methyl-1,2,3,4-tetrahydroisoquinolin-4-yl)phenyl]-N'-(1-carboxy-4-aminocarboxy-2-butyl)}urea,
{N-[3-(6,8-dichloro-2-methyl-1,2,3,4-tetrahydroisoquinolin-4-yl)phenyl]-N'-(1-carboxy-3-hydroxy-2-propyl)}urea,
1-[3-(6,8-dichloro-2-methyl-1,2,3,4-tetrahydroisoquinolin-4-yl)phenyl]-3-(2-hydroxy-1,1-bishydroxymethylethyl)urea,
1-[3-(6,8-dichloro-2-methyl-1,2,3,4-tetrahydroisoquinolin-4-yl)phenyl]-3-(2,3,4,5,6-pentahydroxyhexyl)urea,
5-(6,8-dichloro-2-methyl-1,2,3,4-tetrahydroisoquinolin-4-yl)-N,N'-bis(2-hydroxy-1-hydroxymethylethyl)isophthalamide,
5-(6,8-dichloro-2-methyl-1,2,3,4-tetrahydroisoquinolin-4-yl)-N,N'-bis(2-hydroxy-1,1-bishydroxymethylethyl)isophthalamide,
5-(6,8-dichloro-2-methyl-1,2,3,4-tetrahydroisoquinolin-4-yl)-N-bis(2-hydroxy-1,1-bishydroxymethylethyl)isophthalamide,
5-(6,8-dichloro-2-methyl-1,2,3,4-tetrahydroisoquinolin-4-yl)-N,N'-bis(2,3,4,5,6-pentahydroxyhexyl)isophthalamide,
5-(6,8-dichloro-2-methyl-1,2,3,4-tetrahydroisoquinolin-4-yl)-N-(2,3,4,5,6-pentahydroxyhexyl)isophthalamide,
2-[3-(1-carboxy-2-hydroxyethylcarbamoyl)-5-(6,8-dichloro-2-methyl-1,2,3,4-tetrahydroisoquinolin-4-yl)benzoylamino]-3-hydroxypropionic acid,
N-[3-(6,8-dichloro-2-methyl-1,2,3,4-tetrahydroisoquinolin-4-yl)phenyl]-2-amino-5-guanidinopentanamide,
N-[4-(6,8-dichloro-2-methyl-1,2,3,4-tetrahydroisoquinolin-4-yl)phenyl]-2-amino-5-guanidinopentanamide,
2-amino-N-[3-(6,8-dichloro-2-methyl-1,2,3,4-tetrahydroisoquinolin-4-yl)phenyl]-3-(1H-imidazol-4-yl)propionamide, and
2-amino-N-[4-(6,8-dichloro-2-methyl-1,2,3,4-tetrahydroisoquinolin-4-yl)phenyl]-3-(1H-imidazol-4-yl)propionamide;
and their pharmaceutically acceptable salts and trifluoroacetates.

4. A compound of the formula I as claimed in claim 1, selected from the group of
N-[4-(6,8-dichloro-2-methyl-1,2,3,4-tetrahydroisoquinolin-4-yl)phenyl]-(2R,3S,4R,5R)-2,3,4,5,6-pentahydroxyhexanamide,
N-[3-(6,8-dichloro-2-methyl-1,2,3,4-tetrahydroisoquinolin-4-yl)phenyl]-(2R,3S,4R,5R)-2,3,4,5,6-pentahydroxyhexanamide,
N-[2-(6,8-dichloro-2-methyl-1,2,3,4-tetrahydroisoquinolin-4-yl)phenyl]-(2R,3S,4R,5R)-2,3,4,5,6-pentahydroxyhexanamide,
N-[3-((S)-6,8-dichloro-2-methyl-1,2,3,4-tetrahydroisoquinolin-4-yl)phenyl]-(2R,3S,4R,5R)-2,3,4,5,6-pentahydroxyhexanamide,
N-[3-((R)-6,8-dichloro-2-methyl-1,2,3,4-tetrahydroisoquinolin-4-yl)phenyl]-(2R,3S,4R,5R)-2,3,4,5,6-pentahydroxyhexanamide,
1-[3-(6,8-dichloro-2-methyl-1,2,3,4-tetrahydroisoquinolin-4-yl)phenyl]-3-(2-hydroxy-1-hydroxymethylethyl)urea,
1-[3-(6,8-dichloro-2-methyl-1,2,3,4-tetrahydroisoquinolin-4-yl)phenyl]-3-(2-hydroxy-1,1-bishydroxymethylethyl)urea,
1-[3-(6,8-dichloro-2-methyl-1,2,3,4-tetrahydroisoquinolin-4-yl)phenyl]-3-((2S,3R,4R,5R)-2,3,4,5,6-pentahydroxyhexyl)urea,
1-[3-(6,8-dichloro-2-methyl-1,2,3,4-tetrahydroisoquinolin-4-yl)phenyl]-3-((4R,5S,6R)-2,4,5-trihydroxy-6-hydroxymethyltetrahydropyran-3-yl)urea,
{N-[3-(6,8-dichloro-2-methyl-1,2,3,4-tetrahydroisoquinolin-4-yl)phenyl]-N'-(1-sulfo-2-ethyl)}urea,
{N-[3-(6,8-dichloro-2-methyl-1,2,3,4-tetrahydroisoquinolin-4-yl)phenyl]-N'-(ethyl-2-trimethylammonium)}urea chloride,
{N-[3-(6,8-dichloro-2-methyl-1,2,3,4-tetrahydroisoquinolin-4-yl)phenyl]-N'-(1-carboxy-3-hydroxy-2S-propyl)}urea,
{N-[3-(6,8-dichloro-2-methyl-1,2,3,4-tetrahydroisoquinolin-4-yl)phenyl]-N'-(1-carboxy-4-aminocarboxy-2S-butyl)}urea,
3-(6,8-dichloro-2-methyl-1,2,3,4-tetrahydroisoquinolin-4-yl)-N-((2S,3R,4R,5R)-2,3,4,5,6-pentahydroxyhexyl)benzamide,
3-(6,8-dichloro-2-methyl-1,2,3,4-tetrahydroisoquinolin-4-yl)-N-(2-hydroxy-1-hydroxymethylethyl)benzamide,
2-(S)-[3-(6,8-dichloro-2-methyl-1,2,3,4-tetrahydroisoquinolin-4-yl)benzoylamino]-3-hydroxypropionic acid,
2-(S)-[3-(6,8-dichloro-2-methyl-1,2,3,4-tetrahydroisoquinolin-4-yl)benzoylamino]succinic acid,
2-(S)-[3-(6,8-dichloro-2-methyl-1,2,3,4-tetrahydroisoquinolin-4-yl)benzoylamino]-4-succinamic acid,
N-[3-(6,8-dichloro-2-methyl-1,2,3,4-tetrahydroisoquinolin-4-yl)phenyl]-N'-[1-carboxy-5-guanidino-2S-pentyl]urea,
{N-[3-(6,8-dichloro-2-methyl-1,2,3,4-tetrahydroisoquinolin-4-(R)-yl)phenyl]-N'-(1-carboxy-4-aminocarboxy-2S-butyl)}urea,
{N-[3-(6,8-dichloro-2-methyl-1,2,3,4-tetrahydroisoquinolin-4-(S)-yl)phenyl]-N'-(1-carboxy-4-aminocarboxy-2S-butyl)}urea,
{N-[3-(6,8-dichloro-2-methyl-1,2,3,4-tetrahydroisoquinolin-4-(R)-yl)phenyl]-N'-(1-carboxy-3-hydroxy-2S-propyl)}urea,
{N-[3-(6,8-dichloro-2-methyl-1,2,3,4-tetrahydroisoquinolin-4-(S)-yl)phenyl]-N'-(1-carboxy-3-hydroxy-2S-propyl)}urea,
1-[3-(6,8-dichloro-2-methyl-1,2,3,4-tetrahydroisoquinolin-4-(R)-yl)phenyl]-3-(2-hydroxy-1,1-bishydroxymethylethyl)urea,
1-[3-(6,8-dichloro-2-methyl-1,2,3,4-tetrahydroisoquinolin-4-(S)-yl)phenyl]-3-(2-hydroxy-1,1-bishydroxymethylethyl)urea,
1-[3-((R)-6,8-dichloro-2-methyl-1,2,3,4-tetrahydroisoquinolin-4-yl)phenyl]-3-((2S,3R,4R,5R)-2,3,4,5,6-pentahydroxyhexyl)urea,
1-[3-((S)-6,8-dichloro-2-methyl-1,2,3,4-tetrahydroisoquinolin-4-yl)phenyl]-3-((2S,3R,4R,5R)-2,3,4,5,6-pentahydroxyhexyl)urea,
5-(6,8-dichloro-2-methyl-1,2,3,4-tetrahydroisoquinolin-4-yl)-N,N'-bis(2-hydroxy-1-hydroxymethylethyl)isophthalamide,
5-(6,8-dichloro-2-methyl-1,2,3,4-tetrahydroisoquinolin-4-yl)-N,N'-bis(2-hydroxy-1,1-bishydroxymethylethyl)isophthalamide,
5-(6,8-dichloro-2-methyl-1,2,3,4-tetrahydroisoquinolin-4-yl)-N-(2-hydroxy-1,1-bishydroxymethylethyl)isophthalamide,
5-(6,8-dichloro-2-methyl-1,2,3,4-tetrahydroisoquinolin-4-yl)-N,N'-bis((2S,3R,4R,5R)-2,3,4,5,6-pentahydroxyhexyl)isophthalamide,
5-(6,8-dichloro-2-methyl-1,2,3,4-tetrahydroisoquinolin-4-yl)-N-((2S,3R,4R,5R)-2,3,4,5,6-pentahydroxyhexyl)isophthalamide,
(S)-2-[3-((S)-1-carboxy-2-hydroxyethylcarbamoyl)-5-(6,8-dichloro-2-methyl-1,2,3,4-tetrahydroisoquinolin-4-yl)benzoylamino]-3-hydroxypropionic acid,
(S)-N-[3-(6,8-dichloro-2-methyl-1,2,3,4-tetrahydroisoquinolin-4-yl)phenyl]-2-amino-5-guanidinopentanamide,
(S)-N-[4-(6,8-dichloro-2-methyl-1,2,3,4-tetrahydroisoquinolin-4-yl)phenyl]-2-amino-5-guanidinopentanamide,
(S)-2-amino-N-[3-(6,8-dichloro-2-methyl-1,2,3,4-tetrahydroisoquinolin-4-yl)phenyl]-3-(1H-imidazol-4-yl)propionamide, and
(S)-2-amino-N-[4-(6,8-dichloro-2-methyl-1,2,3,4-tetrahydroisoquinolin-4-yl)phenyl]-3-(1H-imidazol-4-yl)propionamide;
and their pharmaceutically acceptable salts and trifluoroacetates.

5. A compound of the formula I and its pharmaceutically acceptable salts as claimed in one or more of claims 1 to 4 for use as a medicament.

6. A compound of the formula I and the pharmaceutically acceptable salts thereof as claimed in one or more of claims 1 to 4 for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of intestinal function, of high blood pressure, of essential hypertension, of disorders of the central nervous system, of disorders resulting from CNS overexcitability, epilepsy and centrally induced convulsions or of anxiety states, depressions and psychoses, of ischemic states of the peripheral or central nervous system and of stroke, of acute and chronic damage to and disorders of peripheral organs or limbs caused by ischemic or by reperfusion events, of atherosclerosis, of disorders of lipid metabolism, of thromboses, of disorders of biliary function, of infestation by ectoparasites, of disorders resulting from endothelial dysfunction, of protozoal disorders, of malaria, for the preservation and storage of transplants for surgical procedures, for use in surgical operations and organ transplantations or for the treatment of states of shock or of diabetes and late damage from diabetes or of diseases in which cellular proliferation represents a primary or secondary cause, and for maintaining health and prolonging life.

7. A curative composition for human, veterinary or phytoprotective use comprising an effective amount of a compound of the formula I and/or of a pharmaceutically acceptable salt thereof as claimed in one or more of claims 1 to 4.

8. A curative composition for human, veterinary or phytoprotective use comprising an effective amount of a compound of the formula I and/or of a pharmaceutically acceptable salt thereof as claimed in one or more of claims 1 to 4, in combination with other pharmacological active ingredients or medicaments.

## Revendications

1. Composé de formule I 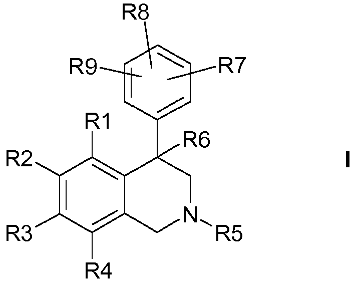 où
R1 et R3 représentent H et R2 et R4 représentent Cl ;
R5 représente H, CH₃, CH₂CH₃, CF₃ ou CH₂CF₃ ;
R6 représente H, OH, CH₃, CF₃, OCH₃ ou OCOCH₃ ;
R7, R8 et R9 représentent chacun indépendamment H, F, Cl, Br, I, OH, CH₃, CH₂CH₃, CF₃, CH₂CF₃, OCH₃, OCH₂CH₃, OCF₃, OCH₂CF₃, SO₂R47, SO₃R60, COR47, COOR60, NR51R52 ou un groupe -L-G ;
R47 représente H, CH₃, CH₂CH₃, CF₃, CH₂CF₃ ou NR48R49 ;
R48 et R49 représentent chacun indépendamment H, CH₃, CH₂CH₃, CF₃, CH₂CF₃, COCH₃, COCH₂CH₃, COCF₃ ou COCH₂CF₃ ;
R60 représente H, CH₃, CH₂CH₃, CF₃, CH₂CF₃ ;
R51 et R52 représentent chacun indépendamment H, CH₃, CH₂CH₃, CF₃, CH₂CF₃, COCH₃, COCH₂CH₃, COCF₃ ou COCH₂CF₃ ;
L représente -NR30-, -NR30CO-, -CONR30-, -NR30CONR31-, -NR30COO- ;
R30 et R31 représentent chacun indépendamment H, CH₃, CH₂CH₃, CF₃ ou CH₂CF₃ ;
G représente un groupe Cₐ(OR32)ₓH₂ₐ₊₁₋ₓ où un ou plusieurs groupes CH₂ peuvent être remplacés par 0 ou NR33,
un groupe C_{b}(OR32)_{y}H_{2b-1-y} où un ou plusieurs groupes CH₂ peuvent être remplacés par 0 ou NR33,
-(CH₂)_{z}-COOR34, -(CH₂)_{z} -SO₃R34, -(CH₂)_{z}-N⁺R35R36R37 où 1 ou 2 atomes d'hydrogène des motifs -(CH₂)_{z} peuvent être remplacés par des groupes OR32, -CR38R39-COOR40 ou -CR38R39NR41R42 ;
a est égal à 2, 3, 4, 5, 6, 7 ou 8 ;
x est égal à 2, 3, 4, 5, 6, 7 ou 8 ;
R32 représente H, CH₃, CH₂CH₃, CF₃, CH₂CF₃, COCH₃, COCH₂CH₃, COCF₃ ou COCH₂CF₃ ;
R33 représente H, CH₃, CH₂CH₃, CF₃ ou CH₂CF₃ ;
b est égal à 3, 4, 5, 6 ou 7 ;
y est égal à 2, 3, 4, 5, 6 ou 7 ;
z est égal à 1 ou 2 ;
R34, R35, R36 et R37 représentent chacun indépendamment H, CH₃, CH₂CH₃, CF₃ ou CH₂CF₃ ;
R38 représente -(CH₂)ₙ -Y;
n est égal à 0, 1, 2, 3 ou 4 ;
Y représente un groupe alkyle ayant 1, 2, 3 ou 4 atomes de carbone parmi lesquels la totalité ou quelques-uns peuvent être fluorés et un ou plusieurs groupes CH₂ peuvent être remplacés par 0, S ou NR43, ou représente un groupe COOR44, CONR45R46, NHC(NH)NH₂, phényle ou hétéroaryle choisis parmi le groupe constitué des groupes imidazolyle, pyrazolyle, pyrrolyle, triazolyle, tetrazolyle, thiazolyle, oxazolyle et pyridyle où les radicaux phényle et hétéroaryle peuvent être substitués par jusqu'à 3 substituants choisis parmi le groupe constitué du groupe CH₃, CF₃, OH, OCH₃ ou NH₂ ;
R43, R44, R45 et R46 représentent chacun indépendamment H, CH₃, CH₂CH₃, CF₃ ou CH₂CF₃ ;
R39 représente H, CH₃, CH₂CH₃, CF₃ ou CH₂CF₃ ;
R40 représente H, CH₃, CH₂CH₃, CF₃ ou CH₂CF₃ ;
R41 et R42 représentent chacun indépendamment H, CH₃, CH₂CH₃, CF₃, CH₂CF₃, COCH₃, COCH₂CH₃, COCF₃ ou COCH₂CF₃ ;
dans lesquels au moins un des radicaux R7, R8 ou R9 doit être définit par le groupe -L-G, et également ses sels et sels trifluoroacétates pharmaceutiquement acceptables.

2. Composé de formule I selon la revendication 1 où R1 et R3 représentent H et R2 et R4 représentent Cl ;
R5 représente un groupe CH₃ ;
R6 représente un atome H ;
R7, R8 et R9 représentent chacun indépendamment H, OH, CH₃, CF₃, OCH₃, SO₂R47, SO₃R60, COR47, COOR60, NR51R52 ou un groupe -L-G ;
R47 représente H, CH₃ ou NR48R49 ;
R48 et R49 représentent chacun indépendamment H, CH₃ ou COCH₃,
R60 représente H, CH₃ ;
R51 et R52 représentent chacun indépendamment H, CH₃, CH₂CH₃ ou COCH₃,
L représente -NR30CO-, -CONR30-, -NR30CONR31- ;
R30 et R31 représentent H ;
G représente un groupe Cₐ(OR32)ₓH₂ₐ₊₁₋ₓ où un ou plusieurs groupes CH₂ peuvent être remplacés par 0 ou NR33,
C_{b}(OR32)_{y}H_{2b-1-y} où un ou plusieurs groupes CH₂ peuvent être remplacés par 0 ou NR33,
-(CH₂)₂-COOH, -(CH₂)₂-SO₃H, -(CH₂)₂-N(CH₃)₃⁺ où 1 ou 2 atomes d'hydrogène des motifs -(CH₂)₂ peuvent être remplacés par des groupes OH, -CR38R39-COOR40 ou -CR38R39NR41R42 ;
a est égal à 3, 4, 5 ou 6 ;
x est égal à 2, 3, 4 ou 5 ;
R32 représente H ;
R33 représente H ou CH₃ ;
b est égal à 5 ou 6 ;
y est égal à 2, 3, 4 ou 5 ;
R38 représente CH₂OH, CH₂SH, CH₂NH₂, CH(OH)CH₃, CH₂CH₂SCH₃, CH₂CH₂CH₂NH₂, CH₂CH₂CH₂CH₂NH₂, CH₂CH₂CH₂NHC(NH)NH₂, CH₂COOH, CH₂CONH₂, CH₂CH₂COOR44, CH₂CH₂CONH₂, COOH, phényle, 4-hydroxyphényle, 4-imidazolyle ou 3-indolyle ;
R39 représente un atome H ;
R40 représente H, CH₃ ou CH₂CH₃ ;
R41 et R42 représentent chacun indépendamment H, CH₃ ou COCH₃ ; et
R44 représente H, CH₃ ou CH₂CH₃ ;
dans lesquels au moins un des radicaux R7, R8 ou R9 doit être défini par le groupe -L-G, et également ses sels et sels trifluoroacétates pharmaceutiquement acceptables.

3. Composé de formule 1 selon la revendication 1, choisi parmi le groupe constitué
du N-[4-(6,8-dichloro-2-méthyl-1,2,3,4-tétrahydroisoquinolin-4-yl)phényl]-2,3,4,5,6-pentahydroxyhexanamide,
du N-[3-(6,8-dichloro-2-méthyl-1,2,3,4-tétrahydroisoquinolin-4-yl)phényl]-2,3,4,5,6-pentahydroxyhexanamide,
du N-[2-(6,8-dichloro-2-méthyl-1,2,3,4-tétrahydroisoquinolin-4-yl)phényl]-2,3,4,5,6-pentahydroxyhexanamide,
du N-[3-((S)-6,8-dichloro-2-méthyl-1,2,3,4-tétrahydroisoquinolin-4-yl)phényl]-2,3,4,5,6-pentahydroxyhexanamide,
du N-[3-((R)-6,8-dichloro-2-méthyl-1,2,3,4-tétrahydroisoquinolin-4-yl)phényl]-2,3,4,5,6-pentahydroxyhexanamide,
de la 1-[3-(6,8-dichloro-2-méthyl-1,2,3,4-tétrahydroisoquinolin-4-yl)phényl]-3-(2-hydroxy-1-hydroxyméthyléthyl)urée,
de la 1-[3-(6,8-dichloro-2-méthyl-1,2,3,4-tétrahydroisoquinolin-4-yl)phényl]-3-(2-hydroxy-1,1-bishydroxyméthyléthyl)urée,
de la 1-[3-(6,8-dichloro-2-méthyl-1,2,3,4-tétrahydroisoquinolin-4-yl)phényl]-3-(2,3,4,5,6-pentahydroxyhexyl)urée,
de la 1-[3-(6,8-dichloro-2-méthyl-1,2,3,4-tétrahydroisoquinolin-4-yl)phényl]-3-(2,4,5-trihydroxy-6-hydroxyméthyltétrahydropyran-3-yl)urée,
de la {N-[3-(6,8-dichloro-2-méthyl-1,2,3,4-tétrahydroisoquinolin-4-yl)phényl]-N'-(1-sulfo-2-éthyl)}urée,
du chlorure de {N-[3-(6,8-dichloro-2-méthyl-1,2,3,4-tétrahydroisoquinolin-4-yl)phényl]-N'-(éthyl-2-triméthylammonium)}urée,
de la {N-[3-(6,8-dichloro-2-méthyl-1,2,3,4-tétrahydroisoquinolin-4-yl)phényl]-N'(1-carboxy-3-hydroxy-2-propyl)}urée,
de la {N-[3-(6,8-dichloro-2-méthyl-1,2,3,4-tétrahydroisoquinolin-4-yl)phényl]-N'-(1-carboxy-4-aminocarboxy-2-butyl)}urée,
du 3-(6,8-dichloro-2-méthyl-1,2,3,4-tétrahydroisoquinolin-4-yl)-N-(2,3,4,5,6-pentahydroxyhexyl)benzamide,
du 3-(6,8-dichloro-2-méthyl-1,2,3,4-tétrahydroisoquinolin-4-yl)-N-(2-hydroxy-1-hydroxyméthyléthyl)benzamide,
de l'acide 2-[3-(6,8-dichloro-2-méthyl-1,2,3,4-tétrahydroisoquinolin-4-yl)benzoylamino]-3-hydroxypropionique,
de l'acide 2-[3-(6,8-dichloro-2-méthyl-1,2,3,4-tétrahydroisoquinolin-4-yl)benzoylamino]succinique,
de l'acide 2-[3-(6,8-dichloro-2-méthyl-1,2,3,4-tétrahydroisoquinolin-4-yl)benzoylamino]-4-succinamique,
de la N-[3-(6,8-dichloro-2-méthyl-1,2,3,4-tétrahydroisoquinolin-4-yl)phényl]-N'-[1-carboxy-5-guanidino-2-pentyl]urée,
de la {N-[3-(6,8-dichloro-2-méthyl-1,2,3,4-tétrahydroisoquinolin-4-yl)phényl]-N'-(1-carboxy-4-aminocarboxy-2-butyl)}urée,
de la {N-[3-(6,8-dichloro-2-méthyl-1,2,3,4-tétrahydroisoquinolin-4-yl)phényl]-N'-(1-carboxy-3-hydroxy-2-propyl)}urée,
de la 1-[3-(6,8-dichloro-2-méthyl-1,2,3,4-tétrahydroisoquinolin-4-yl)phényl]-3-(2-hydroxy-1,1-bishydroxyméthyléthyl)urée,
de la 1-[3-(6,8-dichloro-2-méthyl-1,2,3,4-tétrahydroisoquinolin-4-yl)phényl]-3-(2,3,4,5,6-pentahydroxyhexyl)urée,
du 5-(6,8-dichloro-2-méthyl-1,2,3,4-tétrahydroisoquinolin-4-yl)-N,N'-bis(2-hydroxy-1-hydroxyméthyléthyl)isophthalamide,
du 5-(6,8-dichloro-2-méthyl-1,2,3,4-tétrahydroisoquinolin-4-yl)-N,N'-bis(2-hydroxy-1,1-bishydroxyméthyléthyl)isophthalamide,
du 5-(6,8-dichloro-2-méthyl-1,2,3,4-tétrahydroisoquinolin-4-yl)-N-(2-hydroxy-1,1-bishydroxyméthyléthyl)isophthalamide,
du 5-(6,8-dichloro-2-méthyl-1,2,3,4-tétrahydroisoquinolin-4-yl)-N,N'-bis(2,3,4,5,6-pentahydroxyhexyl)isophthalamide,
du 5-(6,8-dichloro-2-méthyl-1,2,3,4-tétrahydroisoquinolin-4-yl)-N-(2,3,4,5,6-pentahydroxyhexyl)isophthalamide,
de l'acide 2-[3-(1-carboxy-2-hydroxyéthylcarbamoyl)-5-(6,8-dichloro-2-méthyl-1,2,3,4-tétrahydroisoquinolin-4-yl)benzoylamino]-3-hydroxypropionique,
du N-[3-(6,8-dichloro-2-méthyl-1,2,3,4-tétrahydroisoquinolin-4-yl)phényl]-2-amino-5-guanidinopentanamide,
du N-[4-(6,8-dichloro-2-méthyl-1,2,3,4-tétrahydroisoquinolin-4-yl)phényl]-2-amino-5-guanidinopentanamide,
du 2-amino-N-[3-(6,8-dichloro-2-méthyl-1,2,3,4-tétrahydroisoquinolin-4-yl)phényl]-3-(1H-imidazol-4-yl)propionamide,
du 2-amino-N-[4-(6,8-dichloro-2-méthyl-1,2,3,4-tétrahydroisoquinolin-4-yl)phényl]-3-(1H-imidazol-4-yl)propionamide,
et ses sels et sels trifluoroacétates pharmaceutiquement acceptables.

4. Composé de formule I selon la revendication 1, choisi parmi le groupe constitué
du N-[4-(6,8-dichloro-2-méthyl-1,2,3,4-tétrahydroisoquinolin-4-yl)phényl]-(2R,3S,4R,5R)-2,3,4,5,6-pentahydroxyhexanamide,
du N-[3-(6,8-dichloro-2-méthyl-1,2,3,4-tétrahydroisoquinolin-4-yl)phényl]-(2R,3S,4R,5R)-2,3,4,5,6-pentahydroxyhexanamide,
du N-[2-(6,8-dichloro-2-méthyl-1,2,3,4-tétrahydroisoquinolin-4-yl)phényl]-(2R,3S,4R,5R)-2,3,4,5,6-pentahydroxyhexanamide,
du N-[3-((S)-6,8-dichloro-2-méthyl-1,2,3,4-tétrahydroisoquinolin-4-yl)phényl]-(2R,3S,4R,5R)-2,3,4,5,6-pentahydroxyhexanamide,
du N-[3-((R)-6,8-dichloro-2-méthyl-1,2,3,4-tétrahydroisoquinolin-4-yl)phényl]-(2R,3S,4R,5R)-2,3,4,5,6-pentahydroxyhexanamide,
de la 1-[3-(6,8-dichloro-2-méthyl-1,2,3,4-tétrahydroisoquinolin-4-yl)phényl]-3-(2-hydroxy-1-hydroxyméthyléthyl)urée,
de la 1-[3-(6,8-dichloro-2-méthyl-1,2,3,4-tétrahydroisoquinolin-4-yl)phényl]-3-(2-hydroxy-1,1-bishydroxyméthyléthyl)urée,
de la 1-[3-(6,8-dichloro-2-méthyl-1,2,3,4-tétrahydroisoquinolin-4-yl)phényl]-3-((2S,3R,4R,5R)-2,3,4,5,6-pentahydroxyhexyl)urée,
de la 1-[3-(6,8-dichloro-2-méthyl-1,2,3,4-tétrahydroisoquinolin-4-yl)phényl]-3-((4R,5S,6R)-2,4,5-trihydroxy-6-hydroxyméthyltétrahydropyran-3-yl)urée,
de la {N-[3-(6,8-dichloro-2-méthyl-1,2,3,4-tétrahydroisoquinolin-4-yl)phényl]-N'-(1-sulfo-2-éthyl)}urée,
du chlorure de {N-[3-(6,8-dichloro-2-méthyl-1,2,3,4-tétrahydroisoquinolin-4-yl)phényl]-N'-(éthyl-2-triméthylammonium)}urée,
de la {N-[3-(6,8-dichloro-2-méthyl-1,2,3,4-tétrahydroisoquinolin-4-yl)phényl]-N'-(1-carboxy-3-hydroxy-2S-propyl)}urée,
de la {N-[3-(6,8-dichloro-2-méthyl-1,2,3,4-tétrahydroisoquinolin-4-yl)phényl]-N'-(1-carboxy-4-aminocarboxy-2S-butyl)}urée,
du 3-(6,8-dichloro-2-méthyl-1,2,3,4-tétrahydroisoquinolin-4-yl)-N-((2S,3R,4R,5R)-2,3,4,5,6-pentahydroxyhexyl)benzamide,
du 3-(6,8-dichloro-2-méthyl-1,2,3,4-tétrahydroisoquinolin-4-yl)-N-(2-hydroxy-1-hydroxyméthyléthyl)benzamide,
de l'acide 2-(S)-[3-(6,8-dichloro-2-méthyl-1,2,3,4-tétrahydroisoquinolin-4-yl)benzoylamino]-3-hydroxypropionique,
de l'acide 2-(S)-[3-(6,8-dichloro-2-méthyl-1,2,3,4-tétrahydroisoquinolin-4-yl)benzoylamino]succinique,
de l'acide 2-(S)-[3-(6,8-dichloro-2-méthyl-1,2,3,4-tétrahydroisoquinolin-4-yl)benzoylamino]-4-succinamique,
de la N-[3-(6,8-dichloro-2-méthyl-1,2,3,4-tétrahydroisoquinolin-4-yl)phényl]-N'-[1-carboxy-5-guanidino-2S-pentyl]urée,
de la {N-[3-(6,8-dichloro-2-méthyl-1,2,3,4-tétrahydroisoquinolin-4-(R)-yl)phényl]-N'-(1-carboxy-4-aminocarboxy-2S-butyl)}urée,
de la {N-[3-(6,8-dichloro-2-méthyl-1,2,3,4-tétrahydroisoquinolin-4-(S)-yl)phényl]-N'-(1-carboxy-4-aminocarboxy-2S-butyl)}urée,
de la {N-[3-(6,8-dichloro-2-méthyl-1,2,3,4-tétrahydroisoquinolin-4-(R)-yl)phényl]-N'-(1-carboxy-3-hydroxy-2S-propyl)}urée,
de la {N-[3-(6,8-dichloro-2-méthyl-1,2,3,4-tétrahydroisoquinolin-4-(S)-yl)phényl]-N'-(1-carboxy-3-hydroxy-2S-propyl)}urée,
de la 1-[3-(6,8-dichloro-2-méthyl-1,2,3,4-tétrahydroisoquinolin-4-(R)-yl)phényl]-3-(2-hydroxy-1,1-bishydroxyméthyléthyl)urée,
de la 1-[3-(6,8-dichloro-2-méthyl-1,2,3,4-tétrahydroisoquinolin-4-(S)-yl)phényl]-3-(2-hydroxy-1,1-bishydroxyméthyléthyl)urée,
de la 1-[3-((R)-6,8-dichloro-2-méthyl-1,2,3,4-tétrahydroisoquinolin-4-yl)phényl]-3-((2S,3R,4R,5R)-2,3,4,5,6-pentahydroxyhexyl)urée,
de la 1-[3-((S)-6,8-dichloro-2-méthyl-1,2,3,4-tétrahydroisoquinolin-4-yl)phényl]-3-((2S,3R,4R,5R)-2,3,4,5,6-pentahydroxyhexyl)urée,
du 5-(6,8-dichloro-2-méthyl-1,2,3,4-tétrahydroisoquinolin-4-yl)-N,N'-bis(2-hydroxy-1-hydroxyméthyléthyl)isophthalamide,
du 5-(6,8-dichloro-2-méthyl-1,2,3,4-tétrahydroisoquinolin-4-yl)-N,N'-bis(2-hydroxy-1,1-bishydroxyméthyléthyl)isophthalamide,
du 5-(6,8-dichloro-2-méthyl-1,2,3,4-tétrahydroisoquinolin-4-yl)-N-(2-hydroxy-1,1-bishydroxyméthyléthyl)isophthalamide,
du 5-(6,8-dichloro-2-méthyl-1,2,3,4-tétrahydroisoquinolin-4-yl)-N,N'-bis((2S,3R,4R,5R)-2,3,4,5,6-pentahydroxyhexyl)isophthalamide,
du 5-(6,8-dichloro-2-méthyl-1,2,3,4-tétrahydroisoquinolin-4-yl)-N-((2S,3R,4R,5R)-2,3,4,5,6-pentahydroxyhexyl)isophthalamide,
de l'acide (S)-2-[3-((S)-1-carboxy-2-hydroxyéthylcarbamoyl)-5-(6,8-dichloro-2-méthyl-1,2,3,4-tétrahydroisoquinolin-4-yl)benzoylamino]-3-hydroxypropionique,
du (S)-N-[3-(6,8-dichloro-2-méthyl-1,2,3,4-tétrahydroisoquinolin-4-yl)phényl]-2-amino-5-guanidinopentanamide,
du (S)-N-[4-(6,8-dichloro-2-méthyl-1,2,3,4-tétrahydroisoquinolin-4-yl)phényl]-2-amino-5-guanidinopentanamide,
du (S)-2-amino-N-[3-(6,8-dichloro-2-méthyl-1,2,3,4-tétrahydroisoquinolin-4-yl)phényl]-3-(1H-imidazol-4-yl)propionamide,
du (S)-2-amino-N-[4-(6,8-dichloro-2-méthyl-1,2,3,4-tétrahydroisoquinolin-4-yl)phényl]-3-(1H-imidazol-4-yl)propionamide,
et ses sels et sels trifluoroacétates pharmaceutiquement acceptables.

5. Composé de formule I et ses sels pharmaceutiquement acceptables selon l'une ou plusieurs des revendications 1 à 4, pour une utilisation en tant que médicament.

6. Composé de formule I et de ses sels pharmaceutiquement acceptables selon l'une ou plusieurs des revendications 1 à 4, pour le traitement ou la prophylaxie des troubles de la pulsion respiratoire, des troubles respiratoires, des troubles respiratoires associés au sommeil, des apnées du sommeil, du ronflement, des troubles rénaux chroniques et aigus, d'une défaillance rénale aiguë et d'une défaillance rénale chronique, des troubles de la fonction intestinale, d'une pression artérielle élevée, de l'hypertension essentielle, des troubles du système nerveux central, des troubles provenant de l'hyperexcitabilité du SNC, de l'épilepsie et des convulsion induites centralement ou des états d'anxiété, des dépressions et des psychoses, des états ischémiques du système nerveux central ou périphérique et d'un accident vasculo-cérébral, des dommages chroniques et aigus et des troubles des organes périphériques ou des membres provoqués par des cas d'ischémie ou par des cas de reperfusion, de l'athérosclérose, des troubles du métabolisme lipidique, des thromboses, des troubles de la fonction biliaire, d'une infection par des ectoparasites, des troubles provenant d'un dysfonctionnement de l'endothélium, des troubles provoqués par des protozoaires, du paludisme, en vue de la conservation et du stockage des transplants pour des procédures chirurgicales, pour l'utilisation dans des opérations chirurgicales et des transplantations d'organes ou pour le traitement des états de choc ou des diabètes et des dommages tardifs des diabètes ou des maladies dans lesquelles la prolifération cellulaire représente une cause primaire ou secondaire, et pour conserver la santé et pour prolonger la vie.

7. Composition thérapeutique pour une utilisation humaine, vétérinaire ou phytoprotectrice comprenant une quantité efficace d'un composé de formule I et/ou d'un sel pharmaceutiquement acceptable de ce composé selon l'une ou plusieurs des revendications 1 à 4.

8. Composition thérapeutique pour une utilisation humaine, vétérinaire ou phytoprotectrice comprenant une quantité efficace d'un composé de formule I et/ou d'un sel pharmaceutiquement acceptable de ce composé selon l'une ou plusieurs des revendications 1 à 4, en combinaison avec d'autres ingrédients pharmacologiques actifs ou avec d'autres médicaments.
